(19) Europäisches Patentamt
European Patent Office
Office européen des brevets

(11) **EP 1 897 881 A2**

(12) # EUROPEAN PATENT APPLICATION


(43) Date of publication:
**12.03.2008 Bulletin 2008/11**

(21) Application number: **07122274.9**

(22) Date of filing: **19.06.2003**

(51) Int Cl.:
***C07D 471/04*** *(2006.01)* ***C07D 495/04*** *(2006.01)*
***A61P 3/00*** *(2006.01)* ***A61K 31/4365*** *(2006.01)*

(84) Designated Contracting States:
**AT BE BG CH CY CZ DE DK EE ES FI FR GB GR HU IE IT LI LU LV MC NL PT RO SE SI SK TR**

(30) Priority:
**20.06.2002 SE 0201925**
**11.07.2002 SE 0202181**
**26.08.2002 US 406120 P**
**01.10.2002 SE 0202908**
**17.12.2002 US 434010 P**
**10.02.2003 SE 0300357**
**23.04.2003 US 464701 P**

(62) Document number(s) of the earlier application(s) in accordance with Art. 76 EPC:
**03760999.7 / 1 513 828**



(71) Applicant: **Biovitrum AB (publ)**
**112 76 Stockholm (SE)**

(72) Inventors:
- **Johansson, Gary**
**169 66, SOLNA (SE)**
- **Jenmalm Jensen, Annika**
**756 45, UPPSALA (SE)**
- **Beierlein, Katarina**
**756 55, UPPSALA (SE)**


Remarks:
This application was filed on 04-12-2007 as a divisional application to the application mentioned under INID code 62.

(54) **Compounds useful for the treatment of obesity, type II diabetes and CNS disorders**


(57) The present invention relates to compounds of the general formula (I),

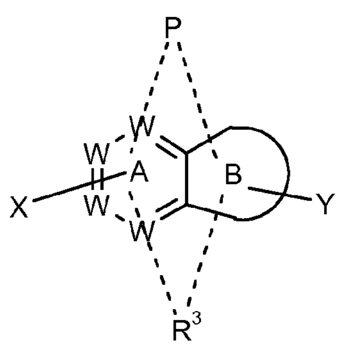

(I)

wherein P is sulfone or sulfonamide; and
A, B, W, X, Y and $R^3$ are as defined in the description; to pharmaceutical compositions comprising these compounds, and to the use of the compounds for the prophylaxis and treatment of medical conditions relating to obesity, type II diabetes, and/or CNS disorders, to achieve reduction of body weight and of body weight gain.


EP 1 897 881 A2

## Description

TECHNICAL FIELD

The present invention relates to substituted sulphone and sulphonamide compounds, to pharmaceutical compositions comprising these compounds, and to the use of the compounds for the prophylaxis and treatment of medical conditions relating to obesity, type 2 diabetes, and/or disorders of the central nervous system (CNS), to achieve reduction of body weight and of body weight gain, as well as for cosmetic use.

BACKGROUND ART

Obesity is a condition characterized by an increase in body fat content resulting in excess body weight above accepted norms. Obesity is the most important nutritional disorder in the western world and represents a major health problem in all industrialized countries. This disorder leads to increased mortality due to increased incidences of diseases such as cardiovascular disease, digestive disease, respiratory disease, cancer and type 2 diabetes. Searching for compounds, which reduce body weight has been going on for many decades. One line of research has been activation of serotoninergic systems, either by direct activation of serotonin receptor subtypes or by inhibiting serotonin reuptake. The exact receptor subtype profile required is however not known.

Serotonin (5-hydroxytryptamine or 5-HT), a key transmitter of the peripheral and central nervous system, modulates a wide range of physiological and pathological functions, including anxiety, sleep regulation, aggression, feeding and depression. Multiple serotonin receptor subtypes have been identified and cloned. One of these, the 5-$HT_6$ receptor, was cloned by several groups in 1993 (Ruat, M. et al. (1993) Biochem. Biophys. Res. Commun. 193: 268-276; Sebben, M. et al. (1994) NeuroReport 5: 2553-2557). This receptor is positively coupled to adenylyl cyclase and displays affinity for antidepressants such as clozapine. Recently, the effect of 5-$HT_6$ antagonist and 5-$HT_6$ antisense oligonucleotides to reduce food intake in rats has been reported (Bentley, J.C. et al. (1999) Br J Pharmac. Suppl. 126, P66; Bentley, J.C. et al. (1997) J. Psychopharmacol. Suppl. A64, 255; Woolley M.L. et al. (2001) Neuropharmacology).

Compounds with enhanced affinity and selectivity for the 5-$HT_6$ receptor have been identified, e.g. in WO 00/34242 and by Isaac, M. et al. (2000) 6-Bicyclopiperazinyl-1-arylsulfonylindoles and 6-Bicyclopiperidinyl-1-arylsulfonylindoles derivatives as novel, potent and selective 5-HT6 receptor antagonists. Bioorganic & Medicinal Chemistry Letters 10: 1719-1721 (2000).

INFORMATION DISCLOSURE

J. Med. Chem. 1970, 13(4), 592-598 describes N-(4-{[2-(diethylamino)ethyl]amino}-1-naphthyl)amides; N-{5,6,7,8-Tetrahydro-4-[(3-piperidinopropyl)amino]-1-naphthyl} amides and related amides and urea derivatives as schistosomicides.

WO 99/42465 discloses sulphonamides derivatives that bind to the 5-$HT_6$ receptor and that can be used for the treatment of CNS disorders such as anxiety, depression, epilepsy, obsessive compulsive disorders, cognitive disorders, ADHD, anorexia and bulimia schizophrenia, drug abuse.

WO 01/32646 A1 discloses compounds that bind to the 5-$HT_6$ receptor and that are used for the treatment of CNS disorders and which inter alia may be used for the treatment of eating disorders.

WO 99/37623 A2 discloses compounds that bind to the 5-$HT_6$ receptor and that are used for the treatment of CNS disorders and which inter alia may be used for the treatment of eating disorders.

WO 99/42465 A3 discloses compounds that bind to the 5-$HT_6$ receptor and that are used for the treatment of CNS disorders and which inter alia may be used for the treatment of eating disorders.

EP 0 815 861 A1 discloses compounds that bind to the 5-$HT_6$ receptor and that are used for the treatment of CNS disorders.

WO 99/02502 A2 discloses compounds that bind to the 5-$HT_6$ receptor and that are used for the treatment of CNS disorders and which inter alia may be used for the treatment of eating disorders.

WO 98/27081 A1 discloses compounds that bind to the 5-$HT_6$ receptor and that are used for the treatment of CNS disorders and which inter alia may be used for the treatment of eating disorders.

EP 0701819 discloses compounds that bind to the 5-$HT_{1D}$ receptor and that are used for the treatment of CNS disorders and obesity.

US 6,191,141 and WO 01/12629 disclose compounds that bind to the 5 -$HT_6$ receptor and that are used for the treatment of CNS disorders.

DISCLOSURE OF THE INVENTION

It has surprisingly been found that the compounds of formula (I) show affinity for the 5-$HT_6$ receptor as antag-

onists at low nanomolar range. Compounds according to the invention and their pharmaceutically acceptable salts have 5-$HT_6$ receptor antagonist, agonist and partial agonist activity and are believed to be of potential use in the treatment or prophylaxis of obesity and type 2 diabetes, to achieve reduction of body weight and of body weight gain, as well as in the treatment or prophylaxis of disorders of the central nervous system such as anxiety, depression, panic attacks, memory disorders, cognitive disorders, sleep disorders, migraine, anorexia, bulimia, binge disorders, obsessive compulsive disorders, psychoses, Alzheimer's disease, Parkinson's disease, Huntington's chorea and/or schizophrenia, Attention Deficit Hyperactive Disorders (ADHD), drug abuse. The reduction of body weight and of body weight gain (e.g. treating body-weight disorders) is achieved *inter alia* by reduction of food intake. As used herein, the term "body weight disorders" refers to the disorders caused by an imbalance between energy intake and energy expenditure, resulting in abnormal body (e.g., excessive) weight. Such body weight disorders include obesity.

*Definitions*

Unless otherwise stated or indicated, the term "$C_{1-6}$ alkyl" (or "$C_{2-6}$ alkenyl") denotes a straight or branched hydrocarbon chain group having from 1 to 6 carbon atoms (or 2 to 6 carbon atoms). Examples of said lower alkyl include methyl, ethyl, n-propyl, iso-propyl, n-butyl, iso-butyl, sec-butyl, t-butyl and straight- and branched-chain pentyl and hexyl. Alkenyl groups have one or more double carbon-carbon bonds in the chain.

Unless otherwise stated or indicated, the term "$C_{1-6}$ alkoxy" denotes a straight or branched alkoxy group having from 1 to 6 carbon atoms. Examples of said lower alkoxy include methoxy, ethoxy, n-propoxy, iso-propoxy, n-butoxy, iso-butoxy, sec-butoxy, t-butoxy and straight- and branched-chain pentoxy and hexoxy.

Unless otherwise stated or indicated, the term "$C_{1-6}$ alkoxyalkyl" denotes a straight or branched alkoxyalkyl group having from 1 to 6 carbon atoms. Examples of said lower alkoxyalkyl include methoxymethyl, ethoxymethyl, iso-propoxymethyl, n-butoxymethyl, t-butoxyethyl and straight- and branched-chain pentoxymethyl.

The expression "$C_{2-6}$ alkenyl" as used herein refers to straight-chained and branched alkenyl groups containing from 2 to 6 carbon atoms. Typical examples include vinyl, allyl, 2,3-dimethylallyl, 1-butenyl groups, 1-pentenyl, and 1-hexenyl groups.

The expression "$C_{2-6}$ alkynyl" as used herein refers to straight-chained and branched alkynyl groups containing from 2 to 6 carbon atoms. Typical examples include ethynyl, 1-propynyl, 1-butynyl, 1-pentynyl, and 1-hexynyl groups.

Unless otherwise stated or indicated, the term "halogen" shall mean fluorine, chlorine, bromine or iodine.

The term "alkylhalide" refers to an alkyl group substituted with one or more halogen groups (e.g., F, Cl, Br, I).

The term "$C_{3-7}$ cycloalkyl" denotes a cyclic alkyl group having a ring size from $C_3$ to $C_7$, which can be saturated or partially unsaturated. Examples of said cycloalkyl include cyclopropyl, cyclobutyl, cyclopentyl, cyclopentenyl, cyclopentadienyl, cyclohexyl, methylcyclohexyl, cyclohexenyl, cyclohexadienyl, and cycloheptyl.

The term "$C_{5-10}$ cycloalkenyl" denotes a cyclic alkenyl group having a ring size from $C_5$ to $C_{10}$. Examples of said cycloalkenyl include 1-cyclopentyl, 2-cyclopentenyl, 1-cyclohexenyl, 1-cyclohepentyl, 1-cyclooctenyl, 1-cyclononenyl, and 1-cyclodecenyl groups.

The term "heterocyclic" refers to a hydrocarbon ring system containing 4 to 8 ring members that have at least one heteroatom (e.g., S, N, or O) as part of the ring. It includes saturated, unsaturated, aromatic, and nonaromatic heterocycles. Suitable heterocyclic groups include thienyl, furyl, pyridyl, pyrrolidinyl, imidazolyl, pyrazolyl, piperidyl, azepinyl, morpholinyl, pyranyl, dioxanyl, pyridazinyl, pyrimidinyl, and piperazinyl groups

Unless otherwise stated or indicated, the term "aryl" refers to a hydrocarbon ring system having at least one aromatic ring. Examples of aryl groups include phenyl, cinnamyl, pentalenyl, indenyl, 1-naphthyl, 2-naphthyl, anthryl and phenanthryl.

The term "heteroaryl" refers to a hydrocarbon ring system having at least one aromatic ring which contains at least one heteroatom such as O, N, or S. Examples of heteroaryl groups include furyl, pyrrolyl, thienyl, oxazolyl, imidazolyl, thiazolyl, pyridinyl, pyrimidinyl, quinazolinyl, and indolyl groups.

*Compounds of Formula (I)*

One object of the present invention is a compound having the general formula (I):

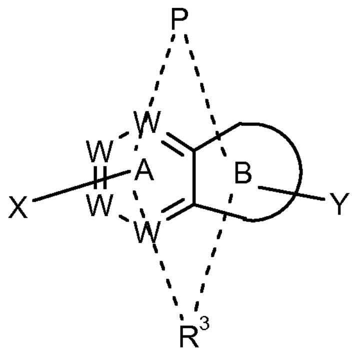

(I)

or a pharmaceutically acceptable salt thereof, wherein:
ring B is a ring

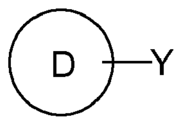

;

in which D is a five-membered heterocyclic or heteroaryl ring, said heteroaryl ring comprising one or two atoms selected from the group consisting of nitrogen, sulfur and oxygen, with the proviso that when D contains an oxygen atom, D is heteroaryl;
each W is independently -N-, -(CH)-, or -C- provided that not more than three groups W are -N-;
P is any one of formula (a), (b) or (c)

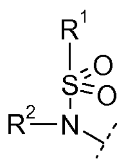 , 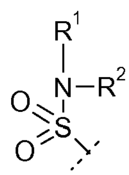 , or 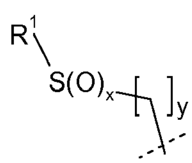 ;

(a) (b) (c)

wherein x = 0, 1, or 2 and y = 0, 1, or 2;
and P and $R^3$ can be attached to any carbon atom that allows the substitution in one of either the A- or B-ring, or when ring A contains at least one nitrogen atom and P is (c), then P can also be attached to any nitrogen in ring B that allows the substitution;
the dashed bonds denote that P and $R^3$, respectively, may be attached to either the A or B ring; but each P or $R^3$ may not be simultaneously bound to both rings A and B;

$R^1$ is

(a) $C_{1-6}$alkyl,
(b) $C_{1-6}$ alkoxyalkyl,
(c) straight-chained or branched $C_{1-6}$ hydroxyalkyl,
(d) straight-chained or branched $C_{1-6}$ alkylhalides,
(e) aryl carbonylmethyl,
(f) $C_{3-7}$ cycloalkyl, which is optionally partially unsaturated,
(g) $C_{3-7}$ cycloalkyl-$C_{1-6}$ alkyl, wherein the cyclic ring is optionally partially unsaturated, or
(h) a group Ar;

wherein Ar is

(a) phenyl,
(b) 1-naphthyl,
(c) 2-naphthyl,
(d) aryl-$C_{1-6}$alkyl,
(e) cinnamyl,
(f) a 5 to 7-membered, optionally aromatic, partially saturated or completely saturated, mono- or bi-cyclic heterocyclic ring, each containing 1 to 4 heteroatoms, selected from oxygen, sulfur, and nitrogen,
(g) a bicyclic ring system comprising at least one heterocyclic ring according to (f) and a group Ar,
wherein the group Ar is substituted in one or more positions with

(a) H, X or Y, or
(b) a 5 to 7-membered, optionally aromatic, partially saturated or completely saturated, heterocyclic ring each containing 1 to 4 heteroatoms selected from oxygen, nitrogen or sulfur;

$R^2$ is

(a) H,
(b) $C_{1-6}$ alkyl,
(c) $C_{2-6}$ alkoxyalkyl,
(d) straight or branched $C_{1-6}$ hydroxyalkyl, or
(e) straight or branched $C_{1-6}$ alkylhalides;
(f) a group Ar,

or $R^1$ and $R^2$ are linked to form a group $-CH_2CH_2OCH_2CH_2-$ or

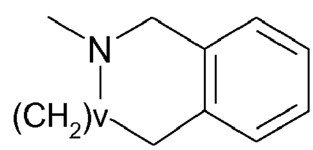

wherein v is 0-2,
X and Y are independently

(a) H,
(b) halogen,
(c) $C_{1-6}$ alkyl,
(d) $CF_3$,
(e) hydroxy,
(f) $C_{1-6}$ alkoxy,
(g) $C_{2-6}$ alkenyl,
(h) phenyl,
(i) phenoxy,
(j) benzyloxy,
(k) benzoyl,
(1) $-OCF_3$,
(m) -CN,
(n) straight or branched $C_{1-6}$ hydroxyalkyl,
(o) straight or branched $C_{1-6}$ alkylhalides,
(p) $-NH_2$,
(q) $-NHR^4$,
(r) $-NR^4R^5$,
(s) $-NO_2$,
(t) $-CONR^4R^5$,
(u) $-NHSO_2R^4$,

(v) $-NR^4COR^5$,
(x) $-SO_2NR^4R^5$,
(z) $-C(=O)R^4$,
(aa) $-CO_2R^4$, or
(ab) $-S(O)_nR^4$, wherein n is 0, 1, 2 or 3,
(ac) $-S-(C_{1-6})$ alkyl, or
(ad) $-SCF_3$; and

$R^4$ and $R^5$ are independently

(a) H,
(b) $C_{1-6}$ alkyl,
(c) $C_{3-7}$ cycloalkyl, or
(d) Ar, as defined above for $R^1$;
alternatively, $R^4$ and $R^5$ are linked to form a group $-CH_2OCH_2-$, $-CH_2CH_2OCH_2CH_2-$ or $(CH_2)_{3-5}$;

$R^3$ is a group selected from any one of

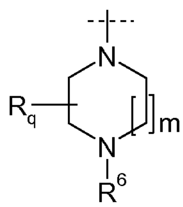
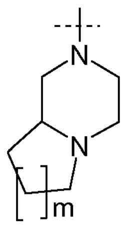
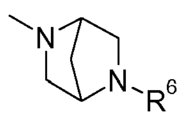
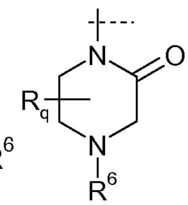
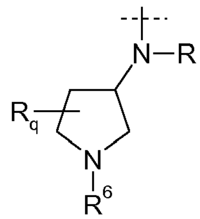

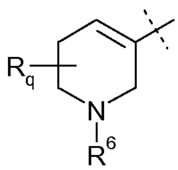
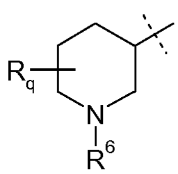
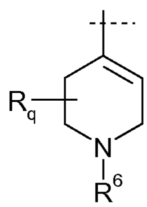
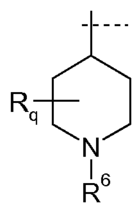
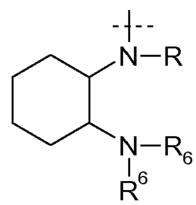

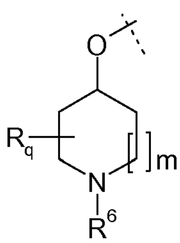
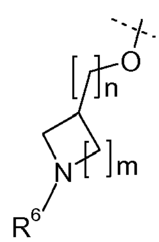
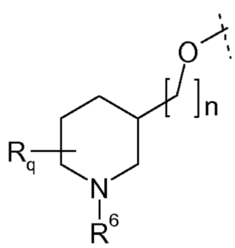
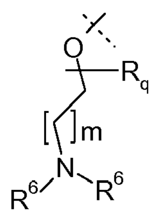

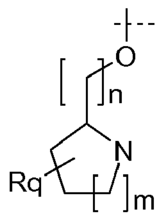
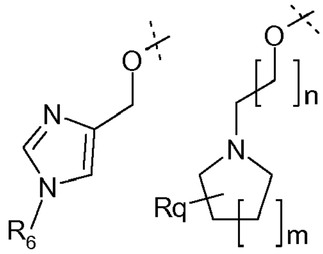
and
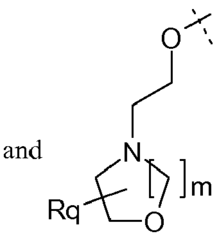

wherein $R^3$ is optionally substituted on each carbon atom that allows the substitution with Rq groups, wherein Rq is independently H, or $(C_{1-6})$ alkyl, and wherein two Rq groups can be present on the same carbon atom simultaneously, wherein

q=1,2,3,4,5 or 6,
m = 1 or 2, and
n = 0,1 or 2;

$R^6$ is independently

(a) H,
(b) linear or branched $C_{1-6}$ alkyl,
(c) benzyl,
(d) $-CH_2-CH_2-OH$, or
(e) $-CH_2-CH_2-O-C_{1-6}$ alkyl;

P and $R^3$ can be attached to the same ring or to different rings of rings A and B;
provided that
when P is

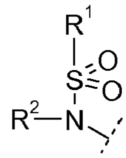
(a) or
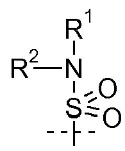

(b), and P and $R^3$ both are attached to ring A in the meta- or para-position relative to one another then $R^3$ is selected from any one of

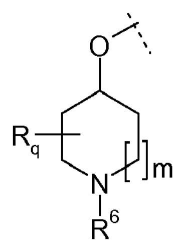
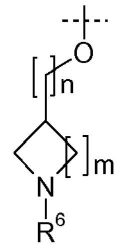
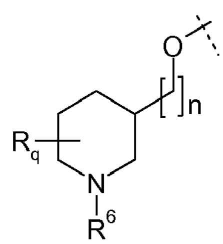
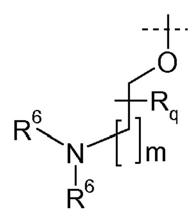

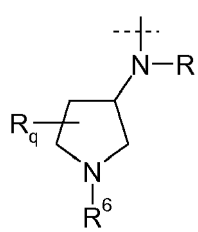
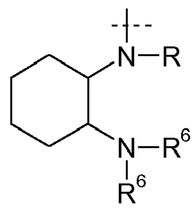
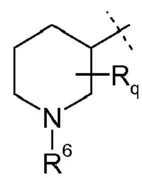
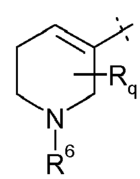

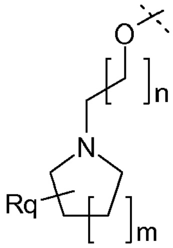 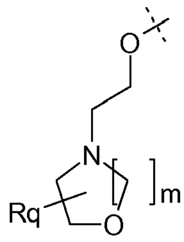 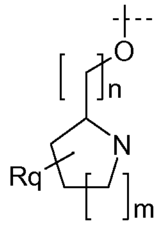 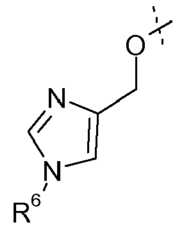 ;

when the ring system A + B is benzofurane or benzothiophene, and P is

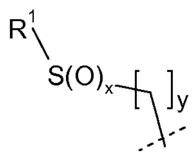 (c)

and attached to position 3 in the A+B ring system, and $R^3$ is a group selected from any one of

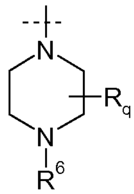 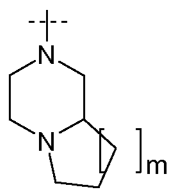 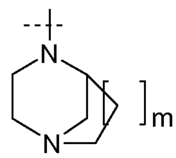

and attached to position 7 in the A+B ring system, then y = 1 or 2;
when the ring system A + B is indole, and P is

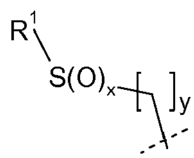 (c)

and P is attached to position 3 in the A+B ring system, and $R^3$ is a group selected from any one of

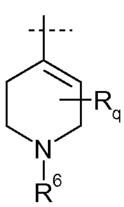 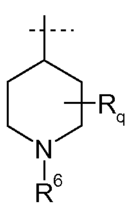 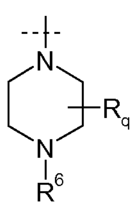 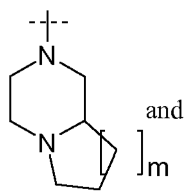 and 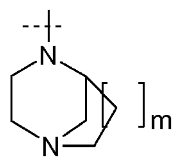

and $R^3$ is attached to any one of positions 5, 6 or 7 in the A+B ring system, then y = 1 or 2; or
when ring D is a pyrrole ring, P is of the formula (c), then $R^3$ is not of the formula

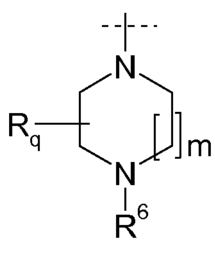
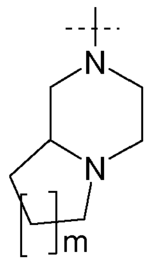
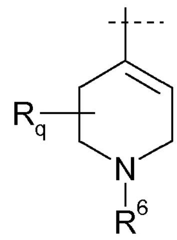
or
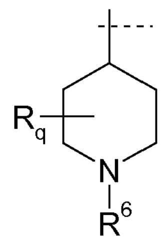

substituted in position 3 on the pyrrole ring.

A pyrrole ring, as connected to an A ring, has the following position numbers:

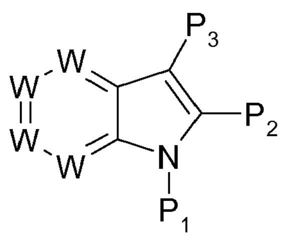

wherein $P_1$-$P_3$ denote the position on the pyrrole ring.

It is preferred that:

$R^1$ is

(a) $C_{1-6}$ alkyl, or
(e) a group Ar;

Ar is

(a) phenyl,
(b) 1-naphthyl,
(c) 2-naphthyl, or
(f) a 5 to 7-membered, optionally aromatic, partially saturated or completely saturated, heterocyclic ring containing 1 to 4 heteroatoms, selected from oxygen, nitrogen and sulfur,

wherein the group Ar is substituted in one or more positions with

(a) H,
(b) halogen,
(c) $C_{1-6}$ alkyl,
(d) $-CF_3$,
(f) $C_{1-6}$ alkoxy,
(g) $C_{2-6}$ alkenyl (preferably $C_{2-4}$ alkenyl),
(1) $-OCF_3$,
(m) straight or branched $C_{1-6}$ hydroxyalkyl,
(n) phenyloxy,
(o) benzyloxy,
(v) $-NR^4COR^5$,
(x) $-SO_2NR^4R^5$,
(z) $-C(=O)R^4$,

(ab) $-S(O)_nR^4$, wherein n is 0, 1, 2 or 3;
(ac) $-S-(C_{1-6})$ alkyl, or
(ad) $-SCF_3$;

$R^2$ is

(a) H, or
(b) $C_{1-6}$ alkyl;

or $R^1$ and $R^2$ are linked to form a group $-CH_2CH_2OCH_2CH_2-$;

X and Y are H;

$R^4$ and $R^5$ are each independently H or $C_{1-3}$ alkyl; and

$R^3$ is selected from any one of

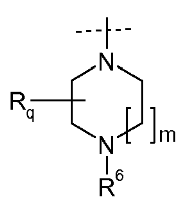
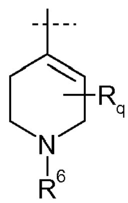
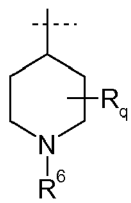

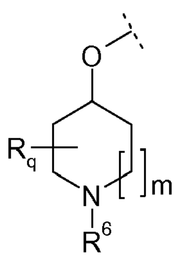
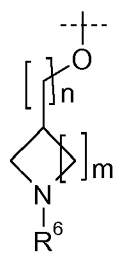
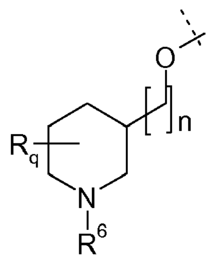
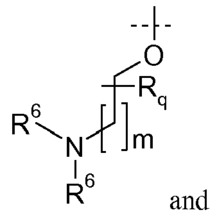
and
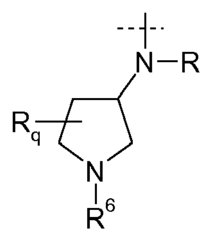

wherein $R^3$ can be substituted on each carbon atom that allows the substitution with Rq groups, wherein Rq is independently H, or $C_{1-6}$ alkyl, and wherein two Rq groups can be present on the same carbon atom simultaneously, wherein

q = 1 or 2,
m = 1 or 2,
n = 0, and

$R^6$ is independently

(a) H,
(b) $C_{1-6}$ alkyl (preferably $C_{1-3}$ alkyl), in particular methyl,
(d) $-CH_2-CH_2-OH$, or
(e) $-CH_2-CH_2-OCH_3$.

It is especially preferred that $R^3$ is selected from any one of

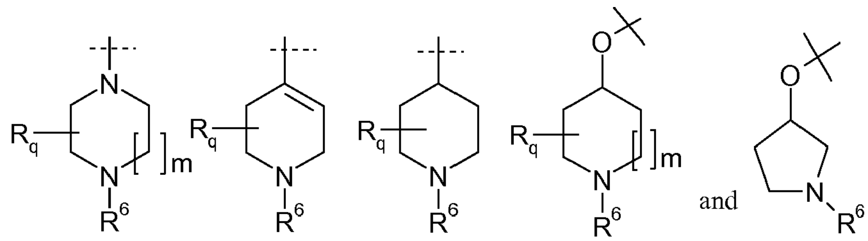

and

wherein $R^3$ can be substituted on each carbon atom that allows the substitution with Rq groups, wherein Rq is independently H, or $C_{1-2}$ alkyl, and wherein two Rq groups can be present on the same carbon atom simultaneously, wherein

q = 1 or 2,
m = 1 or 2; and

$R^6$ is independently

(a) H,
(b) $C_{1-3}$ alkyl,
(d) $-CH_2-CH_2-OH$, or
(e) $-CH_2-CH_2-OCH_3$.

It is also preferred that $R^3$ is selected from any one of

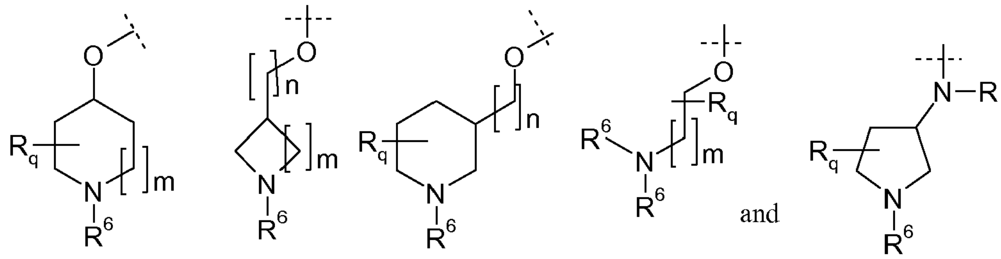

and

wherein $R^3$ can be substituted on each carbon atom that allows the substitution with Rq groups, wherein Rq is independently H, or $C_{1-6}$ alkyl, and wherein two Rq groups can be present on the same carbon atom simultaneously, wherein

q = 1 or 2,
m = 1 or 2,
n = 0, and

$R^6$ is independently

(a) H,
(b) $C_{1-3}$ alkyl,
(d) $-CH_2-CH_2-OH$, or
(e) $-CH_2-CH_2-OCH_3$.

It is also preferred that $R^3$ is selected from any one of

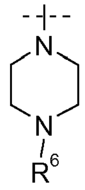 ; 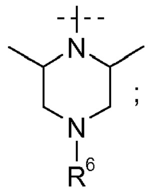 ; 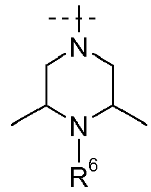 ; 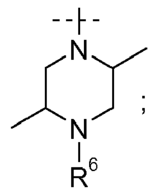 ; 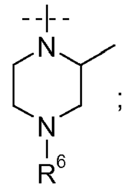 ;

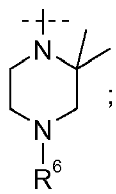 ; 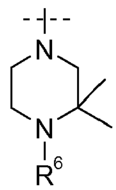 ; 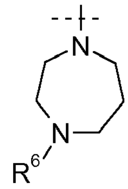 ; 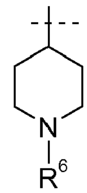 ; 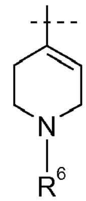 ;

$R^6$ is independently

(a) H,
(b) $C_{1-3}$ alkyl,
(d) $-CH_2-CH_2-OH$, or
(e) $-CH_2-CH_2-OCH_3$.

It is preferred that $R^6$ is H or methyl.
It is also preferred that $R^3$ is piperazine; homopiperazine; 2,6-dimethylpiperazine; 3,5-dimethylpiperazine; 2,5-dimethylpiperazine; 2-methylpiperazine; 3-methylpiperazine; 2,2-dimethylpiperazine; 3,3-dimethylpiperazine; piperidine; 1,2,3,6-tetrahydro-pyrazine; or 4-pyrrolidin-3-yloxy.
It is preferred that the groups Y and X are attached to any unsubstituted carbon atom.
It is preferred that D is pyrrolyl, thienyl or furanyl.
It is preferred that P is

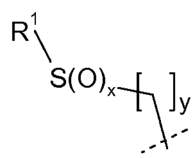 (c)

wherein $R^1$, x, and y are as defined in claim 1.
It is also preferred that P is

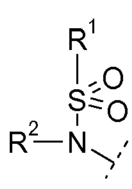 (a) or 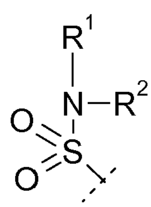 (b)

wherein $R^1$ and $R^2$ are as defined in claim 1.
It is preferred that $R^2$ is H.
Another object of the present invention is a compound of the general formula (IV)

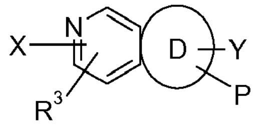

(IV)

wherein P is of the formula (c), $R^1$, x, y, X, and Y are as defined in claim 1, and $R^3$ is as defined in claim 2, and wherein D is a five-membered heteroaryl ring, said ring comprising one or two atoms selected from the group consisting of nitrogen, sulfur and oxygen; and when the heteroaryl ring comprises one or two nitrogen atoms, a group $R^6$ is attached at any nitrogen atom which allows the substitution.

It is preferred that D is a thiophene and P is attached to the D ring, giving a skeleton as any of the following:

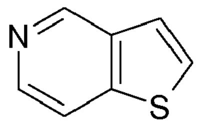 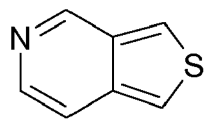 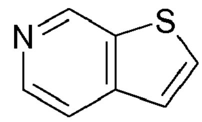

It is also preferred that D is pyrrole and P is attached to the nitrogen atom in the D ring, giving a skeleton as any of the following:

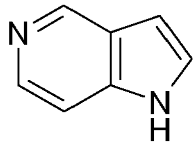 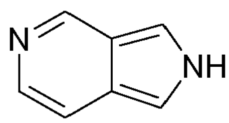 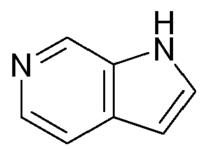

It is also preferred that D is furan and P is attached to the D ring, giving a skeleton as any of the following:

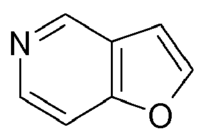 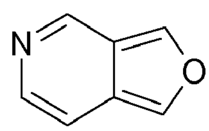 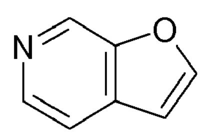

Another object of the present invention is a compound of the general formula (V)

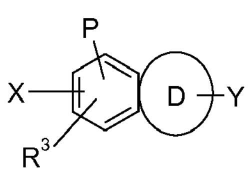

(V)

wherein P is of the formula (c) as defined in claim 1, $R^1$, x, y, X, Y, and $R^3$ are as defined in claim 1, and wherein D is a five-membered heteroaryl ring, said heteroaryl ring comprising one or two atoms selected from the group consisting of nitrogen, sulfur and oxygen; and when the heteroaryl ring comprises one or two nitrogen atoms, a group $R^6$ is attached at any nitrogen atom which allows the substitution.

Another object of the present invention is a compound of the general formula (V)

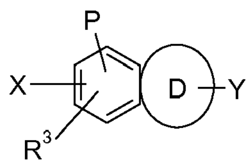

(V)

wherein P is of the formula (a) or (b) as defined in claim 1, preferably wherein $R^2$ is H, X, Y, and $R^3$ are as defined in claim 1, and
wherein D is a five-membered heteroaryl ring, said heteroaryl ring comprising one or two atoms selected from the group consisting of nitrogen, sulfur and oxygen; and when the heteroaryl ring comprises one or two nitrogen atoms, a group $R^6$ is attached at any nitrogen atom which allows the substitution.

Another object of the present invention is a compound of the general formula (VI)

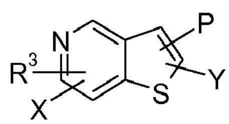

(VI)

wherein P is of the formula (a) or (b) as defined in claim 1, preferably wherein $R^2$ is H, X and Y are as defined in claim 1, and $R^3$ is as defined in claim 2.

Another object of the present invention is a compound of the general formula (VII)

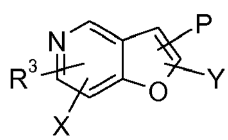

(VII)

wherein P is of the formula (a) or (b) as defined in claim 1, preferably wherein $R^2$ is H, X and Y are as defined in claim 1, and $R^3$ is as defined in claim 4.

Another object of the present invention is a compound of the general formula (VIII)

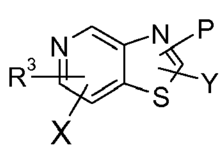

(VIII)

wherein P is of the formula (a) or (b) as defined in claim 1, preferably wherein $R^2$ is H, X, Y, and $R^3$ are as defined in claim 1.

Another object of the present invention is a compound of the general formula (IX)

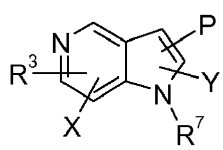

(IX)

wherein $R^7$ in formula (IX) is:

(a) H,
(b) $C_{1-6}$ alkyl,

(c) benzyl,
(d) $-CH_2-CH_2-OH$, or
(e) $CH_2-CH_2-O-CH_3$, and

wherein P is of the formula (a) or (b) as defined in claim 1, preferably wherein $R^2$ is H, X, Y, and $R^3$ are as defined in claim 1.

Another object of the present invention is a compound of the general formula (X)

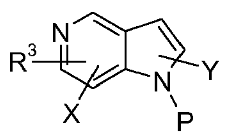

(X)

wherein P is of the formula (a) or (b) as defined in claim 1, preferably wherein $R^2$ is H, X, Y, and $R^3$ are as defined in claim 1.

Another object of the present invention is a compound of the general formula (XII):

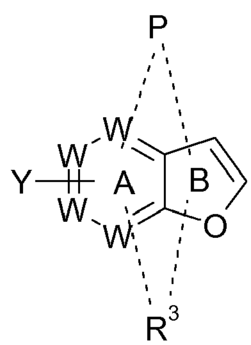

(XII)

or a pharmaceutically acceptable salt thereof, wherein P and $R^3$ are attached to the same ring or to different rings of rings A and B, wherein A, B, Y, P, and $R_3$ are as defined in claim 1.

Preferred compounds of the formula (IV) are

4-(1,4-Diazepan-1-yl)-2-(phenylsulfonyl)thieno[3,2-c]pyridine hydrochloride;
4-(1,4-Diazepan-1-yl)-2-[(3,4-dichlorophenyl)sulfonyl]thieno[3,2-c]pyridine hydrochloride;
4-(1,4-Diazepan-1-yl)-2-[4-tert-butylphenylsulfonyl)thieno[3,2-c]pyridine hydrochloride;
4-(1,4-Diazepan-1-yl)-2-[4-tert-butylphenylsulfonyl)thieno[3,2-c]pyridine hydrochloride;
4-(1,4-Diazepan-1-yl)-2-[3,4-dimethylphenylsulfonyl)thieno[3,2-c]pyridine hydrochloride;
2-[(4-Bromophenyl)sulfonyl]-4-(1,4-diazepan-1-yl)thieno[3,2-c]pyridine hydrochloride;
2-(Phenylsulfonyl)-4-piperazin-1-ylthieno[3,2-c]pyridine hydrochloride;
2-(3-Methoxy-benzenesulfonyl)-4-piperazin-1-yl-thieno[3,2-c]pyridine hydrochloride;
2-(4-Methoxy-benzenesulfonyl)-4-piperazin-1-yl-thieno[3,2-c]pyridine hydrochloride;
4-Piperazin-1-yl-2- {[4-trifluoromethyl)phenyl]sulfonyl}thieno[3,2-c]pyridine hydrochloride;
2-[[2-tert-Butylphenyl)sulfonyl]-4-piperazin-1-ylthieno[3,2-c]pyridine hydrochloride;
2-[(3,4-Dichlorophenyl)sulfonyl]-4-piperazin-1-ylthieno [3,2-c]pyridine-2-sulfonamide hydrochloride;
2-[(4-tert-Butylphenyl)sulfonyl]-4-piperazin-1-ylthieno[3,2-c]pyridine hydrochloride;
2-(1-Naphthyl sulfonyl)-4-piperazin-1-ylthieno[3,2-c]pyridine hydrochloride;
2-[(3-Fluorophenyl)sulfonyl]-4-piperazin-1-ylthieno[3,2-c]pyridine-2-sulfonamide hydrochloride;
2-(Mesitylsulfonyl)-4-piperazin-1-ylthieno[3,2-c]pyridine hydrochloride;
2-[(2-Methoxyphenyl)sulfonyl]-4-piperazin-1-ylthieno[3,2-c]pyridine hydrochloride;
2-[(2,4-Dimethoxyphenyl)sulfonyl]-4-piperazin-1-ylthieno[3,2-c]pyridine hydrochloride;
2-[(2,4-Dimethylphenyl)sulfonyl]-4-piperazin-1-ylthieno[3,2-c]pyridine hydrochloride;
2-[(2,5-Dimethylphenyl)sulfonyl]-4-piperazin-1-ylthieno[3,2-c]pyridine hydrochloride;
2-[(2-Ethylphenyl)sulfonyl]-4-piperazin-1-ylthieno[3,2-c]pyridine hydrochloride;

4-(Piperazinyl)-2-(3-methoxybenzyl-sulfonyl)-thienopyridine hydrochloride;
2-(Benzylsulfonyl)-4-piperazin-1-ylthieno[3,2-c]pyridine hydrochloride;
4-Piperazin-1-yl-2-{[4-(trifluoromethyl)benzyl]sulfonyl}thieno[3,2-c]pyridine hydrochloride;
2-[(3-Bromobenzyl)sulfonyl]-4-piperazin-1-ylthieno[3,2-c]pyridine hydrochloride;
2-[(2,3-Difluorobenzyl)sulfonyl]-4-piperazin-1-ylthieno[3,2-c]pyridine hydrochloride;
2-[(4-Bromobenzyl)sulfonyl]-4-piperazin-1-ylthieno[3,2-c]pyridine hydrochloride;
2- {[2,5-bis(Trifluoromethyl)benzyl]sulfonyl}-4-piperazin-1-ylthieno[3,2-c]pyridine hydrochloride;
2-[(4-Methylbenzyl)sulfonyl]-4-piperazin-1-ylthieno[3,2-c]pyridine hydrochloride;
2-{[5-Chloro-2-(trifluoromethyl)benzyl]sulfbnyl}-4-piperazin-1-ylthieno[3,2-c]pyridine hydrochloride;
2-[(3,5-Dimethoxybenzyl)sulfonyl]-4-piperazin-1-ylthieno[3,2-c]pyridine hydrochloride;
2-[(2-Naphthylmethyl)sulfonyl]-4-piperazin-1-ylthieno[3,2-*c*]pyridine hydrochloride;
4-Piperazin-1-yl-2-{[4-(1,2,3-thiadiazol-4-yl)benzyl]sulfonyl}thieno[3,2-*c*]pyridine hydrochloride;
1-(4-Pyrrolidin-1-ylphenyl)-2-[(4-piperazine-1-ylthieno[3,2-c]pyridin-2-yl) sulfonyl] ethanone hydrochloride; and
1-[4-(Diethylamino)phenyl]-2-[(4-piperazine-1-ylthieno[3,2-c]pyridin-2-yl) sulfonyl] ethanone hydrochloride.

Also preferred compounds of the formula (IV) are

1-(4-Methylphenylsulphonyl)-4-piperazin-1-yl-1*H*-pyrrolo[3,2-*c*]pyridine hydrochloride;
1-(3-Chloro-2-methylphenylsulphonyl)-4-piperazin-1-yl-1*H*-pyrrolo[3,2-*c*]pyridine hydrochloride;
1-(3,4-Dimethoxyphenylsulphonyl)-4-piperazin-1-yl-1*H*-pyrrolo[3,2-*c*]pyridine hydrochloride;
4-(4-Piperazin-1-yl-pyrrolo[3,2-c]pyridine-1-sulfonyl)-benzonitrile hydrochloride;
1-(4,5-Dichloro-thiophene-2-sulfonyl)-4-piperazin-1-yl-1H-pyrrolo[3,2-c]pyridine hydrochloride;
1-(2-Chloro-4-fluorophenylsulphonyl)-4-piperazin-1-yl-1*H*-pyrrolo[3,2-*c*]pyridine hydrochloride;
1-Phenylmethanesulfonyl-4-piperazin-1-yl-1H-pyrrolo [3,2-c]pyridine hydrochloride;
1-(5-Chloro-thiophene-2-sulfonyl)-4-piperazin-1-yl-1H-pyrrolo[3,2-c]pyridine hydrochloride;
1-(4-Butyl-benzenesulfonyl)-4-piperazin-1-yl-1H-pyrrolo(3,2-c)pyridine hydrochloride;
1-(4-Phenoxy-benzenesulfonyl)-4-piperazin-1-yl-1H-pyrrolo(3,2-c)pyridine hydrochloride;
1-(Phenylsulfonyl)-4-piperazin-1-yl-1H-pyrrolo[3,2-c]pyridine hydrochloride;
1-[(4-Chlorophenyl)sulfonyl]-4-piperazin-1-yl-1H-pyrrolo[3,2-c]pyridine hydrochloride;
1-[(4-Methoxyphenyl)sulfonyl]-4-piperazin-1-yl-1H-pyrrolo[3,2-c]pyridine hydrochloride;
1-[(2-Methoxy-5-methylphenyl)sulfonyl]-4-piperazin-1-yl-1H-pyrrolo[3,2-c]pyridine hydrochloride; and
4-Piperazin-1-yl-1- {[2-(trifluoromethyl)phenyl]sulfonyl}-1H-pyrrolo[3,2-c]pyridine hydrochloride.

Preferred compounds of the formula (VI) are

N-(4-Methylphenyl)-4-(4-methylpiperazin-1-yl)thieno[3,2-c]pyridine-2-sulfonamide hydrochloride;
2-Bromo-4-(4-methyl-piperazin-1-yl)-thieno[3,2-c]pyridine-3-sulfonic acid p-tolylamide hydrochloride;
4-(4-Methylpiperazin-1-yl)-n-phenylthieno[3,2-c]pyridine-2-sulfonamide hydrochloride;
4-Piperazin-1-yl-thieno[3,2-c]pyridine-2-sulfonic acid (3-fluoro-5-trifluoromethyl-phenyl)-amide hydrochloride;
4-Piperazin-1-yl-thieno[3,2-c]pyridine-2-sulfonic acid (4-chloro-phenyl)-amide hydrochloride;
4-Piperazin-1-yl-thieno[3,2-c]pyridine-2-sulfonic acid (4-isopropyl-phenyl)-amide hydrochloride;
4-Piperazin-1-yl-thieno[3,2-c]pyridine-2-sulfonic acid *p*-tolylamide hydrochloride;
4-(4-Methylpiperazin-1-yl)-*N*-(2-cyclohex-1-en-1-ylethyl) thieno [3,2-*c*] pyridine-2-sulfonamide hydrochloride;
2-(4-(4-Methylpiperazin-1-yl) thieno [3,2-c] pyridin-2-ylsulfonyl)-1,2,3,4-tetrahydroisoquinoline hydrochloride;
4-(4-Methylpiperazin-1-yl)-*N*- (2-thien-2-ylethyl) thieno [3,2-c] pyridine-2-sulfonamide hydrochloride;
4-(4-Methylpiperazin-1-yl)-*N*- [1-(1-naphthyl) ethyl] thieno [3,2-c] pyridine-2-sulfonamide hydrochloride;
4-(4-Methylpiperazin-1-yl)-*N*- (4-hexylphenyl) thieno [3,2-*c*] pyridine-2-sulfonamide hydrochloride;
*N*- (3-Chlorobenzyl)-4-(4-methylpiperazin-1-yl) thieno [3,2-c] pyridine-2-sulfonamide;
4-(4-Methylpiperazin-1-yl)-*N*- [1-(4-fluorophenyl) ethyl] thieno [3,2-*c*] pyridine-2-sulfonamide hydrochloride;
*N*- (2,3-Difluorobenzyl)-4-(4-methylpiperazin-1-yl) thieno [3,2-c] pyridine-2-sulfonamide hydrochloride;
4-(4-Methylpiperazin-1-yl)-*N*- (4-chloro-2, 5-dimethoxyphenyl) thieno [3,2-c] pyridine-2-sulfonamide hydrochloride;
2-Bromo-4- (4-methylpiperazin-1-yl)-*N*-(2-cyclohex-1-en-1-ylethyl) thieno [3,2-c] pyridine-3-sulfonamide hydrochloride;
2-Bromo-4- (4-methylpiperazin-1-yl)-*N* [(1S)-1-(2-naphthyl) ethyl] thieno [3,2-c] pyridine-3-sulfonamide hydrochloride;
2-Bromo-4- (4-methylpiperazin-1-yl)-*N*-[1-(4-fluorophenyl) ethyl] thieno [3,2-c] pyridine-3-sulfonamide hydrochloride;
2-Bromo-4- (4-methylpiperazin-1-yl)-*N* (2,4,5-trimethoxyphenyl) thieno [3,2-c] pyridine-3-sulfonamide;

N-(3,4-Dichlorophenyl)-4-piperazin-1-ylthieno[3,2-c]pyridine-2-sulfonamide hydrochloride;
N-(2,4-Difluorophenyl)-4-piperazin-1-ylthieno [3,2-c]pyridine-2-sulfonamide hydrochloride;
4-Piperazin-1-yl-N-[-3-(trifluoromethyl)phenyl]thieno[3,2-c]pyridine-2-sulfonamide hydrochloride;
N-(3-Ethylphenyl)-4-piperazin-1-ylthieno[3,2-c]pyridine-2-sulfonamide hydrochloride;
N-(3,4-Dimethoxyphenyl)-4-piperazin-1-ylthieno[3,2-c]pyridine-2-sulfonamide hydrochloride;
N-(4-Bromo-2-methylphenyl)-4-piperazin-1-ylthieno[3,2-c]pyridine-2-sulfonamide hydrochloride;
2-(4-Piperazin-1-yl-thieno[3,2-c]pyridine-2-sulfonyl)-1,2,3,4-tetrahydro-isoquinoline hydrochloride;
4-Piperazin-1-yl-thieno[3,2-c]pyridine-2-sulfonic acid (2-thiophen-2-yl-ethyl)-amide hydrochloride;
4-Piperazin-1-yl-thieno[3,2-c]pyridine-2-sulfonic acid (4-chloro-2,5-dimethoxy-phenyl)-amide hydrochloride;
4-Piperazin-1-yl-thieno[3,2-c]pyridine-2-sulfonic acid phenethyl-amide hydrochloride;
4-Piperazin-1-yl-thieno[3,2-c]pyridine-2-sulfonic acid (2,6-diethyl-phenyl)-amide hydrochloride;
4-Piperazin-1-yl-thieno[3,2-c]pyridine-2-sulfonic acid (3-phenyl-propyl)-amide hydrochloride;
4-Piperazin-1-yl-thieno[3,2-c]pyridine-2-sulfonic acid (3,3-diphenyl-propyl)-amide hydrochloride;
4-Piperazin-1-yl-thieno[3,2-c]pyridine-2-sulfonic acid [2-(5-methoxy-1H-indol-3-yl)-ethyl]-amide hydrochloride;
4-Piperazin-1-yl-thieno[3,2-c]pyridine-2-sulfonic acid 4-trifluoromethyl-benzylamide hydrochloride;
4-Piperazin-1-yl-thieno[3,2-c]pyridine-2-sulfonic acid benzyl-ethyl-amide hydrochloride; N-(3-Ethylphenyl)-4-piperazin-1-ylthieno[3,2-c]pyridine-3-sulfonamide hydrochloride;
N-(4-Isopropylphenyl)-4-piperazin-1-ylthieno [3,2-c]pyridine-3-sulfonamide hydrochloride;
N-(4-Methylphenyl)-4-(pyrrolidin-3-yloxy)thieno[3,2-c]pyridine-2-sulfonamide hydrochloride;
N-(4-Methylphenyl)-4-(piperidin-4-yloxy)thieno[3,2-c]pyridine-2-sulfonamide hydrochloride;
N-(2,3-Difluorobenzyl)-4-piperazin-1-ylthieno[3,2-c]pyridine-2-sulfonamide hydrochloride;
N-(3-Chlorobenzyl)-4-piperazin-1-ylthieno[3,2-c]pyridine-2-sulfonamide hydrochloride;
4-Piperazin-1-yl-thieno[3,2-c]pyridine-2-sulfonic acid phenylamide hydrochloride;
4-Piperazin-1-yl-thieno[3,2-c]pyridine-2-sulfonic acid (4-tert-butyl-phenyl)-amide hydrochloride;
4-(4-Methyl-piperazin-1-yl)-thieno[3,2-c]pyridine-2-sulfonic acid phenylamide hydrochloride;
4-(4-Methyl-piperazin-1-yl)-thieno[3,2-c]pyridine-2-sulfonic acid (3-chloro-phenyl)-amide hydrochloride;
2-Bromo-4-(4-methyl-piperazin-1-yl)-thieno[3,2-c]pyridine-3-sulfonic acid phenylamide hydrochloride;
4-(4-Methyl-piperazin-1-yl)-thieno[3,2-c]pyridine-3-sulfonic acid (4-methylphenyl)-amide hydrochloride; and
N-Phenyl-7-piperazin-1-ylthieno[2,3-c]pyridine-2-sulfonamide hydrochloride.

Preferred compounds of the formula (VII) are

N-(4-methylphenyl)-4-piperazin-1-ylfuro[3,2-c]pyridine-2-sulfonamide hydrochloride;
N-phenyl-4-piperazin-1-ylfuro[3,2-c]pyridine-2-sulfonamide hydrochloride; and N-phenyl-7-piperazin-1-ylfuro[2,3-c]pyridine-2-sulfonamide hydrochloride.

A preferred compound of the formula (VIII) is 4-Piperazin-1-yl-thiazolo[4,5-c]pyridine-2-sulfonic acid phenylamide hydrochloride.

Preferred compounds of the formula (IX) are

N-(4-methylphenyl)-4-piperazin-1-yl- 1H-pyrrolo[3,2-c]pyridine-2-sulfonamide hydrochloride;
N-phenyl-4-piperazin-1-yl-1H-pyrrolo[3,2-c]pyridine-2-sulfonamide hydrochloride; and
N-phenyl-7-piperazin-1-yl-1H-pyrrolo[2,3-c]pyridine-2-sulfonamide hydrochloride.

Another object of the present invention is a process for the preparation of a compound above, wherein

P is

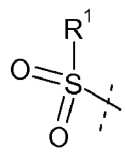

, said method comprising the steps of:

preparation of the heteroaromatic 5-member ring fused halogen-substituted pyridine, reduction of an aromatic nitro group; aromatic nucleophilic substitution with a thiol via a diazointermediate; oxidation of the thiol derivative to a

sulphone; introduction of a halogen atom by electrophilic aromatic substitution; aromatic nucleophilic substitution of the halogen with a diamine.

Another object of the present invention is a process for the preparation of a compound above, wherein

P is

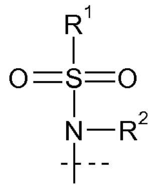

,

said method comprising the steps of: preparation of the heteroaromatic 5-member ring fused pyridine; introduction of a carboxylic moiety; conversion of the carboxylic moiety to amine by Curtius rearrangement; reaction of the amine group with a sulphonylchloride.

Another object of the present invention is a process for the preparation of a compound above, wherein

P is

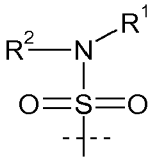

,

said method comprising the steps of: preparation of the heteroaromatic 5-member ring fused pyridine; introduction of sulfonylchloride moiety by nucleophilic addition; reaction of sulphonylchloride moiety with an aniline to obtained a sulfonamide; aromatic nucleophilic substitution of the chloro with a diamine.

All diastereomeric forms possible (pure enantiomers, tautomers, racemic mixtures and unequal mixtures of two enantiomers) are within the scope of the invention. Such compounds can also occur as cis- or trans-, *E*- or *Z*- double bond isomer forms. All isomeric forms are contemplated.

The compounds of the formulae (I) to (XII) may be used as such or, where appropriate, as pharmacologically acceptable salts (acid or base addition salts) thereof.

The pharmacologically acceptable addition salts as mentioned above are meant to comprise the therapeutically active non-toxic acid and base addition salt forms that the compounds are able to form. Compounds that have basic properties can be converted to their pharmaceutically acceptable acid addition salts by treating the base form with an appropriate acid. Exemplary acids include inorganic acids, such as hydrogen chloride, hydrogen bromide, hydrogen iodide, sulphuric acid, phosphoric acid; and organic acids such as acetic acid, propanoic acid, hydroxyacetic acid, lactic acid, pyruvic acid, glycolic acid, maleic acid, malonic acid, oxalic acid, benzenesulphonic acid, toluenesulphonic acid, methanesulphonic acid, trifluoroacetic acid, fumaric acid, succinic acid, malic acid, tartaric acid, citric acid, salicylic acid, p-aminosalicylic acid, pamoic acid, benzoic acid, ascorbic acid and the like. Exemplary base addition salt forms are the sodium, potassium, calcium salts, and salts with pharmaceutically acceptable amines such as, for example, ammonia, alkylamines, benzathine, and amino acids, such as, e.g. arginine and lysine. The term addition salt as used herein also comprises solvates which the compounds and salts thereof are able to form, such as, for example, hydrates, alcoholates and the like.

For clinical use, the compounds of the invention are formulated into pharmaceutical formulations for oral, rectal, parenteral or other mode of administration. Pharmaceutical formulations are usually prepared by mixing the active substance, or a pharmaceutically acceptable salt thereof, with conventional pharmaceutical excipients. The formulations can be further prepared by known methods such as granulation, compression, microencapsulation, spray coating, etc. The formulations may be prepared by conventional methods in the dosage form of tablets, capsules, granules, powders, syrups, suspensions, suppositories or injections. Liquid formulations may be prepared by dissolving or suspending the active substance in water or other suitable vehicles. Tablets and granules may be coated in a conventional manner.

Another object of the present invention is a compound above for use in therapy.

Another object of the present invention is a compound above for use in the treatment or prophylaxis of a 5-$HT_6$ receptor related disorder, such as obesity, type II diabetes, and/or disorders of the central nervous system, to

achieve reduction of body weight and of body weight gain.

Another object of the present invention is a compound above for use in the treatment or prophylaxis of disorders of the central nervous system.

Another object of the present invention is a compound above, and for the case when ring D is a pyrrole ring, P is of the formula (c) and $R^3$ is of the formula

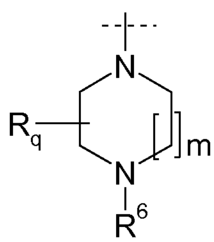 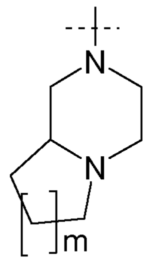 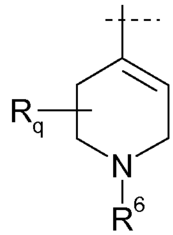 or 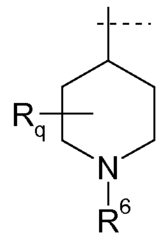

substituted in position 3 on the pyrrole ring, for use in the treatment or prophylaxis of type II diabetes.

Another object of the present invention is a compound above, and for the case when ring D is a pyrrole ring, P is of the formula (c) and $R^3$ is of the formula

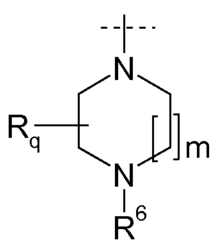 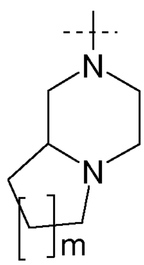 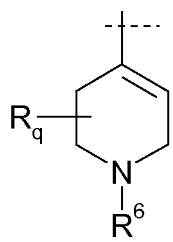 or 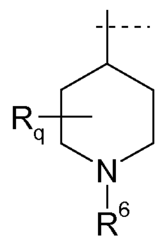

substituted in position 3 on the pyrrole ring, for use in the treatment or prophylaxis of obesity, to achieve reduction of body weight and of body weight gain.

Another object of the present invention is a pharmaceutical formulation comprising a compound above as an active ingredient, in combination with a pharmaceutically acceptable diluent or carrier.

Another object of the present invention is a pharmaceutical formulation comprising a compound above for use in the treatment or prophylaxis of a 5-$HT_6$ receptor related disorder, such as obesity, type II diabetes, and/or disorders of the central nervous system, to achieve reduction of body weight and of body weight gain.

Another object of the present invention is a compound above as an active ingredient, for use in the treatment or prophylaxis of disorders of the central nervous system.

Another object of the present invention is a pharmaceutical formulation comprising a compound above, and for the case when ring D is a pyrrole ring, P is of the formula (c) and $R^3$ is of the formula

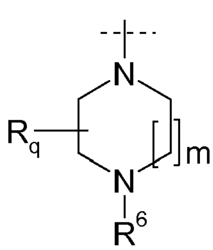 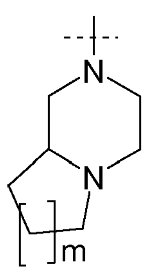 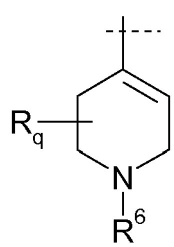 or 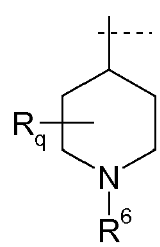

substituted in position 3 on the pyrrole ring, as an active ingredient, for use in the treatment or prophylaxis of type II diabetes.

Another object of the present invention is a pharmaceutical formulation comprising a compound above, and for the case when ring D is a pyrrole ring, P is of the formula (c) and $R^3$ is of the formula

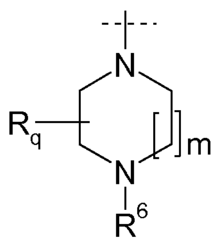
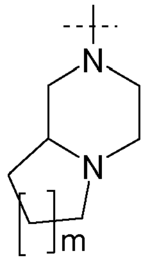
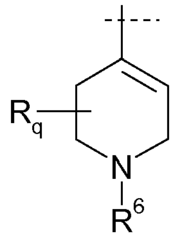
or
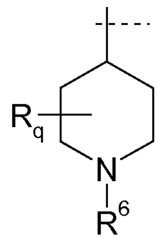

substituted in position 3 on the pyrrole ring, as an active ingredient, for use in the treatment or prophylaxis of obesity, to achieve reduction of body weight and of body weight gain.

Another object of the present invention is a method for the treatment or prophylaxis of a 5-$HT_6$ receptor related disorder, such as obesity, type II diabetes, and/or disorders of the central nervous system, to achieve reduction of body weight and of body weight gain, which comprises administering to a subject (e.g., a mammal, a human, a horse, a dog, or a cat) in need of such treatment an effective amount of one or more compounds of any of the formulae described above, their salt forms or compositions that include the compounds or their salt forms.

Another object of the present invention is a method for the treatment or prophylaxis of disorders of the central nervous system, which comprises administering to a subject in need of such treatment an effective amount of one or more compounds of any of the formulae described above, their salt forms or compositions that include the compounds or their salt forms.

Another object of the present invention is a method for the treatment or prophylaxis of type II diabetes, which comprises administering to a subject in need of such treatment an effective amount of one or more compounds of any of the formulae described above, their salt forms or compositions that include the compounds or their salt forms, and for the case when ring D is a pyrrole ring, P is of the formula (c) and $R^3$ is of the formula

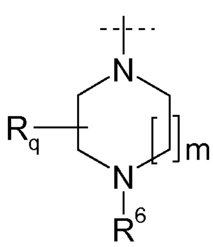
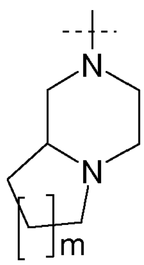
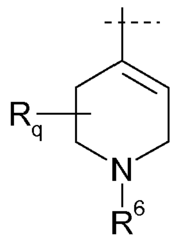
or
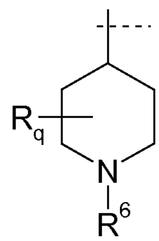

substituted in position 3 on the pyrrole ring.

Another object of the present invention is a method for the treatment or prophylaxis of obesity, which comprises administering to a subject in need of such treatment an effective amound of one or more compounds of any of the formulae described above, their salt forms or compositions that include the compounds or their salt forms, and for the case when ring D is a pyrrole ring, P is of the formula (c) and $R^3$ is of the formula

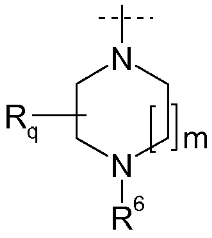 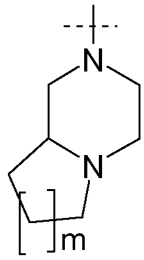 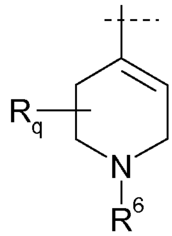 or 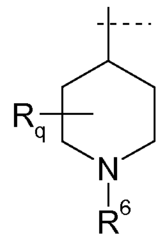

substituted in position 3 on the pyrrole ring.

Another object of the present invention is a method for modulating 5-$HT_6$ receptor activity, comprising administering to a subject in need thereof an effective amount of one or more compounds of any of the formulae described above, their salt forms or compositions that include the compounds or their salt forms.

Another object of the present invention is the use of one or more compounds of any of the formulae described above, their salt forms or compositions that include the compounds or their salt forms, for the manufacture of a medicament for use in the treatment or prophylaxis of a 5-$HT_6$ receptor related disorder, such as obesity, type II diabetes, and/or disorders of the central nervous system, to achieve reduction of body weight and of body weight gain.

Another object of the present invention is the use of one or more compounds of any of the formulae described above, their salt forms or compositions that include the compounds or their salt forms for the manufacture of a medicament for use in the treatment or prophylaxis of disorders of the central nervous system.

Another object of the present invention is the use of one or more compounds of any of the formulae described above, their salt forms or compositions that include the compounds or their salt forms, and for the case when ring D is a pyrrole ring, P is of the formula (c) and $R^3$ is of the formula

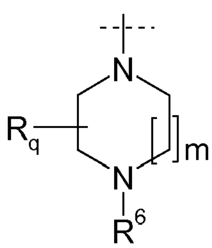 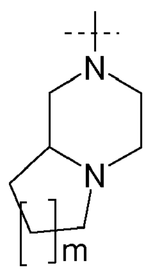 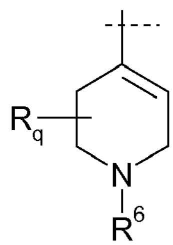 or 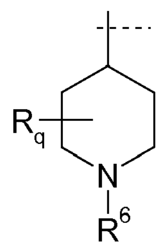

substituted in position 3 on the pyrrole ring, for the manufacture of a medicament for use in the treatment or prophylaxis of type II diabetes.

Another object of the present invention is the use of one or more compounds of any of the formulae described above, their salt forms or compositions that include the compounds or their salt forms, and for the case when ring D is a pyrrole ring, P is of the formula (c) and $R^3$ is of the formula

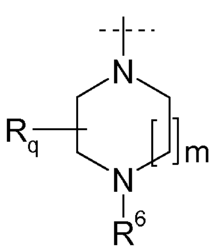 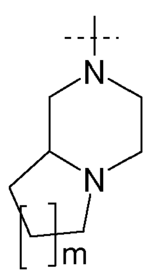 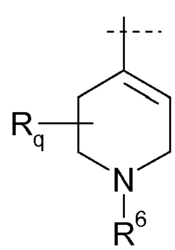 or 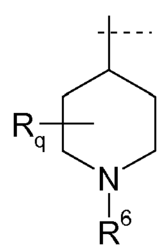

substituted in position 3 on the pyrrole ring, for the manufacture of a medicament for use in the treatment or prophylaxis

of obesity, to achieve reduction of body weight and of body weight gain.

The methods delineated herein can also include the step of identifying that the subject is in need of treatment of obesity, type II diabetes, or disorders of the central nervous system, or in need of reducing body weight and of body weight gain.

The invention further relates to cosmetic use of one or more compounds of any of the formulae described herein, for causing loss of weight, as well as cosmetic compositions containing said compounds.

Still further, the invention relates to a non-therapeutic metod for impriving the bodily appearance of a mammal, including a human, in which the method comprises orally administering to said mammal one or more compounds of any of the formulae described herein.

"An effective amount" refers to an amount of a compound that confers a therapeutic effect on the treated subject. The therapeutic effect may be objective (i.e., measurable by some test or marker) or subjective (i.e., subject gives an indication of or feels an effect).

For clinical use, the compounds of the invention are formulated into pharmaceutical formulations for oral, rectal, parenteral or other mode of administration. Usually the amount of active compounds is between 0.1-95% by weight of the preparation, preferably between 0.2-20% by weight in preparations for parenteral use and preferably between 1 and 50% by weight in preparations for oral administration.

The typical daily dose of the active substance varies within a wide range and will depend on various factors such as, for example, the individual requirement of each patient and the route of administration. In general, oral and parenteral dosages will be in the range of 5 to 1000 mg per day of active substance, preferably 50 to 150 mg per day.

### *Processes for preparation*

In a further aspect the invention relates to methods of making compounds of any of the formulae herein comprising reacting any one or more of the compounds of the formulae delineated herein, including any processes delineated herein. The compounds of the formulae above may be prepared by, or in analogy with, conventional methods, and especially according to or in analogy with the following methods.

The chemicals used in the above-described synthetic route may include, for example, solvents, reagents, catalysts, protecting group and deprotecting group reagents. The methods described above may also additionally include steps, either before or after the steps described specifically herein, to add or remove suitable protecting groups in order to ultimately allow synthesis of the compounds of any of the formulae described above, their salt forms, or compositions that include the compounds or their salt forms. In addition, various synthetic steps may be performed in an alternate sequence or order to give the desired compounds. Synthetic chemistry transformations and protecting group methodologies (protection and deprotection) useful in synthesizing applicable compounds are known in the art and include, for example, those described in R. Larock, Comprehensive Organic Transformations, VCH Publishers (1989); T.W. Greene and P.G.M. Wuts, Protective Groups in Organic Synthesis, 2nd Ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof.

The specific examples below are to be construed as merely illustrative, and not limitative of the remainder of the disclosure in any way whatsoever. Without further elaboration, it is believed that one skilled in the art can, based on the description herein, utilize the present invention to its fullest extent. All publications cited herein are hereby incorporated by reference in their entirety.

### Methods

$^{1}H$ nuclear magnetic resonance (NMR) and $^{13}C$ NMR were recorded on a Bruker Advance DPX 400 spectrometer at 400.1 and 100.6 MHz, respectively. All spectra were recorded using residual solvent or tetramethylsilane (TMS) as internal standard. IR spectra were recorded on a Perkin-Elmer Spectrum 1000 FT-IR spectrophotometer. Ionspray mass spectrometry (MS) spectra were obtained on a Perkin-Elmer API 150EX mass spectrometer. Accurate mass measurements were performed on a Micromass LCT dual probe. Preparative HPLC/MS was performed on a Waters/Micromass Platform ZQ system equipped with System A: ACE 5 C8 column (19x50mm), eluents: MilliQ water, MeCN and MilliQ/MeCN/0.1%TFA and system B: Xterra MS C18, 5 $\mu$m column (19x50mm), eluents: MilliQ water, MeCN and $NH_4HCO_3$ (100mM). Analytical HPLC were performed on Agilent 1100, column: ACE 3 C8 (system A) or column: YMC-Pack (system B), eluents: MilliQ/0.1%TFA and MeCN. Elemental analyses were performed on a Vario El instrument. Preparative flash chromatography was performed on Merck silica gel 60 (230-400 mesh).

**Table 4**

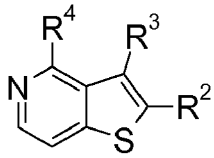

| EXAMPLE | | $R^2$ | $R^3$ | $R^4$ |
|---|---|---|---|---|
| 41 | N-(4-Methylphenyl)-4-(4-methylpiperazin-1-yl)thieno[3,2-c] pyridine-2-sulfonamide hydrochloride | 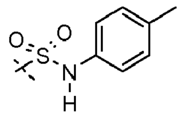 | H | 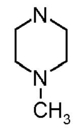 |
| 42 | 2-Bromo-4-(4-methyl-piperazin-1-yl)-thieno[3,2-c]pyridine-3-sulfonic acid p-tolylamide hydrochloride | Br | 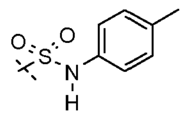 | 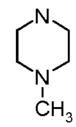 |
| 43 | 4-(4-Methylpiperazin-1-yl)-N-phenylthieno[3,2-c]pyridine-2-sulfonamide hydrochloride | 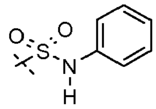 | H | 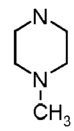 |
| 44 | 4-Piperazin-1-yl-thieno[3,2-c] pyridine-2-sulfonic acid (3-fluoro-5-trifluoromethyl-phenyl)-amide hydrochloride | 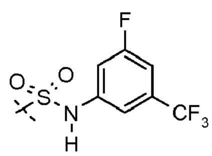 | H | 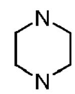 |
| 45 | 4-Piperazin-1-yl-thieno[3,2-c] pyridine-2-sulfonic acid (4-chloro-phenyl)-amide hydrochloride | 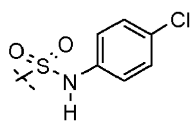 | H | 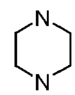 |
| 46 | 4-Piperazm-1-yl-thieno[3,2-c] pyridme-2-sulfonic acid (4-isopropyl-phenyl)-amide hydrochloride | 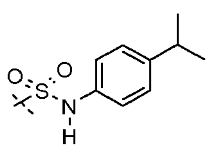 | H | 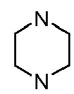 |
| 47 | 4-Piperazin-1-yl-thieno[3,2-c] pyridine-2-sulfonic acid *p*-tolylamide hydrochloride | 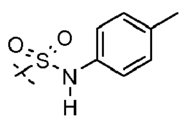 | H | 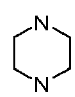 |
| 48 | 4-(4-Methylpiperazin-1-yl)-*N*-(2-cyclohex-1-en-1-ylethyl) thieno [3,2-c] pyridine-2-sulfonamide hydrochloride | 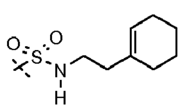 | H | 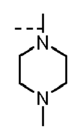 |
| 49 | 2-(4-(4-Methylpiperazin-1-yl) thieno [3,2-c] pyridin-2-ylsulfonyl)-1,2,3,4-tetrahydroisoquinoline hydrochloride | 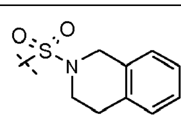 | H | 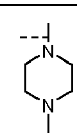 |

(continued)

| EXAMPLE | | $R^2$ | $R^3$ | $R^4$ |
|---|---|---|---|---|
| 50 | 4-(4-Methylpiperazin-1-yl)-*N*- (2-thien-2-ylethyl) thieno [3,2-c] pyridine-2-sulfonamide hydrochloride | 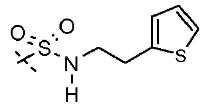 | H | 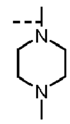 |
| 51 | 4-(4-Methylpiperazin-1-yl)-*N*-[1-(1-naphthyl) ethyl] thieno [3,2-c] pyridine-2-sulfonamide hydrochloride | 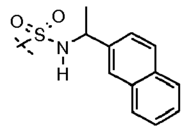 | H | 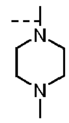 |
| 52 | 4-(4-Methylpiperazin-1-yl)-*N*-(4-hexylphenyl) thieno [3,2-c] pyridine-2-sulfonamide hydrochloride | 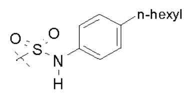 | H | 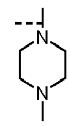 |
| 53 | *N*-(3-Chlorobenzyl)-4-(4-methylpiperazin-1-yl) thieno [3,2-c] pyridine-2-sulfonamide | 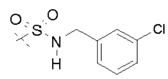 | H | 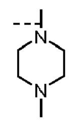 |
| 54 | 4-(4-Methylpiperazin-1-yl)-*N*-[1-(4-fluorophenyl) ethyl] thieno [3,2-c] pyridine-2-sulfonamide hydrochloride | 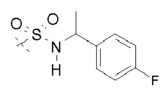 | H | 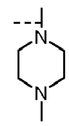 |
| 55 | *N*-(2,3-Difluorobenzyl)-4-(4-methylpiperazin-1-yl) thieno [3,2-c] pyridine-2-sulfonamide hydrochloride | 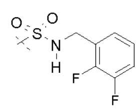 | H | 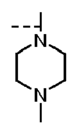 |
| 56 | 4-(4-Methylpiperazin-1-yl)-*N*- (4-chloro-2, 5-dimethoxyphenyl) thieno [3,2-c] pyridine-2-sulfonamide hydrochloride | 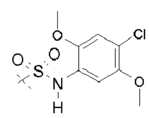 | H | 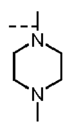 |
| 57 | 2-Bromo-4- (4-methylpiperazin-1-yl)-*N*-(2-cyclohex-1-en-1-ylethyl) thieno [3,2-c] pyridine-3-sulfonamide hydrochloride | Br | 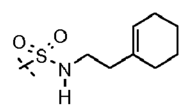 | 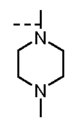 |
| 58 | 2-[2-Bromo-4-(4-methyl-piperazin-1-yl)-benzo[b]thiophene-3-sulfonyl]-1 , 2,3,4- tetrahydro- isoquinoline | Br | 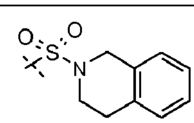 | 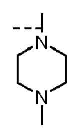 |
| 59 | 2-Bromo-4- (4-methylpiperazin-1-yl)-*N*-[1-(4-fluorophenyl) ethyl] thieno [3,2-c] pyridine-3-sulfonamide hydrochloride | Br | 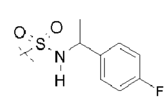 | 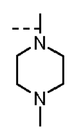 |
| 60 | 2-Bromo-4- (4-methylpiperazin-1-yl)-*N*-(4-chloro)-(2, 5-dimethoxyphenyl) thieno [3,2-c] pyridine-3-sulfonamide hydrochloride | Br | 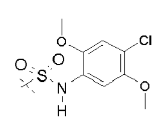 | 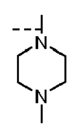 |

(continued)

| EXAMPLE | | $R^2$ | $R^3$ | $R^4$ |
|---|---|---|---|---|
| 61 | N-(3,4-Dichlorophenyl)-4-piperazin-1-ylthieno[3,2-c]pyridine-2-sulfonamide hydrochloride | 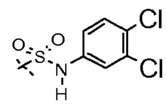 | H | 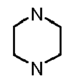 |
| 62 | N-(2,4-Difluorophenyl)-4-piperazin-1-ylthieno[3,2-c]pyridine-2-sulfonamide hydrochloride | 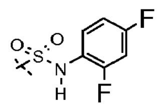 | H | 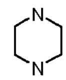 |
| 63 | 4-Piperazin-1-yl-N-[-3-(trifluoromethyl)phenyl]thieno[3,2-c]pyridine-2-sulfonamide hydrochloride | 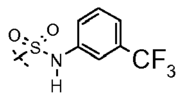 | H | 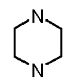 |
| 64 | N-(3-Ethylphenyl)-4-piperazin-1-ylthieno[3,2-c]pyridine-2-sulfonamide hydrochloride | 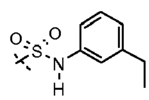 | H | 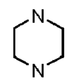 |
| 65 | N-(3,4-Dimethoxyphenyl)-4-piperazin-1-ylthieno[3,2-c]pyridine-2-sulfonamide hydrochloride | 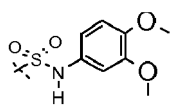 | H | 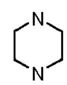 |
| 66 | N-(4-Bromo-2-methylphenyl)-4-piperazin-1-ylthieno[3,2-c]pyridine-2-sulfonamide hydrochloride | 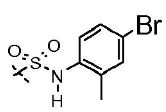 | H | 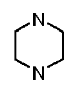 |
| 67 | 2-(4-Piperazin-1-yl-thieno[3,2-c] pyridine-2-sulfonyl)-1,2,3,4-tetrahydro-isoquinoline hydrochloride | 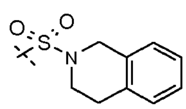 | H | 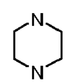 |
| 68 | 4-Piperazin-1-yl-thieno[3,2-c] pyridine-2-sulfonic acid (2-thiophen-2-yl-ethyl)-amide hydrochloride | 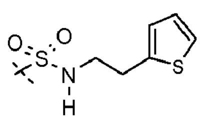 | H | 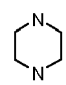 |
| 69 | 4-Piperazin-1-yl-thieno[3,2-c] pyridine-2-sulfonic acid (4-chloro-2,5-dimethoxy-phenyl)-amide hydrochloride | 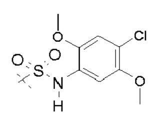 | H | 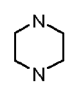 |
| 70 | 4-Piperazin-1-yl-thieno[3,2-c] pyridine-2-sulfonic acid phenethyl-amide hydrochloride | 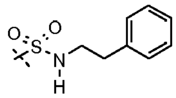 | H | 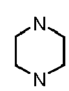 |
| 71 | 4-Piperazin-1-yl-thieno[3,2-c] pyridine-2-sulfonic acid (2,6-diethyl-phenyl)-amide hydrochloride | 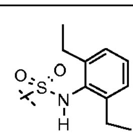 | H | 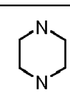 |

(continued)

| EXAMPLE | | $R^2$ | $R^3$ | $R^4$ |
|---|---|---|---|---|
| 72 | 4-Piperazin-1-yl-thieno[3,2-c] pyridine-2-sulfonic acid (3-phenyl-propyl)-amide hydrochloride | 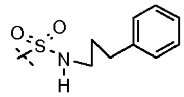 | H | 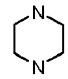 |
| 73 | 4-Piperazin-1-yl-thieno[3,2-c] pyridine-2-sulfonic acid (3,3-diphenyl-propyl)-amide hydrochloride | 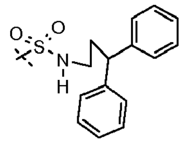 | H | 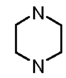 |
| 74 | 4-Piperazin-1-yl-thieno[3,2-c] pyridine-2-sulfonic acid [2-(5-methoxy-1H-indol-3-yl)-ethyl]-amide hydrochloride | 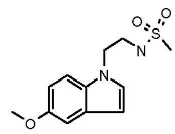 | H | 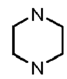 |
| 75 | 4-Piperazin-1-yl-thieno[3,2-c] pyridine-2-sulfonic acid 4-trifluoromethyl-benzylamide hydrochloride | 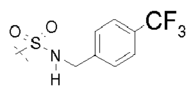 | H | 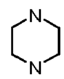 |
| 76 | 4-Piperazin-1-yl-thieno[3,2-c] pyridine-2-sulfonic acid benzyl-ethyl-amide hydrochloride | 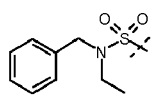 | H | 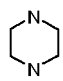 |
| 77 | N-(3-Ethylphenyl)-4-piperazin-1-ylthieno[3,2-c]pyridine-3-sulfonamide hydrochloride | H | 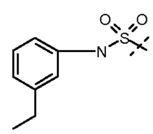 | 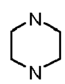 |
| 78 | N-(4-Isopropylphenyl)-4-piperazin-1-ylthieno[3,2-c]pyridine-3-sulfonamide hydrochloride | H | 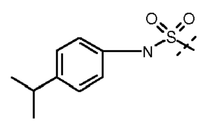 | 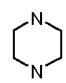 |
| 79 | N-(4-Methylphenyl)-4-(pyrrolidin-3-yloxy)thieno[3,2-c]pyridine-2-sulfonamide hydrochloride | [?] 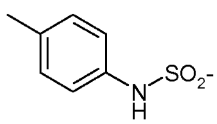 | H | 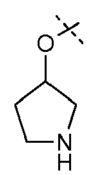 |
| 80 | N-(4-Methylphenyl)-4-(piperidin-4-yloxy)thieno[3,2-c]pyridine-2-sulfonamide hydrochloride | [?] 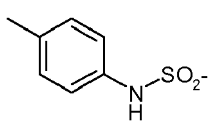 | H | 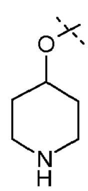 |

(continued)

| EXAMPLE | | $R^2$ | $R^3$ | $R^4$ |
|---|---|---|---|---|
| 81 | N-(2,3-Difluorobenzyl)-4-piperazin-1-ylthieno[3,2-c]pyridine-2-sulfonamide hydrochloride | [?] 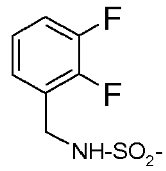 | H | 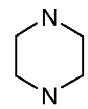 |
| 82 | N-(3-Chlorobenzyl)-4-piperazin-1-ylthieno[3,2-c]pyridine-2-sulfonamide hydrochloride | [?] 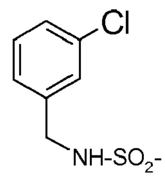 | H | 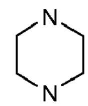 |

## Scheme 5

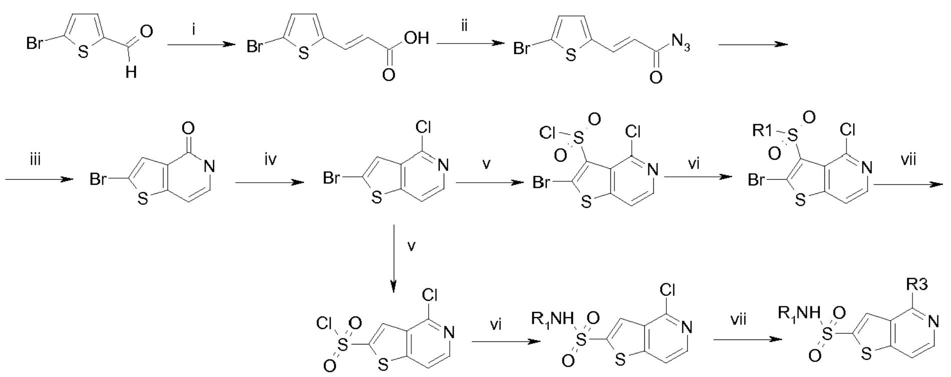


Legend to Scheme 5: i) Malonic acid, pyridine, piperidine, heat; ii) ethylchloroformate, acetone, $NaN_3$ -10 ˚C; iii) diphenyl ether, 220 ˚C; iv) $POCl_3$, heat; v) gas $SO_2$, n-BuLi, N-chlorosuccinimide, $CH_2Cl_2$; vi) $R^1$-$NH_2$, pyridine; vii) $HR^3$, $K_2CO_3$, DMSO, heat.

INTERMEDIATE 47

**(2*E*)-3-(5-Bromothien-2-yl) acrylic acid**

Malonic acid (44.40 g, 426.7 mmol) was added to a mixture of 5-bromothiophene-2-carbaldehyde (50 g, 261.7 mmol), piperidine (2.84 mL) and pyridine (150 mL). The mixture was refluxed for 1 h at 80˚C and than at 100 ˚C over night. The volatiles were evaporated and the residue was dissolved in water and acidified with hydrochloric acid (pH 2). The crude product was crystallized in ethanol. Yield: 55.24 g (90.5 %). $^1H$ NMR (270 MHz, $CH_3OH$-$d_4$) δ ppm 6.14 (d, *J*=15.83 Hz, 1 H) 7.11-7.16 (m, 2 H) 7.68 (d, *J*=16.36 Hz, 1 H); MS 233.1 $(M - H)^+$; Purity (HPLC) 94 %.

INTERMEDIATE 48

**(2*E*)-3-(5-Bromothien-2-yl)acryloyl azide**

Thionyl chloride (1.04 mL) was added to a solution of (2E)-3-(5-bromothien-2-yl) acrylic acid (1.04 g, 4.46 mmol) in chloroform (20 mL) and the mixture was refluxed for 2h at 75˚C and than used in the next step. The above solution was added drop wise to a stirred suspension of sodium azide (0.58 g, 8.93 mmol), dioxane (3 mL) and water

(3 mL) in an ice bath. After 10 min a precipitate appeared which was filtered off and washed with water. The residue was dissolved in dichloromethane, dried with $MgSO_4$, filtered and the solvent was removed to afford: 0.96 g (83.4 %). $^1$H NMR (270 MHz $CH_3OH$-$d_4$) δ ppm 6.20 (d, *J*=15.57 Hz, 1 H) 7.15-7.25 (m, 2 H) 7.80 (d, *J*=15.57 Hz, 1 H); MS 258.1 $(M - H)^+$; Purity (HPLC) 65 %.

INTERMEDIATE 49

**2-Bromothieno [3,2-c] pyridin-4 (5*H*)-one**

A solution of (2E)-3-(5-bromothien-2-yl) acryloyl azide (18.00 g, 69.7 mmol) solved in dichloromethane (100 mL) was added dropwise to diphenyl ether (90 mL) at 150 ˚C. The temperature was increased to 220˚C for 1h. The mixture was cooled to room temperature followed by the addition of ether. The solid precipitated and was separated by filtration. Yield: 13.58 g (84.6 %). $^1$H NMR (270 MHz, DMSO-$d_6$) δ ppm 6.82 (d, *J*=7.13 Hz, 1 H) 7.27 (d, *J*=6.86 Hz, 1 H) 7.54 (s, 1 H) 11.55 (s, 1 H); MS 230.1 $(M - H)^+$; Purity (HPLC) 92%.

INTERMEDIATE 50

**2-Bromo-4-chloro-thieno [3,2-c] pyridine**

Phosphorus oxychloride (4.08 g, 26.6 mmol) was added dropwise to 2-bromothieno [3,2-c] pyridin-4 (5*H*)-one (2.04 g, 8.87 mmol) at 0 ˚C. The mixture was heated at 135 ˚C for 2.5h, then carefully poured over ice water. The precipitated was collected by filtration and dried to yield 1.78 g (80.7 %) of title product. $^1$H NMR (270 MHz, $CH_3OH$-$d_4$) δ ppm 7.67 (d, 1 H) 7.88 (dddd, *J*=6.33 Hz, 2 H) 8.19 (d, *J*=5.54 Hz, 1 H); MS 248.0 $(M - H)^+$ ; Purity (HPLC) 100 %.

INTERMEDIATE 51 and INTERMEDIATE 52

**4-Chlorothieno [3,2-c] pyridine-2-sulfonyl chloride and 2-bromo-4-chlorothieno [3,2-*c*] pyridine-3-sulfonyl chloride**

n-Butyl lithium (1.5 mL, 2.4 mmol) was added to 2-bromo-4-chlorothieno [3,2-c] pyridine (0.5 g, 2 mmol) dissolved in dry THF (15 ml) at -78˚C under nitrogen. The mixture was stirred for 40 min. The above solution was added to a dry ether saturated with $SO_2$ (gas) at -78˚C. The mixture was warmed to room temperature, followed by the addition of ether. The precipitate was separated by filtration. The two title products were obtained and taken to the next step without further purification as follows: N-chlorosuccinimide (2.07 g, 10.3 mmol) was added to [(4-chlorothieno [3,2-c] pyridin-2-yl) sulfonyl] lithium and [(2-bromo-4-chlorothieno [3,2-c] pyridin-3-yl) sulfonyl] lithium in dichloromethane (150 mL) at 0 ˚C. The mixture was heated at 60 ˚C for 2h, extracted with water (3 x 50 mL). The organic phase was separated, dried with $MgSO_4$, filtrated and the volatiles were eliminated by vacuum distillation. The crude products were used in the next step without further purification.

INTERMEDIATE 53 and INTERMEDIATE 54

**4-Chloro-2-thieno [3,2-c] pyridine-2- sulfonic acid p-tolylamide and 2-bromo-4-chloro-thieno [3,2-c] pyridine-3-sulfonic acid p-tolylamide**

p-Toludine (30 mg, 2.87 mmol) was added to a solution of 4-chlorothieno [3,2-c] pyridine-2-sulfonyl chloride and 2-bromo-4-chlorothieno [3,2-c] pyridine-3-sulfonyl chloride (0.07 g, 0.26 mmol) in dichloromethane and pyridine (0.19 mL). The reaction was stirred at room temperature for 2h. The solvent was removed and the crude mixture was taken to the next step without further purification.

EXAMPLE 41 and EXAMPLE 42

**4-(4-Methyl-piperazin-1-yl)-thieno[3,2-c]pyridine-2-sulfonic acid p-tolylamide hydrochloride and 2-bromo-4-(4-methyl-piperazin-1-yl)-thieno[3,2-c]pyridine-3-**sulfonic acid p-tolylamide hydrochloride

A mixture of 4-chloro-2-thieno [3,2-c] pyridine-2- sulfonic acid p-tolylamide and 2-bromo-4-chloro-thieno[3,2-c]pyridine-3-sulfonic acid p-tolylamide (70 mg, 0.21 mmol) in DMSO (2 mL), 1-methyl piperazine (0.344 mL, 3.1 mmol) and $K_2CO_3$ (28.5 mg, 0.21 mmol) was heated to 100˚C over night. The reaction mixture was dissolved in water and extracted with ethyl acetate (3 x 10 mL). The organic layers were collected and the solvent was removed. The products

were purified by HPLC to afford 1.9 mg of 4-(4-Methyl-piperazin-1-yl)-thieno[3,2-c]pyridine-2-sulfonic acid p-tolylamide. The free base was converted into the hydrochloride salt by treatment with HCl in ether: $^1$H NMR (270 MHz, Methanol-$d_4$) δ ppm 2.26 (s, 3H) 2.98 (s, 3 H) 3.40-3.55 (m, 8 H) 7.02-7.10 (m, 6 H) 7.55 (d, *J*=5.81 Hz, 1 H) 7.69 (s, 1 H) 8.13 (d, *J*=5.81 Hz, 1 H); LC-MS 403 $(M + H)^+$; Purity (LC-MS) 92 % and 3.8 mg 2-bromo-4-(4-methyl-piperazin-1-yl)-thieno [3,2-c]pyridine-3-sulfonic acid p-tolylamide. The free base was converted into the hydrochloride salt by treatment with HCl in ether: $^1$H NMR (270 MHz, Methanol-$d_4$) δ ppm 2.21 (s, 1H) 3.00 (d, 3H) 3.50-3.77 (m, 8 H) 7.00-7.10 (m, 6 H) 7.63 (d, *J*=5.81 Hz, 1 H) 8.19 (d, *J*=5.81 Hz, 1 H); LC-MS 481 $(M + H)^+$; Purity (LC-MS) 98 %.

**Reaction of sulfonyl chloride with amines (Method H)**

To a solution of the amine (1.3 equiv.) and pyridine (8 equiv.) in DCM was added the sulfonyl chloride (1 equiv.) and the reaction mixture was stirred over night. After addition of Trisamine™ (ca 2 equiv.), the mixture was gently shaken for additional 3 h. The suspension was then filtered through a short silica plug by the aid of DCM and ethyl acetate. The solvent was evaporated, and the residue was dissolved in DCM and washed with 1 M aqueous HCl (2 times). The combined organic phases were dried ($MgSO_4$), filtered, and the solvent was removed to give the sulfonamide product. In cases of low-purity material, the products were purified by silica gel flash chromatography. The products are used in the next step (Procedure B).

**Coupling with aromatic amines (Method I)**

To the reaction mixtures from the Method H, dissolved in DMSO (2mL), amines (15 equiv.) and $K_2CO_3$ (1 equiv.) are added. The reactions are stirred at 100˚C for 24 and than concentrated. The products are purified by LC-MS. The solvents are removed under vacuum by SpeedVac and purified by preparative LC/MS. The products that were not pure enough (Purity ≤90%) were purified by preparative chromatography using acetonitrile-water gradients containing 0.1% triflouroacetic acid. After HPLC analysis fractions that were ≥ 90% pure were collected and concentrated. Deprotection of the amine in the piperazine was performed by first dissolving the substance in methanol and adding portions of 1M HCl/ether. The reactions are analyzed by TLC. The solvents were concentrated under vacuum by a SpeedVac.

**Deprotection of BOC-group (Method L)**

The sulfone or sulfonamide derivative (prepared by Methods H and I) were dissolved in a small amount of MeOH/DCM 1:1 and treated with an excess of 1 M HCl in diethyl ether. Stirring at ambient temperature overnight resulted in a precipitate which were collected by filtration to give the products as their corresponding hydrochloride salts.

EXAMPLE 43

**4-(4-Methylpiperazin-1-yl)-N-phenylthieno[3,2-c]pyridine-2-sulfonamide** hydrochloride

The synthesis was preformed essentially as described in Method H-L. Yield: 8.1 mg (33.8 %). $^1$H NMR (270 MHz, $CH_3OH$-$d_4$) δ ppm 8.13 (d, *J*=5.81 Hz, 1 H) 7.67 (s, 1 H) 7.54 (d, *J*=5.81 Hz, 1 H) 7.55-7.53 (m, 5H) 2.97 (s, 3 H) (4H obscured by solvent signal); LC-MS 389 $(M - H)^+$; Purity (HPLC) 100 %.

EXAMPLE 44

**4-Piperazin-1-yl-thieno[3,2-c]pyridine-2-sulfonic acid (3-fluoro-5-trifluoromethylphenyl)-amide hydrochloride**

4-[2-(3-Fluoro-5-trifluoromethyl-phenylsulfamoyl)-thieno[3,2-c]pyridin-4-yl]-piperazine-1-carboxylic acid tert-butyl ester (0.235 mmol, 1 equiv.) was used as the thienopyridine in Method H-L. Yield: 25.7 mg HPLC: $t_R$= 3.395 (System: 5% to 50 % ACN in 3 min, C8), Purity: 100 %, LC/MS: $t_R$= 1.375 (System: 30% to 60% ACN in 1.5 min, Hypersil BDS), Purity: 99 %. MS: 461 (M+1) $^1$H NMR (270 MHz, $CH_3OH$-$d_4$) δ ppm 3.47 (m, 4 H) 3.53 (s, 1 H) 3.87 (m, 4 H) 7.21 (m, 1 H) 7.29 (m, 1 H) 7.33 (s, 1 H) 7.66 (d, *J*=6.33 Hz, 1 H).

EXAMPLE 45

**4-Piperazin-1-yl-thieno[3,2-c]pyridine-2-sulfonic acid (4-chloro-phenyl)-amide hydrochloride**

4-Chloro-thieno[3,2-c]pyridine-2-sulfonic acid (3-fluoro-5-trifluoromethyl-phenyl)-amide (0.208 mmol, 1 equiv.) was used as the thienopyridine in Method H-L. Yield: 7.2 mg HPLC: $t_R$= 3.039 (System: 5 % to 50 % ACN in 3 min, C8),

Purity: 100%, LC/MS: $t_R$= 0.905 (System: 30 % to 60 % ACN in 1.5 min, Hypersil BDS), Purity: 97%. MS: 409 (M+1). $^1$H NMR (270 MHz, $CH_3OH-d_4$) δ ppm 3.50 (m, 4 H) 3.91 (m, 4 H) 7.25 (m, 4 H) 7.71 (dd, *J*=6.33, 0.53 Hz, 1 H) 7.96 (d, *J*=0.79 Hz, 1 H) 8.04 (d, *J*=6.33 Hz, 1 H).

EXAMPLE 46

**4-Piperazin-1-yl-thieno[3,2-c]pyridine-2-sulfonic acid (4-isopropyl-phenyl)-amide hydrochloride**

4-Chloro-thieno[3,2-c]pyridine-2-sulfonic acid (4-isopropyl-phenyl)-amide (0.201 mmol, 1 equiv.) was used as the thienopyridine in Method H-L. Yield: 6.9 mg HPLC: $t_R$= 3.255 (System: 5 % to 50 % ACN in 3 min, C8), Purity: 95%, LC/MS: $t_R$= 1.255 (System: 30% to 60 % ACN in 1.5 min, Hypersil BDS), Purity: 98 %. MS: 417 (M+1). $^1$H NMR (270 MHz, $CH_3OH-d_4$) δ ppm 1.18 (d, *J*=6.86 Hz, 6 H) 2.83 (m, 2 H) 3.52 (m, 4 H) 4.00 (m, 4 H) 7.14 (m, 3 H) 7.75 (d, *J*=6.60 Hz, 1 H) 8.02 (m, 1 H).

EXAMPLE 47

**4-Piperazin-1-yl-thieno[3,2-c]pyridine-2-sulfonic acid *p*-tolylamide hydrochloride**

To a solution of 4-chloro-thieno[3,2-c]pyridine-2-sulfonyl chloride (0.640 g, 2.39 mmol) in DCM (20 mL) was added pyridine (1.9 mL, 23.9 mmol) followed byp-tolylamine ( 0.307 g, 2.86 mmol). The reaction mixture was stirred at room temperature for 16 hours. The mixture was concentrated and re-dissolved in DMSO (10 mL), piperazine-1-carboxylic acid *tert*-butyl ester (1.34 g, 7.17 mmol) and $K_2CO_3$ (0.989 g, 7.17 mmol) were added. The mixture was stirred at 100 ˚C for 16 hours and then concentrated. The crude reaction mixture was dissolved in EtOAc (100 mL) and washed with brine (2 x 50 mL). The organic phase was dried ($Na_2SO_4$) and concentrated. The crude intermediate was purified by column chromatography on silica using EtOAc/*n*-pentane (1:1) as eluent. The intermediate was dissolved in EtOAc/ MeOH and diethyl ether saturated with HCl (g) was added. The mixture was stirred at room temperature for 16 hours. The precipitate was collected by filtration and washed with diethyl ether/n-pentane to give 0.475 g of the crude product. Purification by preparative reversed phase HPLC gave 0.133 g of the pure product: $^1$H NMR (DMSO-$d_6$, 25 ˚C, 270.17 MHz) δ 10.61 (br s, 1H), 9.23 (br s, 2H), 8.13 (d, J = 5.80 Hz, 1H), 7.91 (s, 1H); 7.67 (d, J = 5.80 Hz, 1H), 7.09-7.07 (m, 4H), 3.68-3.59 (m, 4H), 3.33-3.22 (m, 4H), 2.20 (s, 3H) ; m/z (posESI) 399 (M+H).

EXAMPLE 48

**4-(4-Methylpiperazin-1-yl)-*N*-(2-cyclohex-1-en-1-ylethyl) thieno [3,2-c] pyridine-2-sulfonamide hydrochloride**

The synthesis was preformed essentially as described in Method H-L. Yield: 25.6 mg $^1$H NMR (270 MHz, DMSO-$d_6$) δ ppm 10.49-10.48 (m, 1H) 8.23-7.95 (m, 3H) 7.72-7.71 (m, 1H) 5.34-5.33 (m, 1H) 4.14-4.11 (m, 2H) 3.53-3.51 (m, 2H) 3.29-3.25 (m, 2H) 2.98-2.97 (m, 2H) 2.85 (s, 3H) 2.04-1.81 (m, 4H) 1.57-1.15 (m, 8H); LC-MS 420.17 $(M - H)^+$; Purity (LC-MS) 97 %.

EXAMPLE 49

**2-(4-(4-Methylpiperazin-1-yl) thieno [3,2-c] pyridin-2-ylsulfonyl)-1,2,3,4-tetrahydroisoquinoline hydrochloride**

The synthesis was preformed essentially as described in Method H-L. Yield: 15.5 mg $^1$H NMR (270 MHz, DMSO-$d_6$) δ ppm 10.49-10.48 (m, 1H) 8.17-8.15 (m, 1H) 7.86-7.85 (m, 1H) 7.70-7.65 (m, 2H) 7.28-7.12 (m, 3H) 3.97-3.94 (m, 2H) 3.87-3.85 (m, 2H) 3.25-3.18 (m, 2H) 2.84 (s, 3H) 1.67-1.65 (m, 2H) 3.51-3.34 (6H obscured by solvent signal); LC-MS 428.13 $(M - H)^+$; Purity (LC-MS) 99 %.

EXAMPLE 50

**4-(4-Methylpiperazin-1-yl)-*N*-(2-thien-2-ylethyl) thieno [3,2-c] pyridine-2-sulfonamide hydrochloride**

The synthesis was preformed essentially as described in Method H-L. Yield: 29.5 mg $^1$H NMR (270 MHz, DMSO-$d_6$) δ ppm 10.28-10.27 (m, 1H) 8.34-8.33 (m, 1H) 8.19-8.17 (m, 1H) 8.01-8.00 (m, 1H) 7.71-7.69 (m, 1H) 7.31-7.30 (m, 1H) 6.91-6.87 (m, 1H) 4.13-4.10 (m, 2H) 3.53-3.51 (m, 2H) 3.29-3.25 (m, 2H) 3.17-3.16 (m, 2H) 2.99-2.95 (m 4H) 2.86 (s, 3H); LC-MS 422.09 $(M - H)^+$; Purity (LC-MS) 99%.

EXAMPLE 51

**4-(4-Methylpiperazin-1-yl)-*N*-[1-(1-naphthyl)ethyl] thieno [3,2-c] pyridine-2-sulfonamide hydrochloride**

The synthesis was preformed essentially as described in Method H-L. 20.1 mg $^1$H NMR (270 MHz, DMSO-$d_6$) δ ppm 10.28-10.27 (m, 1H) 8.85-8.84 (m, 1H) 8.02-8.01 (m, 1H) 7.67-7.60 (m, 4H) 7.53-7.50 (m, 2H) 7.39-7.36 (m, 2H) 4.72-4.70 (m, 1H) 3.87-3.84 (m, 1H) 3.72-3.70 (m, 1H) 3.23-3.13 (m, 4H) 2.84 (s, 3H) 1.42-1.40 (m 3H); LC-MS 466.15 (M - H)$^+$; Purity (LC-MS) 99 %.

EXAMPLE 52

**4-(4-Methylpiperazin-1-yl)-*N*-(4-hexylphenyl) thieno [3,2-*c*] pyridine-2-sulfonamide hydrochloride**

The synthesis was preformed essentially as described in Method H-L. 8.0 mg $^1$H NMR (270 MHz, DMSO-$d_6$) δ ppm 10.39-10.38 (m, 1H) 8.16-8.15 (m, 1H) 7.90-7.89 (m, 1H) 7.66-7.65 (m, 1H) 7.09-7.08 (m, 4H) 4.00-3.98 (m, 2H) 3.51-3.48 (m, 2H) 3.26-3.22 (m, 2H) 2.85 (s, 3H) 2.49-2.45 (m, 2H) 1.48-1.46 (m, 2H) 1.24-1.22 (m, 8H) 0.82-0.81 (m, 3H); LC-MS 472.20 (M - H)$^+$; Purity (LC-MS) 98 %.

EXAMPLE 53

***N*-(3-Chlorobenzyl)-4-(4-methylpiperazin-1-yl) thieno [3,2-c] pyridine-2-sulfonamide** hydrochloride

The synthesis was preformed essentially as described in Method H-L. 30.7 mg $^1$H NMR (270 MHz, DMSO-$d_6$) δ ppm 10.25-10.24 (m, 1H) 8.78-8.77 (m, 1H) 8.18-8.17 (m, 1H) 7.91-7.90 (m, 1H) 7.68-7.67 (m, 1H) 7.26-7.19 (m, 3H) 4.21-4.20 (m, 2H) 4.08-4.05 (m, 2H) 3.54-3.51 (m, 2H) 3.28-3-23 (m, 2H) 2.87 (s, 3H) 2.84-2.60 (2H obscured by solvent signal); LC-MS 436.08 (M - H)$^+$; Purity (LC-MS) 94 %.

EXAMPLE 54

**4-(4-Methylpiperazin-1-yl)-*N*- [1-(4-fluorophenyl) ethyl] thieno [3,2-c] pyridine-2-sulfonamide hydrochloride**

The synthesis was preformed essentially as described in Method H-L. 32.9 mg $^1$H NMR (270 MHz, DMSO-$d_6$) δ ppm 10.16-10.15 (m, 1H) 8.75-8.73 (m, 1H) 7.63-7.62 (m, 2H) 7.25-7.24 (m, 2H) 6.91-6.88 (m, 2H) 4.58-4.55 (m, 1H) 4.02-3.95 (m, 2H) 3.55-3.53 (m, 2H) 3.25-3-21 (m, 2H) 2.68 (s, 3H) 1.31-1.30 (m, 3H) 2.70-2.64 (2H obscured by solvent signal); LC-MS 434.12 (M - H)$^+$; Purity (LC-MS) 92 %.

EXAMPLE 55

***N*-(2,3-Difluorobenzyl)-4-(4-methylpiperazin-1-yl) thieno [3,2-c] pyridine-2-**sulfonamide hydrochloride

The synthesis was preformed essentially as described in Method H-L. 26.7 mg $^1$H NMR (270 MHz, DMSO-$d_6$) δ ppm 10.36-10.35 (m, 1H) 8.82-8.81 (m, 1H) 7.96-7.95 (m, 1H) 7.69-7.68 (m, 1H) 7.27-7.10 (m, 2H) 4.26-4.25 (m, 2H) 4.11-4.08 (m, 2H) 3.54-3.52 (m, 2H) 3.28-3-24 (m, 2H) 2.68 (s, 3H) 2.86-2.60 (2H obscured by solvent signal); LC-MS 438.10 (M - H)$^+$; Purity (LC-MS) 93 %.

EXAMPLE 56

**4-(4-Methylpiperazin-1-yl)-*N*-(4-chloro- 2, 5-dimethoxyphenyl) thieno [3,2-c]** pyridine-2-sulfonamide hydrochloride

The synthesis was preformed essentially as described in Method H-L.14.6 mg $^1$H NMR (270 MHz, DMSO-$d_6$) δ ppm 10.27-10.26 (m, 1H) 10.14-10.13 (m, 1H) 8.18-8.17 (m, 1H) 7.83-7.82 (m, 1H) 7.69-7.68 (m, 1H) 7.09-7.07 (m, 2H) 4.00 (s 2H) 3.76-3.75 (m, 2H) 3.51-3.48 (m, 2H) 3.24-3.22 (m, 2H) 2.85 (s, 3H); LC-MS 482.08 (M - H)$^+$ ; Purity (LC-MS) 95 %.

EXAMPLE 57

**2-Bromo-4-(4-methylpiperazin-1-yl)-*N*-(2-cyclohex-1-en-1-ylethyl) thieno [3,2-c] pyridine-3-sulfonamide hydrochloride**

The synthesis was preformed essentially as described in Method H-L. 4.6 mg $^1$H NMR (270 MHz, DMSO-$d_6$) δ ppm 10.30-10.29 (m, 1H) 8.31-8.27 (m, 2H) 7.89-7.88 (m, 1H) 5.27-5.26 (m, 1H) 3.68-3.52 (m, 4H) 3.05-3.04 (m, 2H) 2.88-2.87 (m, 3H) 2.04-2.03 (m, 2H) 2.77-1.81 (m, 2H) 1.54-1.15 (m, 10H); LC-MS 498.08 $(M - H)^+$; Purity (LC-MS) 93 %.

EXAMPLE 58

**2-[2-Bromo-4-(4-methyl-piperazin-1-yl)-benzo[b]thiophene-3-sulfonyl]-1 ,2,3,4-tetrahydro-isoquinoline hydrochloride**

The synthesis was preformed essentially as described in Method H-L. Yield: 3.7 mg $^1$H NMR (270 MHz, DMSO-$d_6$) δ ppm 10.30-10.29 (m, 1H) 9.24-9.22 (m, 1H) 8.09-8.08 (m, 1H) 7.67-7.35 (m, 7H) 4.69-4.66 (m, 1H) 2.86-2.85 (m, 3H) 1.50-1.48 (m, 3H) 3.23-2.51 (8H obscured by solvent signal); LC-MS 544.06 $(M - H)^+$; Purity (LC-MS) 92 %.

EXAMPLE 59

**2-Bromo-4-(4-methylpiperazin-1-yl)-*N*-[1-(4-fluorophenyl) eth-2-yl] thieno [3,2-*c*] pyridine-3-sulfonamide hydrochloride**

The synthesis was preformed essentially as described in Method H-L. Yield: 4.3 mg $^1$H NMR (270 MHz, DMSO-$d_6$) δ ppm 10.35-10.34 (m, 1H) 9.16-9.14 (m, 1H) 8.23-8.22 (m, 1H) 7.80-7.79 (m, 1H) 7.26-7.25 (m, 1H) 4.55-4.52 (m, 1H) 3.54-3.52 (m, 2H) 2.88-2.87 (m, 3H) 1.38-1.36 (m, 3H) 3.17-2.83 (6H obscured by solvent signal); LC-MS 512.04 $(M - H)^+$; Purity (LC-MS) 93 %.

EXAMPLE 60

**2-Bromo-4-(4-methylpiperazin-1-yl)-*N*-(4-chloro)-(2, 5-dimethoxyphenyl) thieno [3,2-c] pyridine-3-sulfonamide hydrochloride**

The synthesis was preformed essentially as described in Method H-L. Yield: 0.7 mg, LC-MS 561.91$(M - H)^+$; Purity (LC-MS) 95%.

EXAMPLE 61

**N-(3,4-dichlorophenyl)-4-piperazin-1-ylthieno[3,2-c]pyridine-2-sulfonamide hydrochloride**

3,4-Dichloroaniline (0.49 mmol), was dissolved in acetonitrile (1 mL) and pyridine (0.440 mL, 4.03 mmol) was added to a solution of 4-chlorothieno [3,2-c] pyridine-2-sulfonyl chloride (0.445 mmol) dissolved in acetonitrile (1 mL). The reaction was shaken for 1h, controlled with HPLC and the solvent was removed. The crude product was used in the next step without further purification. To the reaction mixture from the previous step, dissolved in DMSO (1mL), piperazine (15 equiv.) and $K_2CO_3$ (1 equiv.) was added. The reaction was stirred at 100 ˚C for 24 and than concentrated. The product was purified by LC-MS and gave 5.8 mg (2.6 %) of the title product. $^1$H NMR (270 MHz, $CH_3OH$-$d_4$) δ ppm 8.07-8.05 (m, 2 H) 7.73 (d, *J*=6.60 Hz, 1 H) 7.46-7.41 (m, 2 H) 7.16 (dd, *J*=8.71, 2.38 Hz, 1 H) 3.94-3.90 (m, 4 H) 3.92 (m, 4 H) 3.53-3.50 (m, 4 H); LC-MS 443 $(M - H)^+$; Purity (HPLC) 95%.

EXAMPLE 62

**N-(2,4-Difluorophenyl)-4-piperazin-1-ylthieno [3,2-c] pyridine-2-sulfonamide hydrochloride**

The synthesis was preformed essentially as described in Method H-L. Yield: 4.3 mg (2.1 %). $^1$H NMR (270 MHz, $CH_3OH$-$d_4$) δ ppm 8.22 (s, 1 H) 8.07-8.01 (m, 2H) 7.79-7.72 (m, 1H) 7.55-7.47 (m, 1H) 7.04-6.94 (m, 2H) 4.00-3.96 8m, 4H) 3.53-3.43 (m, 4H) 2.66 (s, 1H); LC-MS 411 $(M - H)^+$; Purity (HPLC) 98 %.

EXAMPLE 63

**4-Piperazin-1-yl-N-[-3-(trifluoromethyl)phenyl]thieno[3,2-c]pyridine-2-sulfonamide hydrochloride**

The synthesis was preformed essentially as described in Method H-L. Yield: 2.6 mg (1.2 %). $^1$H NMR (270 MHz, $CH_3OH-d_4$) δ ppm 8.05 (d, *J*=6.60 Hz, 2 H) 7.81-7.60 (m, 3H) 7.50-7.47 (m, 2H) 3.94-3.90 (m, 4H) 3.56-3.49 (m, 4H); LC-MS 443 $(M - H)^+$; Purity (HPLC) 99 %.

EXAMPLE 64

**N-(3-Ethylphenyl)-4-piperazin-1-ylthieno[3,2-c]pyridine-2-sulfonamide hydrochloride**

The synthesis was preformed essentially as described in Method H-L. Yield: 1.4 mg (0.7 %). $^1$H NMR (270 MHz, $CH_3OH-d_4$) δ ppm 8.35 (s, 1H) 7.58-6.92 (m, 7H) 3.54-3.44 (m, 2H) 3.01-2.95 (m, 4H) 2.66 (s, 1H) 2.18-2.01 (m, 3H)); LC-MS 403 $(M - H)^+$; Purity (HPLC) 100 %.

EXAMPLE 65

**N-(3,4-Dimethoxyphenyl)-4-piperazin-1-ylthieno[3,2-c]pyridine-2-sulfonamide hydrochloride**

The synthesis was preformed essentially as described in Method H-L. Yield: 7.7 mg (3.6 %). $^1$H NMR (270 MHz, $CH_3OH-d_4$) δ ppm 8.04 (d, *J*=6.60 Hz, 1 H) 7.77-7.75 /m, 2H) 6.85-6.83 (m, 2H) 6.68-6.83 (m, 1H) 3.87-3.85 (m, 4H) 3.77-3.75 (m, 6H) 3.49-3.45 (m, 4H) 2.65 (s, 1H); LC-MS 435 $(M - H)^+$; Purity (HPLC) 98 %.

EXAMPLE 66

**N-(4-Bromo-2-methylphenyl)-4-piperazin-1-ylthieno[3,2-c]pyridine-2-sulfonamide hydrochloride**

The synthesis was preformed essentially as described in Method H-L. Yield: 12.2 mg (5.3 %). $^1$H NMR (270 MHz, $CH_3OH-d_4$) δ ppm 8.07 (d, *J*=6.33 Hz, 1 H) 7.86-7.79 (m, 2H) 7.40 (d, *J*=1.58 Hz, 1 H) 7.30-7.29 (m, 1H) 7.08 (d, *J*=8.71 Hz, 1 H) 3.96-3.92 (m, 4H) 3.53-3.51 (4H) 2.66 (s, 1H) 2.11 (s, 3H); LC-MS 467 $(M - H)^+$; Purity (HPLC) 90 %.

EXAMPLE 67

**2-(4-Piperazin-1-yl-thieno[3,2-c]pyridine-2-sulfonyl)-1,2,3,4-tetrahydro-isoquinoline hydrochloride**

The synthesis was preformed essentially as described in Method H-L from 4-[2-(3,4-dihydro-1H-isoquinoline-2-sulfonyl)-thieno[3,2-c]pyridin-4-yl]-piperazine-1-carboxylic acid *tert*-butyl ester (0.235 mmol, 1 equiv.). Yield: 4.0 mg. LC/MS: $t_R$= 0.801 (System: 30 % to 60 % ACN in 1.5 min, Hypersil BDS), Purity: 92%. MS: 415 (M+1) 1H NMR (270 MHz, DMSO-$d_6$) δ ppm 2.87 (t, *J*=5.81 Hz, 2 H) 3.30 (s, 4 H) 4.43 (s, 2 H) 7.15 (m, 4 H) 7.70 (d, *J*=5.54 Hz, 1 H) 8.12 (s, 1 H) 8.18 (d, *J=5.54* Hz, 1 H) 6 aliphatic protons were obscured by the water-peak in the spectra and so could not be analyzed.

EXAMPLE 68

**4-Piperazin-1-yl-thieno[3,2-c]pyridine-2-sulfonic acid (2-thiophen-2-yl-ethyl)-amide hydrochloride**

The synthesis was preformed essentially as described in Method H-L from 4-[2-(2-thiophen-2-yl-ethylsulfamoyl)-thieno[3,2-c]pyridin-4-yl]-piperazine-1-carboxylic acid *tert*-butylester (0.235 mmol, 1 equiv.). Yield: 8.7 mg. LC/MS: $t_R$= 0.430 (System: 30 % to 60 % ACN in 1.5 min, Hypersil BDS), Purity: 93 %. MS: 409 (M+1)$^1$H NMR (270 MHz, DMSO-$d_6$) δ ppm 2.25 (s, 1 H) 2.75 (s, 1 H) 2.96 (t, *J*=6.99 Hz, 1 H) 3.16 (q, *J*=6.51 Hz, 1 H) 3.31 (s, 4 H) 3.70 (s, 4 H) 6.90 (m, 1 H) 7.32 (t, *J*=5.54 Hz, 1 H) 7.70 (d, *J*=5.81 Hz, 1 H) 8.04 (d, *J*=1.85 Hz, 1 H) 8.18 (d, *J*=5.54 Hz, 1 H) 8.36 (m, 1 H) 9.05 (s, 1 H).

EXAMPLE 69

**4-Piperazin-1-yl-thieno[3,2-c]pyridine-2-sulfonic acid (4-chloro-2,5-dimethoxyphenyl)-amide hydrochloride**

The synthesis was preformed essentially as described in Method H-L from 4-[2-(4-chloro-2,5-dimethoxy-phenylsulfamoyl)-thieno[3,2-c]pyridin-4-yl]-piperazine-1-carboxylic acid *tert*-butyl ester (0.112 mmol, 1 equiv.) was used as the thienopyridine in Method C. Yield: 14.7 mg. LC/MS: $t_R$= 0.610 (System: 30 % to 60 % ACN in 1.5 min, YMC), Purity: 92 %. MS: 469 (M+1) $^1$H NMR (270 MHz, DMSO-$d_6$) δ ppm 3.17 (s, 1 H) 3.27 (s, 4 H) 3.38 (s, 3 H) 3.58 (d, *J*=4.22 Hz, 4 H) 3.77 (s, 3 H) 7.08 (s, 1 H) 7.69 (d, *J*=5.81 Hz, 1 H) 7.81 (s, 1 H) 8.16 (d, *J*=5.81 Hz, 1 H) 9.07 (s, 1 H) 10.17 (s, 1 H).

EXAMPLE 70

**4-Piperazin-1-yl-thieno[3,2-c]pyridine-2-sulfonic acid phenethyl-amide hydrochloride**

The synthesis was preformed essentially as described in Method H-L from 4-(2-phenethylsulfamoyl-thieno[3,2-c]pyridin-4-yl)-piperazine-1-carboxylic acid *tert*-butyl ester (0.112 mmol, 1 equiv.). Yield: 3.8 mg. LC/MS: $t_R$= 0.410 (System: 30% to 60% ACN in 1.5 min, YMC), Purity: 91 %. MS: 403 (M+1) $^1$H NMR (270 MHz, $CH_3OH$-$d_4$) δ ppm 1.44 (d, *J*=7.13 Hz, 2 H) 3.51 (d, J=4.75 Hz, 4 H) 3.54 (s, 2 H) 3.94 (m, 4 H) 7.05 (m, 4 H) 7.16 (m, 1 H) 7.62 (s, 1 H) 7.72 (d, *J*=6.60 Hz, 1 H) 8.00 (d, *J*=6.60 Hz, 1 H).

EXAMPLE 71

**4-Piperazin-1-yl-thieno[3,2-c]pyridine-2-sulfonic acid (2,6-diethyl-phenyl)-amide hydrochloride**

The synthesis was preformed essentially as described in Method H-L from 4-[2-(2,6-dethyl-phenylsulfamoyl)-thieno[3,2-c]pyridin-4-yl]-piperazine-1-carboxylic acid *tert*-butyl ester (0.112 mmol, 1 equiv.). Yield: 9.0 mg. LC/MS: $t_R$= 0.830 (System: 30 % to 60 % ACN in 1.5 min, YMC), Purity: 92 %. MS: 431 (M+1) $^1$H NMR (270 MHz, DMSO-D6) δ ppm 0.96 (t, *J*=7.52 Hz, 6 H) 2.25 (m, 1 H) 2.43 (s, 2 H) 2.75 (t, *J*=1.72 Hz, 1 H) 3.26 (s, 4 H) 3.62 (s, 4 H) 7.09 (s, 1 H) 7.12 (s, 1 H) 7.23 (m, 1 H) 7.73 (d, *J*=5.81 Hz, 1 H) 7.77 (s, 1 H) 8.19 (d, *J*=5.81 Hz, 1 H) 9.04 (s, 1 H) 9.95 (s, 1 H).

EXAMPLE 72

**4-Piperazin-1-yl-thieno[3,2-c]pyridine-2-sulfonic acid (3-phenyl-propyl)-amide hydrochloride**

The synthesis was preformed essentially as described in Method H-L from 4-[2-(3-phenyl-propylsulfamoyl)-thieno[3,2-c]pyridin-4-yl]-piperazine-1-carboxylic acid *tert*-butyl ester (0.112 mmol, 1 equiv.). Yield: 13.0 mg. LC/MS: $t_R$= 0.726 (System: 30 % to 60 % ACN in 1.5 min, YMC), Purity: 91 %. MS: 417 (M+1) $^1$H NMR (270 MHz, $CH_3OH$-$d_4$) δ ppm 1.82 (m, 2 H) 2.63 (m, 2 H) 3.04 (t, *J*=6.86 Hz, 2 H) 3.55 (s, 4H) 4.09 (s, 4H) 7.16 (m, 4 H) 7.82 (d, *J*=6.60 Hz, 1 H) 8.05 (d, *J*=6.33 Hz, 1 H) 8.14 (s, 1 H).

EXAMPLE 73

**4-Piperazin-1-yl-thieno[3,2-c]pyridine-2-sulfonic acid (3,3-diphenyl-propyl)-amide hydrochloric acid**

The synthesis was preformed essentially as described in Method H-L from 4-[2-(3,3-diphenyl-propylsulfamoyl)-thieno[3,2-c]pyridin-4-yl]-piperazine-1-carboxylic acid *tert*-butyl ester (0.112 mmol, 1 equiv.). Yield: 14.4 mg. LC/MS: $t_R$= 1.109 (System: 30 % to 60 % ACN in 1.5 min, YMC), Purity: 93 %. MS: 493 (M+1) $^1$H NMR (270 MHz, DMSO-$d_6$) δ ppm 2.20 (m, 2 H) 2.80 (m, 2 H) 3.29 (s, 4 H) 3.67 (d, *J*=5.01 Hz, 4 H) 4.01 (m, 1 H) 7.14 (m, 8 H) 7.71 (d, *J*=5.81 Hz, 1 H) 7.95 (s, 1 H) 8.18 (d, *J*=5.81 Hz, 1 H) 8.27 (m, 2 H) 9.13 (s, 2 H).

EXAMPLE 74

**4-Piperazin-1-yl-thieno[3,2-c]pyridine-2-sulfonic acid [2-(5-methoxy-1H-indol-3-yl)-ethyl]-amide hydrochloride**

The synthesis was preformed essentially as described in Method H-L from 4-{2-[2-(5-methoxy-1H-indol-3-yl)-ethylsulfamoyl]-thieno[3,2-c]pyridin-4-yl}-piperazine-1-carboxylic acid *tert*-butyl ester (0.112 mmol, 1 equiv.). Yield: 6.1 mg. LC/MS: $t_R$= 0.364 (System: 30 % to 60 % ACN in 1.5 min, YMC), Purity: 91 %. MS: 472 (M+1) $^1$H NMR (270 MHz, $CH_3OH$-$d_4$) δ ppm 2.85 (t, *J*=6.20 Hz, 2 H) 3.48 (t, *J*=6.20 Hz, 2 H) 3.55 (m, 4 H) 3.80 (s, 3 H) 4.02 (m, 4 H) 6.44

(dd, *J*=8.71, 2.37 Hz, 1 H) 6.80 (m, 2 H) 6.97 (s, 1 H) 7.64 (s, 1 H) 7.67 (s, 1 H) 7.97 (d, *J*=6.60 Hz, 1 H).

EXAMPLE 75

**4-Piperazin-1-yl-thieno[3,2-c]pyridine-2-sulfonic acid 4-trifluoromethyl-benzylamide hydrochloride**

The synthesis was preformed essentially as described in Method H-L from 4-[2-(4-trifluoromethyl-benzylsulfamoyl)-thieno[3,2-c]pyridin-4-yl]-piperazine-1-carboxylic acid *tert*-butyl ester (0.112 mmol, 1 equiv.) was used as the thienopyridine in Method C. Yield: 1.9 mg. LC/MS: $t_R$= 0.771 (System: 30% to 60% ACN in 1.5 min, YMC), Purity: 91%. MS: 457 (M+1) $^1$H NMR (270 MHz, $CH_3OH-d_4$) δ ppm 3.54 (m, 4 H) 3.98 (m, 4 H) 4.36 (s, 2 H) 7.49 (m, 4 H) 7.74 (d, *J*=6.86 Hz, 1 H) 8.02 (s, 1 H) 8.07 (d, *J*=6.60 Hz, 1 H).

EXAMPLE 76

**4-Piperazin-1-yl-thieno[3,2-c]pyridine-2-sulfonic acid benzyl-ethyl-amide hydrochloride**

The synthesis was preformed essentially as described in Method H-L from 4-[2-(benzyl-ethyl-sulfamoyl)-thieno [3,2-c]pyridin-4-yl]-piperazine-1-carboxylic acid *tert*-butyl ester (0.112 mmol, 1 equiv.). Yield: 6.4 mg. LC/MS: $t_R$= 0.930 (System: 30 % to 60 % ACN in 1.5 min, YMC), Purity: 95 %. MS: 417 (M+1) $^1$H NMR (270 MHz, $CH_3OH-d_4$) δ ppm 1.03 (t, J=7.13 Hz, 3 H) 3.37 (m, 2 H) 3.57 (s, 2 H) 3.75 (m, 2 H) 4.11 (s, 2 H) 4.50 (s, 2 H) 5.80 (s, 1 H) 7.32 (m, 5 H) 7.84 (d, *J*=6.60 Hz, 1 H) 8.07 (d, J=6.60 Hz, 1 H) 8.14 (s, 1 H).

INTERMEDIATE 55

***tert*-Butyl-4-(3-{[(3-ethylphenyl)amino]sulfonyl}thieno[3,2-c]pyridin-4-yl)piperazine-1-carboxylate**

Prepared from *tert*-butyl4-[3-(chlorosulfonyl)thieno[3,2-c]pyridin-4-yl]piperazine-1-carboxylate (90.0 mg, 0.215 mmol) and 3-ethylaniline (33.9 mg, 0.28 mmol) to give the title compound as an off-white solid (82.7 mg, 76 %). $^1$H NMR (400 MHz, $CDCl_3$) δ 1.03 (t, J = 7.5 Hz, 3 H), 1.48 (s, 9 H), 2.47 (q, J = 7.7 Hz, 2 H), 3.00-3.53 (m, 6 H), 4.02-4.44 (m, 2 H), 6.66 (d, J = 8.0 Hz, 1 H), 6.75 (s, 1 H), 6.66 (d, J = 8.0 Hz, 1 H), 7.02 (t, J = 7.8 Hz, 1 H), 7.67 (d, J = 5.5 Hz, 1 H), 8.24 (s, 1 H), 8.39 (d, J = 5.5 Hz, 1 H), 9.80 (s, 1 H). MS (ESI+) m/z 503.2 $(M+H)^+$. HPLC 97 %, $R_T$: 3.93 min (5-99 % MeCN over 3 min).

EXAMPLE 77

**N-(3-Ethylphenyl)-4-piperazin-1-ylthieno[3,2-c]pyridine-3-sulfonamide hydrochloride**

Prepared from *tert*-butyl 4-{[(3-ethylphenyl)amino]sulfonyl}thieno[3,2-c]pyridin-4-yl)piperazine-1-carboxylate (81.1 mg, 0.161 mmol) which afforded 19 mg (98 %) of the product as a white solid (38.0 mg, 54 %). $^1$H NMR (400 MHz, $CH_3OH-d_4$) δ 1.09 (t, J= 7.5 Hz, 3 H), 2.51 (q,J= 7.5 Hz, 2 H), 3.59 (br.s, 8 H), 6.89-6.92 (m, 3 H), 7.12-7.14 (m, 1 H), 8.06 (d, J = 6.0 Hz, 1 H), 8.36 (d, J = 6.0 Hz, 1 H), 8.49 (s, 1 H). MS (ESI+) *m/z* 403.2 $(M+H)^+$. HPLC 95%, $R_T$: 3.02 min (5-99% MeCN over 3 min).

INTERMEDIATE 56

***tert*-Butyl 4-(3-bromothieno [3,2-c] pyridin-4-yl)piperazine-1-carboxylate**

A mixture of 3-bromo-4-chlorothieno[3,2-c]pyridine (729 mg, 2.93 mmol), *tert*-butyl piperazine-1-carboxylate (1.64 g, 8.80 mmol) and $K_2CO_3$ (811 mg, 5.87 mmol) in DMSO (45 mL) was stirred for 5 days at 100 ˚C. After addition of $H_2O$ and ethyl acetate, the layers were separated. The water phase was extracted twice with ethyl acetate, and the combined organic phases were washed with water and brine and dried ($MgSO_4$). After filtration and removal of the solvent, the residue was purified by silica gel flash chromatography (pentane/ethyl acetate, 8:2) to give the product as a white powder (398 mg, 34 %). HPLC 99%, $R_T$: 3.27 min (5-99% MeCN over 3 min).$^1$H NMR (400 MHz, $CH_3OH-d_4$) δ 1.48 (s, 9 H), 3.21 (br.s, 4 H), 3.71 (s br., 4 H), 7.61 (d, *J*= 6.1 Hz, 1 H), 7.72 (s, 1 H), 8.08 (d, *J*= 5.6 Hz, 1 H). MS (ESI+) *m/z* 398.2 $(M+H)^+$.

INTERMEDIATE 57

**{4-[4-(*tert*-Butoxycarbonyl)piperazin-1-yl]thieno[3,2-c]pyridin-3-yl}sulfonyl)lithium**

To a suspension of *tert*-Butyl 4-(3-bromothieno[3,2-c]pyridin-4-yl)piperazine-1-carboxylate (4.055 g, 10.18 mmol) in diethyl ether (30 mL) at -78 °C under $N_2$ atmosphere was added dropwise an 1.6 M solution of *n*-BuLi in hexanes (9.5 mL, 15.2 mmol). After 1 h of stirring, a saturated solution of $SO_2$ in THF (25 mL) at -78 °C was transferred via a cannula to the mixture. The reaction was allowed to gradually increase to ambient temperature over night. The solvent was evaporated, and the residue was washed with several portions of diethyl ether and then dried under vacuum to give 4.094 g of an off-white solid consisting of 66 % of the title compound and 34 % of (n-butylsulfonyl)lithium as by-product. This mixture was used without any further purification in the next step. $^1$H NMR (400 MHz, $CH_3OH-d_4$) δ 1.48 (s, 9 H), 3.22 (br.s, 4 H), 3.72 (s br., 4 H), 7.60 (d, *J*= 5.5 Hz, 1 H), 8.06 (d, *J*= 5.5 Hz, 1 H), 8.14 (s, 1 H). MS (ESI+) *m/z* 384.0 $(M+H)^+$. HPLC $R_T$: 2.62 min (5-99% MeCN over 3 min).

INTERMEDIATE 58

***tert*-Butyl-4-[3-(chlorosulfonyl)thieno[3,2-c]pyridin-4-yl]piperazine-1-carboxylate**

To a suspension of ({4-[4-(*tert*-butoxycarbonyl)piperazin-1-yl]thieno[3,2-c]pyridin-3-yl}sulfonyl)lithium (2.751 g, 7.06 mmol (3.126 g of the crude product mixture)) in DCM (40 mL) at 0 °C was added *N*-chlorosuccinimide (1.338 g, 10.0 mmol). After 20 minutes, the temperature was raised to ambient, and the reaction mixture was stirred for an additional 2.5 h. The resulting product solution was washed with water, and the water phase was extracted with DCM. The combined organic extracts were washed with brine and dried over $MgSO_4$. After filtration and evaporation of the solvent, the residue was washed with several portions of pentane to yield the product as an off-white solid (2.024 g, 69 %). $^1$H NMR (400 MHz, $CH_3OH-d_4$) δ 1.47 (s, 9 H), 3.11 (br.s, 4 H), 3.2-4.3 (s br., 4 H), 7.68 (d, J = 5.5 Hz, 1 H), 8.45 (d, J = 5.5 Hz, 1 H), 8.60 (s, 1 H). MS (ESI+) m/z 418.2 $(M+H)^+$. HPLC 92%, $R_T$: 3.76 min (5-99% MeCN over 3 min).

INTERMEDIATE 59

***tert*-Butyl-4-(3-{[(4-isopropylphenyl)amino]sulfonyl}thieno[3,2-c]pyridin-4-yl)piperazine-1-carboxylate**

Prepared from *tert*-butyl4-[3-(chlorosulfonyl)thieno[3,2-c]pyridin-4-yl]piperazine-1-carboxylate (90.0 mg, 0.215 mmol) and 4-isopropylaniline (37.9 mg, 0.28 mmol) to give the title compound as an off-white solid (58.3 mg, 52 %). $^1$H NMR (400 MHz, $CDCl_3$) δ 1.12 (d,*J*= 7.0 Hz, 6 H), 1.47 (s, 9 H), 2.76 (sept., *J*= 6.9 Hz, 2 H), 3.01-3.53 (m, 6 H), 4.04-4.41 (m,2H), 6.79 (d,*J*= 8.5 Hz, 2H), 6.66 (d,*J*= 8.5 Hz, 2H), 7.69 (d,*J*= 5.5 Hz, 1H), 8.23 (s, 1H), 8.40 (d,*J*= 5.5 Hz, 1H), 9.90 (s, 1H). MS (ESI+) *m/z* 517.2 $(M+H)^+$. HPLC 97%, $R_T$: 4.01 min (5-99% MeCN over 3 min).

EXAMPLE 78

**N-(4-Isopropylphenyl)-4-piperazin-1-ylthieno[3,2-c]pyridine-3-sulfonamide hydrochloride**

Prepared from *tert*-butyl 4-(3-{[(4-isopropylphenyl)amino]sulfonyl}thieno[3,2-c]pyridin-4-yl)piperazine-1-carboxylate (60.0 mg, 0.116 mmol) which afforded 19 mg (98 %) of the product as a white solid (25.8 mg, 49 %) according to Method H-L. $^1$H NMR (400 MHz, $CH_3OH-d_4$) δ 1.16 (d, J = 7.0 Hz, 6 H), 2.81 (sept.,*J*= 6.8 Hz, 1 H), 3.59 (s, br., 8 H), 7.00 (d, J= 8.0 Hz, 2 H), 7.10 (d, J= 8.0 Hz, 2 H), 8.07 (s, br., 1 H), 8.37 (s, br., 1 H), 8.50 (s, 1 H). MS (ESI+) *m/z* 417.2 $(M+H)^+$. HPLC 94%, $R_T$: 3.14 min (5-99% MeCN over 3 min).

EXAMPLE 79

**N-(4-Methylphenyl)-4-(pyrrolidin-3-yloxy)thieno[3,2-c]pyridine-2-sulfonamide hydrochloride**

4-Chloro-*N*-(4-methylphenyl)thieno[3,2-*c*]pyridine-2-sulfonamide (60.0 mg, 0.17 mmol) in dry DMF (1 mL) and NaH (5,1 mg, 0.21 mmol) was added to pyrrolidin-3-ol (18.5 mg, 0.21 mmol) under nitrogen. The mixture was heated in the microwave at 200°C in 5 min. The product was purified by preparative HPLC. Yield: 29.9 mg (43.4 %). $^1$H NMR (270 MHz, $CH_3OH-d_4$) δ ppm 8.09 (s, 1H) 7.71 (d, *J*=6.93 Hz, 1 H) 7.46 (d, *J*=6.93 Hz, 1 H) 7.47-7.44 (m, 4H) 4.67 (d, *J*=3.22 Hz, 1 H) 3.97 (s, 2 H) 2.26 (s, 3H). LC-MS 390 (M - H)$^+$; Purity (HPLC) 99 %.

EXAMPLE 80

**N-(4-Methylphenyl)-4-(piperidin-4-yloxy)thieno[3,2-c]pyridine-2-sulfonamide hydrochloride**

The synthesis was preformed essentially as described for compound of N-(4-methylphenyl)-4-(pyrrolidin-3-yloxy)thieno[3,2-c]pyridine-2-sulfonamide hydrochloride. Yield: 16.2 mg (22.7 %). $^1$H NMR (270 MHz, $CH_3OH$-$d_4$) δ ppm 7.81-7.78 (m, 2H) 7.62 (d, *J*=6.93 Hz, 1 H) 7.13-7.04 (m, 4H) 4.05-3.94 (m, 1H) 3.90-3.88 (m, 2H) 3.63-3.58 (m, 2H), 2.27 (s, 3H) 2.06-2.03 (m, 2H) 2.01-1.71 (m, 2H); LC-MS 404 (M - H)$^+$; Purity (HPLC) 99 %.

EXAMPLE 81

**N-(2,3-Difluorobenzyl)-4-piperazin-1-ylthieno[3,2-c]pyridine-2-sulfonamide hydrochloride**

Yield: 74.4 mg (39.2 %). $^1$H NMR (270 MHz, $CH_3OH$-$d_4$) δ ppm 8.10-8.03 (m, 2H) 7.81 (d, *J*=6.68 Hz, 1 H) 7.16-7.05 (m, 3H) 4.37 (s, 2 H) 4.11-4.07 (m, 4H) 3.59-3.53 (m, 4H); LC-MS 425 (M - H)$^+$; Purity (HPLC) 90 %.

EXAMPLE 82

**N-(3-Chlorobenzyl)-4-piperazin-1-ylthieno[3,2-c]pyridine-2-sulfonamide hydrochloride**

Yield: 74.4 mg (44.7 %). $^1$H NMR (270 MHz, $CH_3OH$-$d_4$) δ ppm 8.04-8.01 7.85 (d, *J*=6.93 Hz, 1 H) 7.22-7.15 (m, 4H) 4.30 (s, 2 H) 4.14-4.10 (m, 4H) 3.60-3.54 (m, 4H); LC-MS 423 (M - H)$^+$; Purity (HPLC) 90%.

**Table 5**

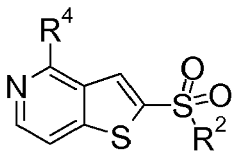

| EXAMPLE | | $R^2$ | $R^4$ |
|---|---|---|---|
| 83 | 4-(1,4-Diazepan-1-yl)-2-(phenylsulfonyl)thieno[3,2-c]pyridine hydrochloride | 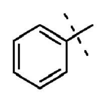 | 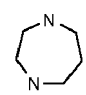 |
| 84 | 4-(1,4-Diazepan-1-yl)-2-[(3,4-dichlorophenyl)sulfonyl]thieno [3,2-c]pyridine hydrochloride | 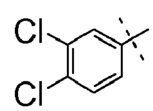 | 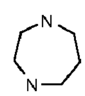 |
| 85 | 4-(1,4-Diazepan-1-yl)-2-[1-naphthylsulfonyl)thieno[3,2-c] pyridine hydrochloride | 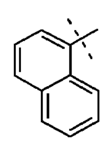 | 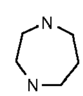 |
| 86 | 4-(1,4-Diazepan-1-yl)-2-[4-tert-butylphenylsulfonyl)thieno[3,2-c]pyridine hydrochloride | 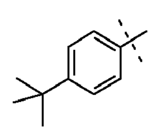 | 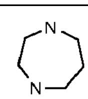 |
| 87 | 4-(1,4-Diazepan-1-yl)-2-[3,4-dimethylphenylsulfonyl)thieno [3,2-c]pyridine hydrochloride | 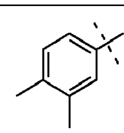 | 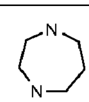 |

(continued)

| EXAMPLE | | $R^2$ | $R^4$ |
|---|---|---|---|
| 88 | 2-[(4-Bromophenyl)sulfonyl]-4-(1,4-diazepan-1-yl)thieno[3,2-c] pyridine hydrochloride | 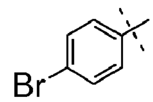 | 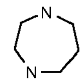 |
| 89 | 2-(Phenylsulfonyl)-4-piperazin-1-ylthieno[3,2-c]pyridine hydrochloride | 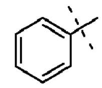 | 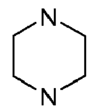 |
| 90 | 2-(3-Methoxy-benzenesulfonyl)-4-piperazin-1-yl-thieno[3,2-c] pyridine hydrochloride | 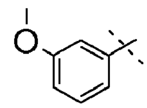 | 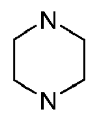 |
| 91 | 2-(4-Methoxy-benzenesulfonyl)-4-piperazin-1-yl-thieno[3,2-c] pyridine hydrochloride | 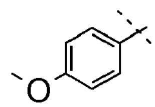 | 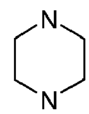 |
| 92 | 4-Piperzain-1-yl-2-{[4-trifluoromethyl)phenyl]sulfonyl}thieno [3,2-c]pyridine hydrochloride | 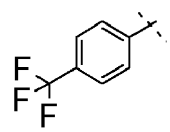 | 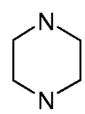 |
| 93 | 2-[[2-tert-Butylphenyl)sulfonyl]-4-piperazin-1-ylthieno[3,2-c] pyridine hydrochloride | 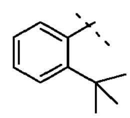 | 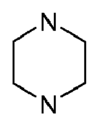 |
| 94 | 2-[(3,4-Dichlorophenyl)sulfonyl]-4-piperazin-1-ylthieno[3,2-c] pyridine hydrochloride | 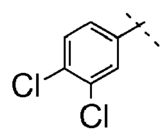 | 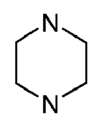 |
| 95 | 2-[(4-tert-Butylphenyl)sulfonyl]-4-piperazin-1-ylthieno[3,2-c] pyridine hydrochloride | 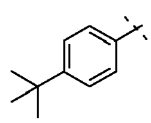 | 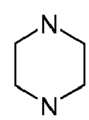 |
| 96 | 2-(1-Naphthyl sulfonyl)-4-piperazin-1-ylthieno[3,2-c]pyridine hydrochloride | 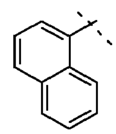 | 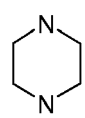 |

(continued)

| EXAMPLE | | $R^2$ | $R^4$ |
|---|---|---|---|
| 97 | 2-[(3-Fluorophenyl)sulfonyl]-4-piperazin-1-ylthieno[3,2-c] pyridine hydrochloride | 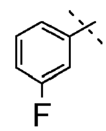 | 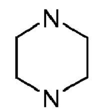 |
| 98 | 2-(Mesitylsulfonyl)-4-piperazin-1-ylthieno[3,2-c]pyridine hydrochloride | 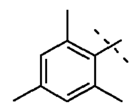 | 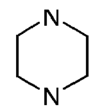 |
| 99 | 2-[(2-Methoxyphenyl)sulfonyl]-4-piperazin-1-ylthieno[3,2-c] pyridine hydrochloride | 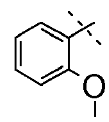 | 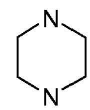 |
| 100 | 2-[(2,4-Dimethoxyphenyl)sulfonyl]-4-piperazin-1-ylthieno[3,2-c]pyridine hydrochloride | 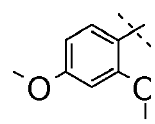 | 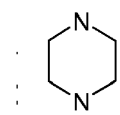 |
| 101 | 2-[(2,4-Dimethylphenyl)sulfonyl]-4-piperazin-1-ylthieno[3,2-c] pyridine hydrochloride | 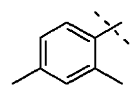 | 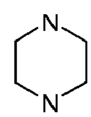 |
| 102 | 2-[(2,5-Dimethylphenyl)sulfonyl]-4-piperazin-1-ylthieno[3,2-c] pyridine hydrochloride | 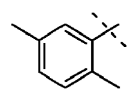 | 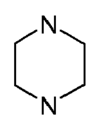 |
| 103 | 2-[(2-Ethylphenyl)sulfonyl]-4-piperazin-1-ylthieno[3,2-c] pyridine hydrochloride | 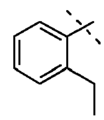 | 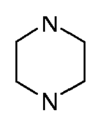 |
| 104 | 4-(Piperazinyl)-2-(3-methoxybenzyl-sulfonyl)-thienopyridine hydrochloride | 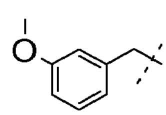 | 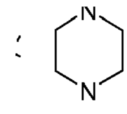 |

(continued)

| EXAMPLE | | $R^2$ | $R^4$ |
|---|---|---|---|
| 105 | 2-(Benzylsulfonyl)-4-piperazin-1-ylthieno[3,2-c]pyridine hydrochloride | 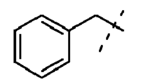 | 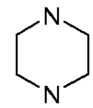 |
| 106 | 4-Piperazin-1-yl-2-{[4-(trifluoromethyl)benzyl]sulfonyl}thieno [3,2-c]pyridine hydrochloride | 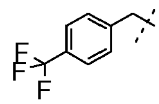 | 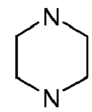 |
| 107 | 2-[(3-Bromobenzyl)sulfonyl]-4-piperazin-1-ylthieno[3,2-c] pyridine hydrochloride | 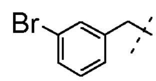 | 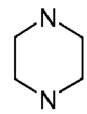 |
| 108 | 2-[(2,3-Difluorobenzyl)sulfonyl]-4-piperazin-1-ylthieno[3,2-c] pyridine hydrochloride | 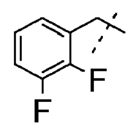 | 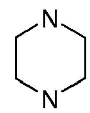 |
| 109 | 2-[(4-Bromobenzyl)sulfonyl]-4-piperazin-1-ylthieno[3,2-c] pyridine hydrochloride | 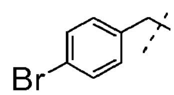 | 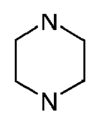 |
| 110 | 2-{[2,5-bis(Trifluoromethyl)benzyl]sulfonyl}-4-piperazin-1-ylthieno[3,2-c]pyridine hydrochloride | 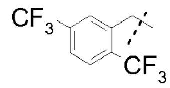 | 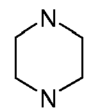 |
| 111 | 2-[(4-Methylbenzyl)sulfonyl]-4-piperazin- I -ylthieno[3,2-c] pyridine hydrochloride | 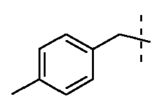 | 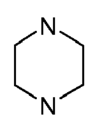 |
| 112 | 2-{[5-Chloro-2-(trifluoromethyl)benzyl]sulfonyl}-4-piperazin-1-ylthieno[3,2-c]pyridine hydrochloride | 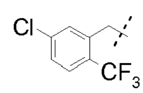 | 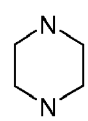 |

(continued)

| EXAMPLE | | $R^2$ | $R^4$ |
|---|---|---|---|
| 113 | 2-[(3,5-Dimethoxybenzyl)sulfonyl]-4-piperazin-1-ylthieno[3,2-c]pyridine hydrochloride | 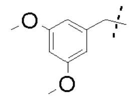 | 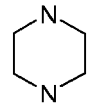 |
| 114 | 2-[(2-Naphthylmethyl)sulfonyl]-4-piperazin-1-ylthieno[3,2-c] pyridine hydrochloride | 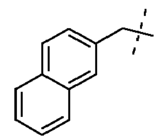 | 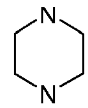 |
| 115 | 4-Piperazin-1-yl-2-1[4-(1,2,3-thiadiazol-5-yl)benzyl]sulfonyl} thieno[3,2-c]pyridine hydrochloride | 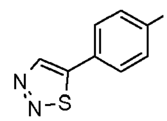 | 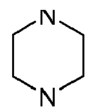 |
| 116 | 1-(4-Pyrrolidin-1-ylphenyl)-2-[(4-piperazine-1-ylthieno[3,2-c] pyridin-2-yl) sulfonyl] propanone hydrochloride | 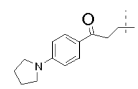 | 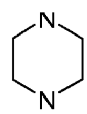 |
| 117 | 1-[4-(Diethylamino)phenyl]-2-[(4-piperazine-1-ylthieno[3,2-*c*] pyridin-2-yl) sulfonyl] propanone hydrochloride | 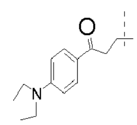 | 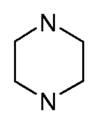 |
| 118 | 1-(4-Bromophenyl)-2-[(4-piperazin-1-ylthieno[3,2-*c*]pyridin-2-yl) sulfonyl] propanone | 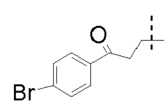 | 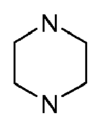 |
| 119 | 1-(3-Methoxyphenyl)-2-[(4-piperazin-1-ylthieno[3,2-*c*]pyridin-2-yl) sulfonyl] propanone | 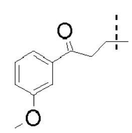 | 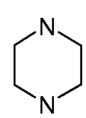 |
| 120 | 1-Phenyl-2-[(4-piperazin-1-ylthieno[3,2-*c*]pyridin-2-yl)sulfonyl] propanone | 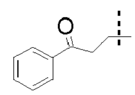 | 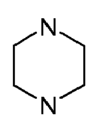 |

**Scheme 6**

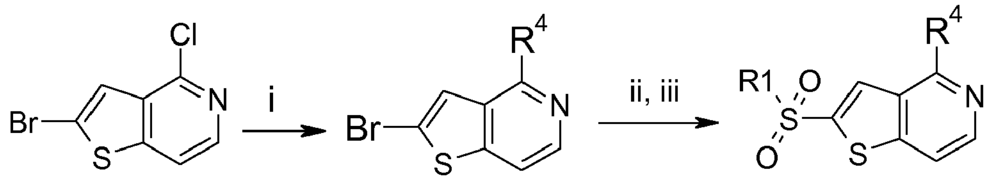


**Legend to Scheme 6:** i) BOC protected amines ($R^4$), $K_2CO_3$, DMSO; ii) thiophenols ($R^1$-SH), $Cu_2$(I)O, DMF; iii) NaOAc, oxone, water; iv) a.TFA, b.HCl, methanol

INTERMEDIATE 60

***tert*-Butyl 4-(2-bromothieno[3,2-c]pyridin-4-yl)piperazine-1-carboxylate**

2-Bromo-4-chlorothieno[3,2-c]pyridine (5.0 g, 20.24 mmol) and $K_2CO_3$ (13.97 g, 101.2 mmol) was stirred in DMSO (20 mL) followed by addintion of *tert*-butyl piperazine-1-carboxylate (4.14 g, 22.26 mmol). The reaction mixture was stirred at 100 ˚C for 6 days. The reaction mixture was filtered to eliminate the carbonate and addition of water (50 mL) and ethyl was followed. The phases were separated and the aqueous phase was extracted with ethyl acetate. The combined organic phases were dried ($MgSO_4$) and the solvent was evaporated. The crude product was purified by flash chromatography using ethyl acetate/hexanes (2/8) as eluent to give 2 g of the desired product, yield 25%, 99% pure. $^1H$ NMR (270 MHz, $CDCl_3$) δ 1.48 (s, 9H), 1.52-1.63 (m, 1H), 3.42-3.47 (m, 4H), 3.61-3.64 (m, 4H), 7.22 (dd, J= 5.4, 1 Hz, 1H), 7.35 (d, J= 1 Hz, 1H), 8.04 (d, J= 5.4 Hz, 1H). m/z = 398.91 (M+H), bromide pattern.

INTERMEDIATE 61

***tert*-butyl 4-(2-bromothieno[3,2-*c*]pyridin-4-yl)-1,4-diazepane-1-carboxylate**

The same procedure above intermediate was used starting from 2-bromo-4-chlorothieno[3,2-c]pyridine (7.5 g, 30.45 mmol), $K_2CO_3$ (6.7 g, 33.5 mmol) and *tert*-butyl 1,4-diazepane-1-carboxylate (21.0 g, 152.2 mmol) in DMSO (30 mL). Purification by flash chromatography 3.04 g of the title compound (Yield 25%).HPLC purity 92%; $^1H$ NMR (270 MHz, $CDCl_3$)δ1.38 (s, 4.5 H), 1.43 (s, 4.5 H), 1.96-2.11 (m, 2H), 3.36-3.41 (m, 1H), 3.46-3.51 (m, 1H), 3.65-3.87 (m, 6H), 7.02-7.04 (m, 1H), 7.40-7.42 (m, 1H), 7.94 (d, J= 5.4 Hz, 1H). m/z = 411.97 (M+H).

**Coupling with thiophenols (Method M)**

INTERMEDIATE 62

***tert*-butyl 4-(2-phenylthio)thieno[3,2-*c*]pyridin-4-yl)-1,4-diazepane-1-carboxylate**

*tert*-Butyl 4-(2-bromothieno[3,2-*c*]pyridin-4-yl)-1,4-diazepane-1-carboxylate (0.31 g, 0.752 mmol), pulverized KOH (0.084 g, 1.5 mmol) and $Cu_2$(I)O (0.1 g, 0.75 mmol) was mixed with DMF (1 mL) before the addition of a solution of benzenethiol (.016 g, 1.5 mmol) in DMF (1 mL). The reaction mixture was heated to 120 ˚C for 15 h. The reaction mixture was poured in a silica plug and eluted with chloroform, to give the crude product. The crude product was purified by flash chromatography using ethyl acetate/hexanes (2/8) as eluent to give 0.21 g of the desired product, yield 64%, 90% pure. $^1H$ NMR (270 MHz, $CDCl_3$) δ 1.37 (s, 4.5H), 1.43 (s, 4.5 H), 1.97-2.10 (m, 2H), 3.36-3.43 (m, 1H), 3.46-3.53 (m, 1H), 3.64-3.73 (m, 2H), 3.78-3.96 (m, 4H), 7.05 (d, J= 5.4 Hz, 1H), 7.22-7.32 (m, 5H), 7.59-7.63 (m, 1H), 7.97 (d, J= 5.4 Hz, 1H). m/z = 442.15 (M+H).

**Oxidation of thio-derivatives (Method N)**

INTERMEDIATE 63

**tert-Butyl 4-(2-phenylsulfonyl)thieno[3,2-*c*]pyridin-4-yl)-1,4-diazepane-1-carboxylate**

A solution of *tert*-Butyl 4-(2-phenylthio)thieno[3,2-c]pyridin-4-yl)-1,4-diazepane-1-carboxylate (0.21 g, 0.48

mmol) and NaOAc (0.5 g) in ethanol (10 mL), (pH ~5) followed by the addition of Oxone (0.64 g, 1.04 mmol) dissolved in water (1 mL). The reaction mixture was stirred at RT for 16 h. Additional Oxone (0.32 g) in water (1mL) was added. Full conversion of the SM was obtained after 8 h. Water (50 mL) and chloroform (30 mL) were added. The phases were separated and the aqueous phase was extracted with chloroform. The combined organic phases were dried over ($MgSO_4$), the solvent was evaporated to give the crude product which was purified by reverse-phase chromatography (10→90), to give 0.191 g of the desired product as yellow oil (Yield 86%) 98% pure. $^1H$ NMR (270 MHz, $CDCl_3$) δ 1.28 (s, 4.5 H), 1.38 (s, 4.5 H), 1.99-2.03 (m, 2H), 3.31-3.40 (m, 1H), 3.42-3.47 (m, 1H), 3.63-3.69 (m, 2H), 3.85-3.98 (m, 4H), 7.02 (d, J= 5.4 Hz, 1H), 7.48-7.61 (m, 3H), 7.76-8.01 (m, 3H), 8.06-8.08 (m, 1H). m/z = 474.01 (M+H).

**Removal of the t-butyl-carboxylate protecting group (Method O)**

EXAMPLE 83

**4-(1,4-Diazepan-1-yl)-2-(phenylsulfonyl)thieno[3,2-c]pyridine hydrochloride**

*tert-Butyl* 4-(2-phenylsulfonyl)thieno[3,2-*c*]pyridin-4-yl)-1,4-diazepane-1-carboxylate (0.165 g, 0.348 mmol) was dissolved in DCM (2 mL) and TFA (1 mL) was added. The reaction mixture was stirred for 2 h. The solvent was evaporated. Methanol and HCl in ether was added (x 3) to give 0.118 g of the desired HCl salt, yield 85%, 98% pure. $^1H$ NMR (270 MHz, CHOH-$d_4$) δ 2.45-2.52 (m, 2H), 3.45-3.52 (m, 2H), 3.70-3.79 (m, 2H), 4.18-4.22 (m, 2H), 4.30-4.40 (m, 2H), 7.62-7.76 (m, 5H), 7.91 (d, J = 5.4 Hz, 1H), 8.11 (dd, J= 5.4, 1 Hz, 1H), 8.41 (d, J= 1 Hz, 1H). m/z = 374.09 (M+H-HCl).

INTERMEDIATE 64

***tert*-Butyl 4-[2-(4-*tert*-butylphenyl)thio]thieno[3,2-*c*]pyridin-4-yl)-1,4-diazepane-1-carboxylate**

The product was prepared according to Method M. Purification by flash chromatography using ethyl acetate/hexanes (2/8) as eluent gave 0.035 g, 99% pure. $^1H$ NMR (270 MHz, $CDCl_3$)δ 1.28 (s, 9H), 1.38 (s, 4.5 H), 1.43 (s, 4.5 H), 1.98-2.03 (m, 2H), 3.35-3.41 (m, 1H), 3.46-3.52 (m, 1H), 3.62-3.72 (m, 2H), 3.77-3.93 (4H), 7.04 (d, J= 5.4 Hz, 1H), 7.27-7.34 (m, 4H), 7.54-7.56 (m, 1H), 7.95 (d, J= 5.4 Hz, 1H). m/z = 498.0 (M+H).

INTERMEDIATE 65

***tert*-Butyl 4-[2-(4-*tert*-butylphenyl)sulfonyl]thieno[3,2-*c*]pyridin-4-yl)-1,4-diazepane-1-carboxylate**

Procedure B from *tert*-butyl 4-[2-(4-tert-butylphenyl)thio]thieno[3,2-*c*]pyridin-4-yl)-1,4-diazepane-1-carboxylate (0.035 g, 0.070 mmol), Oxone (0.17 g, 0.28 mmol), NaOAc (0.5 g) in EtOH (2 mLfollowed by reversed phase chromatography (40→70), gave 6 mg of the product. Yield 17%, 98% pure. $^1H$ NMR (270 MHz, $CDCl_3$) δ 1.32 (s, 9H), 1.35 (s, 9H), 2.05-2.15 (m, 2H), 3.45-3.62 (m, 2H), 3.75-4.13 (m, 6H), 7.20-7.27 (m, 5H), 7.58 (d, J= 10.8 Hz, 1H), 7.93 (d, J= 10.8 Hz, 1H). m/z = 530.0 (M+H).

INTERMEDIATE 66

***tert*-Butyl 4-[2-(3,4-dimethylphenyl)thio]thieno[3,2-*c*]pyridin-4-yl)-1,4-diazepane-1-carboxylate**

The title compound was obtained according to Method M. Purification by flash chromatography using ethyl acetate/hexanes (2/8) as eluent gave 0.022 g, 95% pure. $^1H$ NMR (270 MHz, $CDCl_3$) δ 1.38 (s, 4.5 H), 1.43 (s, 4.5 H), 1.96-2.04 (m, 2H), 2.21 (s, 3H), 2.22 (s, 3H), 3.37-3.45 (m, 2H), 3.47-3.50 (m, 2H), 3.77-3.95 (m, 4H), 7.01-7.12 (m, 3H), 7.16 (s, 1H), 7.53 (dd, J= 5.4, 1 Hz, 1H), 7.94 (d, J= 5.4 Hz, 1H). m/z = 470.3 (M+H).

INTERMEDIATE 67

***tert*-Butyl 4-[2-(3,4-dimethylphenyl)sulfonyl]thieno[3,2-c]pyridin-4-yl)-1,4-diazepane-1-carboxylate**

Procedure B from *tert*-Buty14-[2-(3,4-dimethylphenyl)thio]thieno[3,2-*c*]pyridin-4-yl)-1,4-diazepane-1-carboxylate (0.022 g, 0.047 mmol); OXONE (0.11 g, 0.19 mmol); NaOAc (0.5 g) inEtOH (2 mL) followed by reversed phase chromatography (40→70), 9 mg of the product. Yield 38%, 92% pure. $^1H$ NMR (270 MHz, $CDCl_3$) δ 1.35 (s, 9H), 2.08-2.20 (m, 2H), 2.33 (s, 6H), 3.52-3.59 (m, 2H), 3.83-3.88 (m, 2H), 4.08-4.18 (m, 4H), 7.21-7.28 (m, 2H), 7.31-7.35 (m, 1H), 7.73-7.75 (m, 2H), 8.02 (d, J= 5.4 Hz, 1H). m/z = 502.21 (M+H).

INTERMEDIATE 68

***tert*-Butyl 4-[2-(1-naphthyl)thio] thieno[3,2-*c*]pyridin-4-yl)-1,4-diazepane-1-carboxylate**

The title compound was obtained according to Method M. Purification by flash chromatography using ethyl acetate/hexanes (2/8) as eluent gave 0.055 g. HPLC purity 99 %; $^1H$ NMR (270 MHz, $CDCl_3$)δ 1.37 (s, 4.5 H), 1.43 (s, 4.5 H), 1.89-2.20 (m, 2H), 3.30-3.40 (m, 1H), 3.43-3.50 (m, 1H), 3.60.3.90 (m, 6H), 6.99 (d, J= 5.4 Hz, 1H), 7.39 (dd, J= 8.1, 1 Hz, 1H), 7.50-7.61 (m, 5H), 7.79-7.88 (m, 2H), 7.92 (d, J= 5.4 Hz, 1H), 8.40-8.44 (m, 1H). m/z = 498.26 (M+H).

INTERMEDIATE 69

***tert*-Butyl 4-[2-(1-naphthyl)sulfonyl]thieno[3,2-*c*]pyridin-4-yl)-1,4-diazepane-1-carboxylate**

Procedure B from *tert*-Butyl 4-[2-(1-naphthyl)thio]thieno[3,2-*c*]pyridin-4-yl)-1,4-diazepane-1-carboxylate (0.055 g, 0.112 mmol); Oxone (0.27 g, 0.448 mmol); NaOAc (0.5 g) in EtOH (2 mL) followed reversed phase chromatography (40→70) gave 15 mg of the product. Yield 26%, 93% pure. $^1H$ NMR (270 MHz, $CDCl_3$) δ 1.34 (s, 9H), 2.06-2.10 (m, 2H), 3.48-3.62 (m, 2H), 3.78-3.86 (m, 2H), 3.95-4.16 (m, 4H), 7.19-7.31 (m, 2H), 7.60-7.75 (m, 3H), 7.92-7.99 (m, 2H), 8.18 (m, J= 8.1 Hz, 1H), 8.50-8.53 (m, 1H), 8.77-8.80 (m, 1H). m/z = 524.22 (M+H);

EXAMPLE 84

**4-(1,4-Diazepan-1-yl)-2-[(3,4-dichlorophenyl)sulfonyl]thieno[3,2-*c*]pyridine hydrochloride**

*tert*-Butyl 4- {2-[(3,4-dichlorophenyl)sulfonyl]thieno[3,2-c]pyridin-4-yl}-1,4-diazepane-1-carboxylate was prepared from 3,4-dichlorothiophenol (60 mg, 15%), as a beige solid, by the application of the general procedures A and B described above. $^1H$ NMR ($CDCl_3$) δ 8.27-8.14 (m, 1H), 8.11-8.04 (m, 2H), 7.87-7.80 (m, 1H), 7.67-7.62 (m, 1H), 7.26-7.20 (m, 1H), 4.18-3.98 (m, 4H), 3.87-3.74 (m, 2H), 3.61-3.44 (m, 2H), 2.20-2.00 (m, 2H), 1.33 (s, 9H); MS m/z 542 (M+1). The title compound (50 mg, 95%) was obtained as a beige solid, by the application of the general procedure C described above. $^1H$ NMR (270 MHz, $CH_3OH-d_4$) δ 8.48 (s, 1H), 8.30 (d, J = 1.85 Hz, 1H), 8.05 (dd, J = 8.58, 1.98 Hz, 1H), 7.92 (d, J= 6.86 Hz, 1H), 7.83 (d, J= 8.44 Hz, 1H), 7.69 (d, J= 6.86 Hz, 1H), 4.4.41-4.34 (m, 2H), 4.24-4.16 (m, 2H), 3.76-3.69 (m, 2H), 3.51-3.43 (m, 2H), 2.52-2.42 (m, 2H); MS m/z 442 (M+1).

EXAMPLE 85

**4-(1,4-Diazepan-1-yl)-2-[1-naphthylsulfonyl)thieno[3,2-*c*]pyridine hydrochloride**

The title compound was obtained from *tert*-butyl 4-[2-(1-naphthyl)sulfonyl]thieno-[3,2-*c*]pyridin-4-yl)-1,4-diazepane-1-carboxylate (15 mg, 0.029 mmol) following Method O to give 12 mg of the desired product yield 90 %, 95 % pure. $^1H$ NMR (270 MHz, $CH_3OH-d_4$) δ 2.40-2.50 (m, 2H), 3.45-3.55 (m, 2H), 3.65-3.75 (m, 2H), 4.06-4.26 (m, 2H), 4.27-4.46 (m, 2H), 7.58-7.80 (m, 4H), 7.83-7.86 (m, 1H), 8.06 (d, J= 8.1 Hz, 1H), 8.20 (d, J= 8.1 Hz, 1H), 8.48 (s, 1H), 8.53-8.56 (m, 1H), 8.83-8.86 (m, 1). m/z = 424.06 (M+H-HCl).

EXAMPLE 86

**4-(1,4-Diazepan-1-yl)-2-[4-*tert*-butylphenylsulfonyl)thieno[3,2-c]pyridine** hydrochloride

The title compound was obtained from *tert*-butyl 4-[2-(4-tert-butylphenyl)-sulfonyl]thieno[3,2-c]pyridin-4-yl)-1,4-diazepane-1-carboxylate (6 mg, 11.3 mmol) following Method O to give 4 mg of the desired product, yield 76 %, 88 % pure. $^1H$ NMR (270 MHz, $CH_3OH-d_4$) δ 1.33 (s, 9H), 2.41-2.47 (m, 2H), 3.41-3.49 (m, 2H), 3.65-3.78 (m, 2H), 4.15-4.25 (m, 2H), 4.29-4.40 (m, 2H), 7.65-7.70 (m, 3H), 7.90 (d, J= 5.4 Hz, 1H), 8.00-8.04 (m, 2H), 8.37 (s, 1H). m/z = 430.06 (M+H-

EXAMPLE 87

**4-(1,4-Diazepan-1-yl)-2-[3,4-dimethylphenylsulfonyl)thieno[3,2-c]pyridine hydrochloride**

The title compound was obtained from *tert*-butyl 4-[2-(3,4 dimethylphenyl)-sulfonyl]thieno[3,2-c]pyridin-4-yl)-1,4-diazepane-1-carboxylate (6 mg, 0.012 mmol) following Method O to give 6 mg of the desired product, yield 88 %, 89 % pure. $^1H$ NMR (270 MHz, $CH_3OH-d_4$) δ 2.34 (s, 6H), 2.45-2.55 (m, 2H), 3.42-3.51 (m, 2H), 3.67-3.76 (m, 2H),

4.10-4.20 (m, 2H), 3.58-3.70 (m, 2H), 7.39-7.41 (m, 1H), 7.64-7.67 (m, 1H), 7.79-7.84 (m, 2H), 7.89-7.91 (m, 1H), 8.36 (s, 1H). m/z = 402.07 (M+H-HCl).

EXAMPLE 88

**2-[(4-Bromophenyl)sulfonyl]-4-(1,4-diazepan-1-yl)thieno[3,2-c]pyridine hydrochloride**

Trifluoroacetic acid (1 mL) was added slowly to a solution of *tert*-butyl 4-{2-[(4-bromophenyl)thio]thieno[3,2-c] pyridin-4-yl}-1,4-diazepane-1-carboxylate (26 mg, 0.047 mmol) in $CH_2Cl_2$ at 0 ˚C. The reaction mixture was allowed to reach room temperature, stirred for 40 min and then concentrated *in vacuo.* The residue was twice re-dissolved in MeOH and concentrated *in vacuo.* The residue was again dissolved in MeOH and an excess of 1M HCl in diethyl ether (4 mL) was slowly added to the solution. Removal of the *solvents in vacuo* afforded the title compound (21 mg, 91 %) as a yellowish solid. $^1$H NMR (270 MHz, $CH_3OH-d_4$) δ 8.41 (s, 1H), 8.06-7.99 (m, 2H), 7.92 (d, J= 6.86 Hz, 1H), 7.87-7.80 (m, 2H), 7.66 (d, J= 6.86 Hz, 1H), 4.38-4.31 (m, 2H), 4.22-4.14 (m, 2H), 3.74-3.67 (m, 2H), 3.50-3.42 (m, 2H), 2.51-2.39 (m, 2H); MS *m*/*z* 452 (M+1).

EXAMPLE 89

**2-(Phenylsulfonyl)-4-piperazin-1-ylthieno[3,2-c]pyridine hydrochloride**

To a stirred solution of *tert*-butyl 4-[2-(phenylthio)thieno[3,2-c]pyridin-4-yl]piperazine-1-carboxylate (350 mg, 0.819 mmol) in ethanol was added oxone in water solution. The reaction was monitored by LCMS. When all starting material was consumed, the chromatogram showed two major peaks, the product and the N-oxide. After purification by preparative HPLC, the resulting Boc-material was treated with HCl in ether. The solution was centrifugated and the supernatant was removed. Ether was added, then centrifugated and decanted (repeated three times) to remove the excess HCl. The remaining ether was finally evaporated in a SpeedVac concentrator. Yield 18 %, HPLC purity = 98%, m/z = 360.0 (M+H). $^1$H NMR (270 MHz, $CH_3OH-d_4$) δ ppm 3.56 (m, 4 H) 4.08 (m, 4 H) 7.68 (m, 4 H) 7.77 (dd, *J*=6.60, 0.79 Hz, 1 H) 8.04 (d, *J*=6.33 Hz, 1 H) 8.12 (m, 2 H) 8.39 (d, *J=0.79* Hz, 1 H).

EXAMPLE 90

**2-(3-Methoxy-benzenesulfonyl)-4-piperazin-1-yl-thieno[3,2-c]pyridine hydrochloride**

4-[2-(3-Methoxy-phenylsulfanyl)-thieno[3,2-c]pyridin-4-yl]-piperazine-1-carboxylic acid *tert*-butyl ester was obtained from 3-methoxythiophenol (130 µl, 1 mmol) and *tert*-Butyl 4-(2-bromothieno[3,2-c]pyridin-4-yl)piperazine-1-carboxylate (215 mg, 0.52 mmol). 120 mg, 50%) were obtained by the application of the general Method M described above. $^1$H NMR (270 MHz, $CDCl_3$) δ 1.48 (s, 9 H), 3.42-3.51 (m, 4 H), 3.58-3.67 (m, 4 H), 3.74 (s, 3 H), 6.76 (dd, *J*=8.18, 2.38 Hz, 1 H), 6.84-6.92 (m, 2 H), 7.16-7.23 (m, 2 H), 7.51 (s, 1 H), 8.04 (d, *J*=5.81 Hz, 1 H); MS *m*/*z* 458 (M+1). The title compound was therefore obtained from 4-[2-(3-methoxy-phenylsulfanyl)-thieno[3,2-c]pyridin-4-yl]-piperazine-1-carboxylic acid *tert*-butyl (7 mg, 7%), after triturating with diethyl ether, as a beige solid, by the application of the general procedures B and C described above. $^1$H NMR (270 MHz, $CD_3OD$) δ 8.31 (s, 1H), 8.09 (d, J = 6.33 Hz, 1H), 7.71-7.62 (m, 2H), 7.59-7.50 (m, 2H), 7.30-7.23 (m, 1H), 3.98-3.92 (m, 4H), 3.87 (s, 3H), 3.54-3.48 (m, 4H); MS m/z 390 (M+1).

EXAMPLE 91

**2-(4-Methoxy-benzenesulfonyl)-4-piperazin-1-yl-thieno[3,2-c]pyridine hydrochloride**

4-[2-(4-Methoxy-phenylsulfanyl)-thieno[3,2-c]pyridin-4-yl]-piperazine-1-carboxylic acid *tert*-butyl ester was obtained from 4-methoxythiophenol (130 ul, 1 mmol) and *tert*-butyl 4-(2-bromothieno[3,2-c]pyridin-4-yl)piperazine-1-carboxylate (215 mg, 0.52 mmol). 100 mg, 42% were isolated by the application of the general Method M described above. $^1$H NMR (270 MHz, $CDCl_3$) δ 1.48 (s, 9 H), 3.40-3.47 (m, 4 H), 3.58-3.65 (m, 4 H), 3.79 (s, 3 H), 6.83-6.89 (m, 2 H), 7.15 (d, *J*=5.54 Hz, 1 H), 7.35 (s, 1 H), 7.38-7.43 (m, 2 H), 7.99 (d, *J*=5.81 Hz, 1 H); MS m/z 458 (M+1). 4-[2-(4-methoxy-phenylsulfanyl)-thieno[3,2-c]pyridin-4-yl]-piperazine-1-carboxylic acid *tert*-butyl ester was obtained (25 mg, 23%) as a clear liquid by the application of the general procedure B described above. $^1$H NMR (270 MHz, $CDCl_3$) δ 1.48 (s, 9 H), 3.67-3.91 (m, 11 H), 7.01 (d, J = 8.97 Hz, 2H), 7.27-7.37 (m, 1H), 7.93 (d, J = 8.97 Hz, 2H), 8.01-8.19 (m, 2H); MS m/z 490 (M+1). The title compound was thereby obtained following Procedure C): $^1$H NMR ($CD_3OD$) δ 8.25 (s, 1H), 8.09-7.89 (m, 3H), 7.69 (d, J = 6.33 Hz, 1H), 7.17-7.10 (m, 2H), 4.00-3.93 (m, 4H), 3.87 (s, 3H), 3.55-3.48 (m, 4H); MS m/z 390 (M+1).

EXAMPLE 92

**4-Piperazin-1-yl-2-{[4-trifluoromethyl)phenyl]sulfonyllthieno[3,2-c]pyridine hydrochloride**

2-{[4-(Trifluoromethyl)phenyl]thio}-4-piperazin-1-ylthieno[3,2-*c*]pyridine (0.42 mmol) was dissolved in TFA (1.5 mL) at 0˚C, stirred for 15 min and $H_2O_2$ (100 μL) was added. The mixture was stirred at room temperature over night. NaOH (2 M) was added, extraction with ethyl acetate (3X), washed with brine, dried over $NaSO_4$, The solvent was removed and the product was purified by preparative HPLC to afford 154.7 mg (86.2 %). $^1$H NMR (270 MHz, DMSO-$d_6$) δ ppm 9.79 (s, 1H) 8.56 (s, 1 H) 8.35 (d, *J*=8.44 Hz, 2 H) 8.12-8.05 (m, 3H) 7.79 (d, *J*=6.33 Hz, 1 H) 3.98-3.96 (m, 4H) 3.32-3.31 (m, 4H); LC-MS 428 (M - H)$^+$; Purity (HPLC) 95%

EXAMPLE 93

**2-[[2-*tert*-Butylphenyl)sulfonyl]-4-piperazin-1-ylthieno[3,2-c]pyridine hydrochloride**

The title compound was prepared following Method M-O. Yield: 10.6 mg (6.3 %) of 2-[[2-*tert*-butylphenyl) sulfonyl]-4-piperazin-1-ylthieno[3,2-c]pyridine hydrochloride. $^1$H NMR (270 MHz, DMSO-$d_6$) δ ppm 9.57 (s, 1 H) 8.42 (s, 1 H) 8.26-8.22 (m, 1H) 8.06-8.04 (m, 1H) 7.68-7.55 (m, 4H) 3.87-3.86 (m, 4H) 3.34-3.33 (m, 4H) 1.51-1.43 (m, 9H); LC-MS 400 (M - H)$^+$; Purity (HPLC) 90%.

EXAMPLE 94

**2-[(3,4-Dichlorophenyl)sulfonyl]-4-piperazin-1-ylthieno [3,2-c] pyridine-2-sulfonamide hydrochloride**

The title compound was prepared following Method M-O. Yield: 47.9 mg (22.9 %). $^1$H NMR (270 MHz, DMSO-$d_6$) δ ppm 9.36 (s, 1 H) 8.51 (s, 1 H) 8.38 (d, *J*=2.11 Hz, 1 H 8.06-7.94 (m, 3H) 7.70-7.68 (m, 1H) 3.81-3.77 (m, 4H) 3.31-3.29 (m, 4H); LC-MS 427 (M - H)$^+$; Purity (HPLC) 95 %.

EXAMPLE 95

**2-[(4-*tert*-Butylphenyl)sulfonyl]-4-piperazin-1-ylthieno[3,2-c]pyridine hydrochloride**

Oxone (0.52 g, 0.84 mmol) in water (4 mL), buffered to pH - 6 with sodium oxide acetate, was added to 2-[(4-*tert*-butylphenyl)thio]-4-piperazin-1-ylthieno[3,2-*c*]pyridine (0.42 mmol) in ethanol (30 mL). The mixture was stirred in room temperature for 2 h and more oxone (0.52 g, 0.84 mmol) was added. The reaction was stirred over night. Water was added to the mixture, extraction with dichloromethane (2X 20 mL) and the solvent was removed. The products were purified by preparative HPLC. Yield: 41.9 mg (22.0%). $^1$H NMR (500 MHz, $CH_3OH$-$d_4$) δ ppm 8.38 (s, 1 H) 8.05-8.01 (m, 3H) 7.80 (d, *J*=6.59 Hz, 1 H) 7.71-7.69 (m, 2H) 4.15-4.13 (m, 4H) 3.59-3.57 (4H) 1.37-1.33 (m, 9H); LC-MS 416 (M - H)$^+$; Purity (HPLC) 95%.

EXAMPLE 96

**2-(1-Naphthylsulfonyl)-4-piperazin-1-ylthieno[3,2-c]pyridine hydrochloride**

The title compound was prepared following Method M-O. Yield: 3.4 mg (0.2 %). $^1$H NMR (270 MHz, DMSO-$d_6$) δ ppm 9.34 (s, 1 H) 8.82 (s, 1 H) 8.44 (s, 1 H) 8.26-8.06 (m, 5H) 7.79-7.65 (m, 3H) 3.79-3.78 (m, 4H) 3.32-3.30 (m, 4H); LC-MS 410 (M - H)$^+$; Purity (HPLC) 95%.

EXAMPLE 97

**2-[(3-Fluorophenyl)sulfonyl]-4-piperazin-1-ylthieno[3,2-c]pyridine-2-sulfonamide hydrochloride**

2-Bromo-4-chlorothieno[3,2-c]pyridine (190 mg, 0.50 mmol) in DMF (1 mL) was added to 3-fluorobenzenethiol (95.5 mg, 1.0 mmol), KOH (56 mg, 0.2 mmol) and $Cu_2O$ (71 mg, 0.5 mmol) in DMF (1 mL). The reaction was heated to 120˚C over night. The mixture was filtrated through a silica plug and the solvent was removed. The product was dissolved in TFA (1.5 mL) at 0˚C and the solution were stirred for 15 min, $H_2O_2$ (100 μL) was added and the mixture was stirred at room temperature over night. 2M NaOH was added, extraction with etylacetate, washed with brine and solvent was removed. The product was purified by preparative HPLC. Yield: 30.1 mg (16.1%) $^1$H NMR (270 MHz, DMSO-$d_6$) δ ppm

9.34 (s, 1 H) 8.45 (s, 1 H) 8.16 (d, *J*=5.69 Hz, 1 H) 7.97-7.93 (m, 2H) 7.76-7.62 (m, 3H) 3.30 (s, 4 H) (4H obscured by solvent signal); LC-MS 378 $(M - H)^+$; Purity (HPLC) 99%.

EXAMPLE 98

**2-(Mesitylsulfonyl)-4-piperazin-1-ylthieno[3,2-c]pyridine hydrochloride**

The title compound was prepared following Method M-O. Yield: 32.0 mg (16.1 %). $^1H$ NMR (270 MHz, DMSO-$d_6$) δ ppm 9.32 (s, 1 H) 8.20-8.14 (m, 2H) 7.66 (d, *J*=5.69 Hz, 1 H) 7.14 (s, 2 H) 3.29 (s, 4 H) 2.65 (s, 6H) 2.28 (s, 3H) (4H obscured by solvent signal); LC-MS 402 $(M - H)^+$; Purity (HPLC) 95%.

EXAMPLE 99

**2-[(2-Methoxyphenyl)sulfonyl]-4-piperazin-1-ylthieno[3,2-c]pyridine hydrochloride**

The title compound was prepared following Method M-O.Yield: 14.7 mg (7.6 %). $^1H$ NMR (270 MHz, DMSO-$d_6$) δ ppm 9.33 (s, 1 H) 8.24 (s, 1 H) 8.15 (d, *J*=5.94 Hz, 1 H) 8.00 (dd, *J*=7.92, 1.48 Hz, 1 H) 7.77-7.68 (m, 2H) 7.28-7.18 (m, 2H) 3.30 (s, 4H) (7H obscured by solvent signal); LC-MS 390 $(M - H)^+$Purity (HPLC) 99%.

EXAMPLE 100

**2-[(2,4-Dimethoxyphenyl)sulfonyl]-4-piperazin-1-ylthieno[3,2-c]pyridine hydrochloride**

The title compound was prepared following Method M-O. Yield: 42.7 mg (20.5 %). $^1H$ NMR (270 MHz, DMSO-$d_6$) δ ppm 9.39 (s, 1 H) 8.37 (s, 1 H) 8.13 (d, *J*=5.69 Hz, 1 H) 7.68-7.66 (m, 2H) 7.54 (d, *J*=2.23 Hz, 1 H) 7.19 (d, *J*=8.66 Hz, 1 H) 3.29 (s, 4 H) (10H obscured by solvent signal); LC-MS 420 $(M - H)^+$; Purity (HPLC) 98%.

EXAMPLE 101

**2-[(2,4-Dimethylphenyl)sulfonyl]-4-piperazin-1-ylthieno[3,2-c]pyridine hydrochloride**

The title compound was prepared following Method M-O. Yield: 17.8 mg (9.3 %). $^1H$ NMR (270 MHz, DMSO-$d_6$) δ ppm 9.32 (s, 1H) 8.27 (s, 1 H) 8.15 (d, *J*=5.94 Hz, 1 H) 8.00 (d, *J*=8.17 Hz, 1 H) 7.67 (d, *J*=5.94 Hz, 1 H) 7.35-7.26 (m, 2H) 3.29 (s, 4H) 2.34 (s, 3H) (7H obscured by solvent signal); LC-MS 388 $(M - H)^+$ Purity (HPLC) 98%.

EXAMPLE 102

**2-[(2,5-Dimethylphenyl)sulfonyl]-4-piperazin-1-ylthieno[3,2-c]pyridine hydrochloride**

The title compound was prepared following Method M-O. Yield: 16.9 mg (8.8 %). $^1H$ NMR (270 MHz, DMSO-$d_6$) δ ppm 9.17 (s, 1 H) 8.29 (s, 1 H) 8.18-18.15 (m, 1H) 7.94 (s, 1 H) 7.66 (d, *J*=5.69 Hz, 1 H) 7.47 (d, *J*=7.67 Hz, 1 H) 7.32 (d, *J*=8.16 Hz, 1 H) 3.29 (s, 2 H) 2.42 (s, 3 H) (7H obscured by solvent signal); LC-MS 388 $(M - H)^+$; Purity (HPLC) 99%.

EXAMPLE 103

**2-[(2-Ethylphenyl)sulfonyl]-4-piperazin-1-ylthieno[3,2-c]pyridine hydrochloride**

The title compound was prepared following Method M-O. Yield: 22.6 mg (11.2 %). $^1H$ NMR (270 MHz, DMSO-$d_6$) δ ppm 9.19 (s, 1 H) 8.32 (s, 1 H) 8.17 (d, *J*=5.69 Hz, 1 H) 8.08 (d, *J*=7.92 Hz, 1 H) 7.73-7.66 (m, 2H) 7.52 (t, *J*=7.67 Hz, 2 H) 3.29 (s, 4 H) 3.00 (q, *J*=7.34 Hz, 2 H) 1.10 (m, 3 H) (4H obscured by solvent signal); LC-MS 388 $(M - H)^+$; Purity (HPLC) 100%.

## Scheme 7

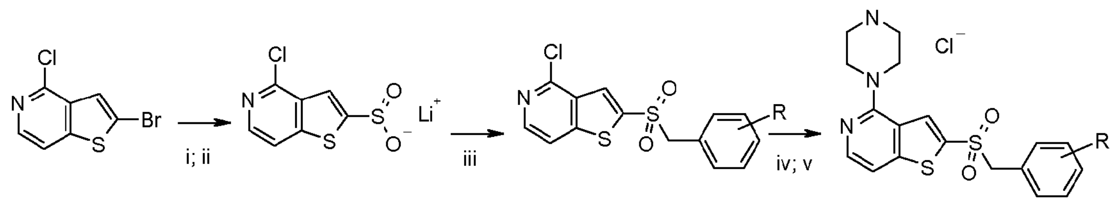


Legend to Scheme 7: i) nBuLi, diethyl ether; ii) $SO_2$ gas; iii) benzylbromine(s), DMF, heat; iv) diamine(s), $K_2CO_3$, DMF, heat; v) HCl, diethyl ether.

INTERMEDIATE 70

**Lithium 4-chlorothieno[3,2-c]pyridine-2-sulfinate**

2-Bromo-4-chlorothieno[3,2-c]pyridine (5.00 g, 20.1 mmol) was suspended in dry ether (100 ml) and the mixture was cooled to -78 ˚C under $N_2$-atmosphere . n-BuLi (1.6M in hexane, 15 mL) was added and the reaction mixture was stirred at -78 ˚C for 2h. $SO_2$ (g) was then bubbled threw the reaction mixture for 1h. After the gas bubbling had stopped the reaction mixture was stirred fore one more hour at -78 ˚C and was then allowed to warm to room temperature. The precipitate that had formed was filtered and washed with ether to give the sulfonate lithium salt (3.59 g, 74 %) that was used in the next step without further purification. $^1$H NMR (270 MHz, DMSO-$d_6$) δ ppm 7.26 (s, 1 H) 7.99 (d, *J=5.54* Hz, 1 H) 8.14 (d, *J=5.54* Hz, 1 H). MS (M-Li+1) 234.

**Benzylation of sulfinate salts (Method P)**

To a suspension of lithium 4-chlorothieno[3,2-c]pyridine-2-sulfinate (100 mg, 0.42 mmol) in dry DMF (2 mL) was added a benzylbromide (0.83 mmol, 2 equiv.) and the mixture heated with stirring for 16 h at 110 ˚C. Analysis by LCMS showed desired product and no starting material remaining. The mixture was treated with polystyrene-thiophenol (200 mg) and was rolled for 16 h. The suspension was filtered washing with further DMF (2mL). This material was reacted further without purification.

**Nucleophilic substitution of chlorine (Method Q)**

To a crude solutions of benzylsulfone in DMF (4 mL) are added potassium carbonate (172 mg, 1.25 mmol) and *tert*-butyl-piperazine-1-carboxylate (155 mg, 0.84 mmol). The resulting mixtures are heated for 16 h at 110 ˚C. LCMS shows desired compound and no starting material. The reaction mixtures are filtered and then the solvent removed under reduced pressure. The desired compounds are isolated pure following preparative HPLC.

**BOC-deprotection (Method R)**

The BOC N-protected piperazine derivatives are dissolved in HCl/ diethyl ether (1 mL, 1.0M) at room temperature and stirred for 16 h. Removal of the solvent under reduced pressure gave the crude hydrochloride salts. Trituration with acetonitrile gives the desired compound as a white solid.

INTERMEDIATE 71

***tert*-Butyl-4-[2-(benzylsulfonyl)thieno[3,2-c]pyridin-4-yl]piperazine-1-carboxylate**

Lithium 4-chlorothieno[3,2-c]pyridine-2-sulfinate (0.44 mmol) was treated with benzylbromide (0.59 mmol) as described in Method P above and then reacted further with *tert*-butyl-piperazine-1-carboxylate as described in Method Q. Yield 0.009 g (7 % over two steps). $^1$H NMR (300 MHz, $CDCl_3$) δ 8.14 (d, *J*=5.5 Hz 1 H), 7.27-7.40 (m, 5 H), 7.15-7.21 (m, 2 H), 4.45 (s, 2 H), 3.50-3.56 (m, 4 H), 3.40-3.45 (m, 4 H), 1.49 (s, 9 H); MS (ESI+) for C23 H27 N3 04 $S_2$ *m/z* 474 $(M+H)^+$. HPLC 77%, $R_T$ 3.93 min (ACE3 C8 50x4mm, 5-50% acetonitrile in 3 min).

INTERMEDIATE 72

***tert*-Butyl-4-(2-1[4-(trifluoromethyl)benzyl]sulfonyllthieno[3,2-c]pyridin-4-yl)piperazine-1-carboxylate**

Lithium 4-chlorothieno[3,2-c]pyridine-2-sulfinate (0.44 mmol) was treated with 4-(trifluoromethyl)benzylbromide (0.59 mmol) as described in Method P above and then reacted further with *tert*-butyl-piperazine-1-carboxylate as described in Method QF. Yield 0.02 g(16 % over two steps). Beige solid. $^1$H NMR (300 MHz, $CDCl_3$) δ 8.16 (d, *J*=6 Hz1 H), 7.53-7.61 (d, *J*= 9 Hz 2 H), 7.49 (s, 1 H), 7.26-7.36 (m, 4 H), 4.51 (s, 2 H), 3.49-3.60 (m, 4 H), 3.36-3.49 (m, 4 H), 1.49 (s, 9 H); MS (ESI+) for C24 H26 F3 N3 04 $S_2$ *m/z* 542 $(M+H)^+$. HPLC 71 %, $R_T$ 4.07min (ACE3 C8 50x4mm, 5-50% acetonitrile in 3 min).

INTERMEDIATE 73

***tert*-Butyl-4-{2-[(3-bromobenzyl)sulfonyl]thieno[3,2-c]pyridin-4-yl}piperazine-1-carboxylate**

Lithium 4-chlorothieno[3,2-c]pyridine-2-sulfinate (0.44 mmol) was treated with 3-bromobenzylbromide (0.59 mmol) as described in Method P above and then reacted further with *tert*-butyl-piperazine-1-carboxylate as described in Method Q. Yield 0.023 g (10 % over two steps). Beige solid. $^1$H NMR (300 MHz, $CDCl_3$) δ 8.16 (d, *J*=6 Hz, 1H), 7.50-7.55 (m, 2 H), 7.32-7.40 (m, 2 H), 7.10-7.24 (m, 3 H), 4.44 (s, 2 H), 3.61-3.73 (m, 8 H), 1.50 (s, 9 H); MS (ESI+) for C23 H26 Br N3 04 $S_2$ *m/z* 554 $(M+H)^+$. HPLC 77 %, $R_T$ 4.07min (ACE3 C8 50x4.6mm, 5-50% acetonitrile in 3 min).

INTERMEDIATE 74

***tert*-Butyl-4-(2-{[3-(trifluoromethyl)benzyl]sulfonyl}thieno[3,2-c]pyridin-4-yl)piperazine-1-carboxylate**

Lithium 4-chlorothieno[3,2-c]pyridine-2-sulfinate (0.44 mmol) was treated with 3-(trifluoromethyl)benzylbromide (0.59 mmol) as described in Method P above and then reacted further with *tert*-butyl-piperazine-1-carboxylate as described in Method Q. Yield 0.023 g (10 % over two steps). Beige solid. $^1$H NMR (300 MHz, $CDCl_3$) δ 8.14 (d, *J*= 6 Hz, 1 H), 7.85-7.91 (m, 1 H), 7.61-7.72 (m, 2 H), 7.50-7.60 (m, 1 H), 7.12-7.31 (m, 2 H), 4.74 (s, 2 H), 3.52-3.71 (m, 8 H), 1.50 (s, 9 H); MS (ESI+) for C24 H26 F3 N3 O4 $S_2$ *m/z* 542 $(M+H)^+$. HPLC 85 %, $R_T$ 2.13min (YMC ODS AQ, 33x3mm, 10-90% acetonitrile in 3 min).

INTERMEDIATE 75

***tert*-Butyl-4-(2-{[2,5-bis(trifluoromethyl)benzyl]sulfonyl}thieno[3,2-c]pyridin-4-yl)piperazine-l-carboxylate**

Lithium 4-chlorothieno[3,2-c]pyridine-2-sulfinate (0.44 mmol) was treated with 2,5-bis(trifluoromethyl)benzylbromide (0.59 mmol) as described in Method P above and then reacted further with *tert*-butyl-piperazine-1-carboxylate as described in Method Q. Yield 0.01 g (4 % over two steps). Beige solid. $^1$H NMR (300 MHz, $CDCl_3$) δ 8.16 (d, *J*= 5.8 Hz1 H), 8.00 (s, 1 H), 7.74-7.85 (m, 3 H), 4.76 (s, 2 H), 3.56-3.64 (m, 4 H), 3.47-3.56 (m, 4 H), 1.49 (s, 9 H); MS (ESI+) for C25 H25 F6 N3 O4 $S_2$ *m/z* 610 $(M+H)^+$. HPLC 73 %, $R_T$ 2.36min (YMC ODS AQ, 33x3mm, 10-90 % acetonitrile in 3 min).

INTERMEDIATE 76

***tert*-Butyl 4-{2-[(4-methylbenzyl)sulfonyl]thieno[3,2-c]pyridin-4-yl}piperazine-1-carboxylate**

Lithium 4-chlorothieno[3,2-c]pyridine-2-sulfinate (0.44 mmol) was treated with 4-methylbenzylbromide (0.59 mmol) as described in Method P above and then reacted further with *tert*-butyl-piperazine-1-carboxylate as described in Method Q. Yield 0.005 g (3 % over two steps). Beige solid. $^1$H NMR (300 MHz, $CDCl_3$) δ 8.15 (d, *J*= 6 Hz 1 H), 7.40 (s, 1 H), 7.00-7.16 (m, 4 H), 4.42 (s, 2 H), 3.46-3.60 (m, 4 H), 3.37-3.46 (m, 4 H), 2.34 (s, 3 H), 1.49 (s, 9 H); MS (ESI+) for C24 H29 N3 04 $S_2$ *m/z* 488 $(M+H)^+$. HPLC 69 %, $R_T$ 2.06min (YMC ODS AQ, 33x3mm, 10-90 % acetonitrile in 3 min).

INTERMEDIATE 77

***tert*-Butyl 4-(2-{[5-chloro-2-(trifluoromethyl)benzyl]sulfonyl}thieno[3,2-c]pyridin-4-yl)piperazine-1-carboxylate**

Lithium 4-chlorothieno[3,2-c]pyridine-2-sulfinate (0.44 mmol) was treated with 5-chloro-2-(trifluoromethyl)benzylbromide (0.59 mmol) as described in Method P above and then reacted further with *tert*-butyl-piperazine-1-carboxylate as described in Method Q. Yield 0.019 g (7.5 % over two steps). Beige solid. $^1$H NMR (300 MHz, $CDCl_3$) δ 8.12-8.14 (m, 1 H), 7.80-7.88 (m, 2 H), 7.47-7.66 (m, 2 H), 4.71 (s, 2 H), 3.74-3.83 (m, 4 H), 3.63-3.72 (m, 4 H), 1.49 (s, 9 H); MS (ESI+) for C24 H25 Cl F3 N3 04 $S_2$ *m/z* 576 $(M+H)^+$. HPLC 74 %, $R_T$ 2.30min (YMC ODS AQ, 33x3mm, 10-90 % acetonitrile in 3 min).

INTERMEDIATE 78

**tert-Butyl 4-{2-[(3,4-difluorobenzyl)sulfonyl]thieno[3,2-c]pyridin-4-yl}piperazine-1-carboxylate**

Lithium 4-chlorothieno[3,2-c]pyridine-2-sulfinate (0.44 mmol) was treated with 3,4-bis(trifluoromethyl)benzylbromide (0.59 mmol) as described in Method P above and then reacted further with *tert*-butyl-piperazine-1-carboxylate as described in Method Q. Yield 0.014 g (6 % over two steps). Beige solid. $^1$H NMR (300 MHz, $CDCl_3$) δ 8.17-8.21 (m, 1 H), 7.71 (s, 1 H), 7.07-7.39 (m, 4 H), 4.59 (s, 2 H), 3.55-3.68 (m, 8 H), 1.49 (s, 9 H); MS (ESI+) for C23 H25 F2 N3 04 $S_2$ *m/z* 510 $(M+H)^+$. HPLC 64 %, $R_T$ 2.02min (YMC ODS AQ, 33x3mm, 10-90 % acetonitrile in 3 min).

INTERMEDIATE 79

**tert-Butyl 4-{2-[(3,5-dimethoxybenzyl)sulfonyl]thieno[3,2-c] pyridin-4-yl}piperazine-1-carboxylate**

Lithium 4-chlorothieno[3,2-c]pyridine-2-sulfinate (0.44 mmol) was treated with 3,5-dimethoxybenzylbromide (0.59 mmol) as described in Method P above and then reacted further with *tert*-butyl-piperazine-1-carboxylate as described in Method Q. Yield 0.02 g (10 % over two steps). Beige solid. $^1$H NMR (300 MHz, $CDCl_3$) δ 8.14-8.18 (m, 1 H), 7.55 (s, 1 H), 6.40-6.45 (m, 1 H), 6.26-6.34 (m, 2 H), 4.39 (s, 2 H), 3.54-3.72 (m, 14 H), 1.50 (s, 9 H); MS (ESI+) for C25 H31 N3 06 $S_2$ *m/z* 534 $(M+H)^+$. HPLC 69 %, $R_T$ 1.99min (YMC ODS AQ, 33x3mm, 10-90 % acetonitrile in 3 min).

EXAMPLE 104

**4-(Piperazinyl)-2-(3-methoxybenzyl-sulfonyl)-thienopyridine**

A 1:1 mixture of lithium 4-chloro-thienopyridine-2-sulfinate (0.176 g, 0.734 mmol) and 2-methoxybenzylbromide (0.295 g, 1.47 mmol) in DMF (5 mL) was heated at 100 ˚C for 2h. To the mixture was added Boc-piperazine (546 mg, 2.94 mmol) and the reaction was heated at 110 ˚C for 1.5h. The solvent was removed and the crude product was purified by preparative HPLC to obtain 17.4 mg of 4-(4-t-butyl-oxy-carbonyl-piperazinyl)-2-(3-methoxybenzyl-sulfonyl)-thienopyridine. The boc-protected product was dissolved in 2 mL of MeOH and 4 mL of HCl/ether was added to obtain 21.9 mg of 4-(piperazinyl)-2-(3-methoxybenzyl-sulfonyl)-thienopyridine. $^1$HNMR ($CD_3OD/D_2O$ 1:1) δ 6.42-6.38 (m, 1H), 7.51-7.48 (m, 1H), 7.39-7.35 (m, 1H), 6.92-6.85 (m, 1H), 6.64-6.59 (m, 1H), 6.48-6.39 (m, 2H), 3.64-3.58 (m, 4H), 3.19-3.13 (m, 4H), 2.96 (s, 3H), 2.92 (s, 2H); MS (ESI) 404 $(M + H)^+$; Purity (HPLC, column YMC) 94%.

INTERMEDIATE 80

**tert-Butyl-4-[2-(benzylsulfonyl)thieno[3,2-c]pyridin-4-yl]piperazine-1-carboxylate**

Lithium 4-chlorothieno[3,2-c]pyridine-2-sulfinate (0.44 mmol) was treated with benzylbromide (0.59 mmol) as described in Method P above and then reacted further with *tert*-butyl-piperazine-1-carboxylate as described in Method Q. Yield 0.00 g (7 % over two steps). Beige solid. $^1$H NMR (300 MHz, $CDCl_3$) δ 8.14 (d, *J=5.5* Hz 1 H), 7.27-7.40 (m, 5 H), 7.15-7.21 (m, 2 H), 4.45 (s, 2 H), 3.50-3.56 (m, 4 H), 3.40-3.45 (m, 4 H), 1.49 (s, 9 H); MS (ESI+) for C23 H27 N3 04 $S_2$ *m/z* 474 $(M+H)^+$. HPLC 77 %, $R_T$ 3.93min (ACE3 C8 50x4mm, 5-50 % acetonitrile in 3 min).

EXAMPLE 105

**2-(Benzylsulfonyl)-4-piperazin-1-ylthieno[3,2-c]pyridine hydrochloride**

*tert*-Butyl 4-[2-(benzylsulfonyl)thieno[3,2-c]pyridin-4-yl]piperazine-1-carboxylate (0.01 g, 0.02 mmol) was treated as described in Method R to give the desired product as a white solid. Yield 0.009 g, (100 %). White solid. $^1$H NMR (300 MHz, DMSO-d6) δ 8.98 (s, 1 H), 8.13-8.20 (d, *J*=8 Hz1 H), 8.00 (s, 1 H), 7.64-7.71 (m, 1 H), 7.18-7.35 (m, 5 H), 4.93 (s, 2 H), 3.60-3.70 (m, 4 H), 3.22.3.34 (m, 4 H); MS (ESI+) for C18 H19 N3 02 S2 . Cl H *m/z* 374 $(M+H)^+$. HPLC 90%, $R_T$ 2.91min (ACE3 C8 50x4.6mm, 5-50% acetonitrile in 3 min).

INTERMEDIATE 81

***tert*-Butyl-4-(2-{[4-(trifluoromethyl)benzyl]sulfonyl}thieno[3,2-c]pyridin-4-yl)piperazine-1-carboxylate**

Lithium 4-chlorothieno[3,2-c]pyridine-2-sulfinate (0.44 mmol) was treated with 4-(trifluoromethyl)benzylbromide (0.59 mmol) as described in Method P above and then reacted further with *tert*-butyl-piperazine-1-carboxylate as described in Method Q. Yield 0.02 g (16 % over two steps). Beige solid. $^1$H NMR (300 MHz, $CDCl_3$) δ 8.16 (d, *J*=6 Hz1 H), 7.53-7.61 (d, *J*= 9 Hz 2 H), 7.49 (s, 1 H), 7.26-7.36 (m, 4 H), 4.51 (s, 2 H), 3.49-3.60 (m, 4 H), 3.36-3.49 (m, 4 H), 1.49 (s, 9 H); MS (ESI+) for C24 H26 F3 N3 04 $S_2$ *m/z* 542 $(M+H)^+$. HPLC 71 %, $R_T$ 4.07min (ACE3 C8 50x4mm, 5-50 % acetonitrile in 3 min).

EXAMPLE 106

**4-Piperazin-1-yl-2-{[4-(trifluoromethyl)benzyl]sulfonyl}thieno[3,2-c]pyridine hydrochloride**

*tert*-Butyl4-(2-{[4-(trifluoromethyl)benzyl]sulfonyl}thieno[3,2-c]pyridin-4-yl)piperazine-1-carboxylate (0.02 g, 0.03 mmol) was treated as described in Method R to give the desired product as a white solid. Yield 0.014 g (100 %) White solid. $^1$H NMR (300 MHz, DMSO-d6) δ 9.25 (s, 1 H), 8.12-8.22 (m, 2 H), 7.66-7.77 (m, 4 H), 7.41-7.52 (m, 2 H), 5.12 (s, 2 H), 3.22-3.35 (m, 4 H); MS (ESI+) for C19 H18 F3 N3 02 S2 . Cl H *m/z* 442 $(M+H)^+$. HPLC 90 %, $R_T$ 3.53min (ACE3 C8 50x4.6mm, 5-50 % acetonitrile in 3 min).

INTERMEDIATE 82

***tert*-Butyl-4-{2-[(3-bromobenzyl)sulfonyl]thieno[3,2-c]pyridin-4-yl}piperazine-1-carboxylate**

Lithium 4-chlorothieno[3,2-c]pyridine-2-sulfinate (0.44 mmol) was treated with 3-bromobenzylbromide (0.59 mmol) as described in Method P above and then reacted further with *tert*-butyl-piperazine-1-carboxylate as described in Method Q. Yield 0.023 g (10 % over two steps). Beige solid. $^1$H NMR (300 MHz, $CDCl_3$) δ 8.16 (d, *J*=6 Hz, 1H), 7.50-7.55 (m, 2 H), 7.32-7.40 (m, 2 H), 7.10-7.24 (m, 3 H), 4.44 (s, 2 H), 3.61-3.73 (m, 8 H), 1.50 (s, 9 H); MS (ESI+) for C23 H26 Br N3 04 $S_2$ *m/z* 554 $(M+H)^+$. HPLC 77 %, $R_T$ 4.07min (ACE3 C8 50x4.6mm, 5-50 % acetonitrile in 3 min).

EXAMPLE 107

**2-[(3-Bromobenzyl)sulfonyl]-4-piperazin-1-ylthieno[3,2-c]pyridine hydrochloride**

*tert*-Butyl 4- {2-[(3-bromobenzyl)sulfonyl]thieno[3,2-c]pyridin-4-yl}piperazine-1-carboxylate (0.023 g, 0.04 mmol) was treated as described in Method R to give the desired product as a white solid. Yield 0.013 g (67 %) White solid. $^1$H NMR (300 MHz, DMSO-d6) δ 9.19 (s, 1 H), 8.18 (d, *J*= 6 Hz, 1 H), 8.05 (s, 1 H), 7.70 (d, *J*= 6 Hz, 1 H), 7.53-7.58 (m, 1 H), 7.43-7.45 (m, 1 H), 7.19-7.32 (m, 2 H), 4.98 (s, 2 H), 3.24-3.35 (m, 4 H); MS (ESI+) for C18 H18 BrN3 02 S2. Cl H *m/z* 452 $(M+H)^+$. HPLC 90 %, $R_T$ 3.30min (ACE3 C8 50x4.6mm, 5-50 % acetonitrile in 3 min).

INTERMEDIATE 83

**tert-Butyl 4-{2-[(3,4-difluorobenzyl)sulfonyl]thieno[3,2-c]pyridin-4-yl}piperazine-1-carboxylate**

Lithium 4-chlorothieno[3,2-c]pyridine-2-sulfinate (0.44 mmol) was treated with 3,4-bis(trifluoromethyl)benzylbromide (0.59 mmol) as described in Method P above and then reacted further with *tert*-butyl-piperazine-1-carboxylate as described in Method Q. Yield 0.014 g (6 % over two steps). Beige solid. $^1$H NMR (300 MHz, $CDCl_3$) δ 8.17-8.21 (m, 1 H), 7.71 (s, 1 H), 7.07-7.39 (m, 4 H), 4.59 (s, 2 H), 3.55-3.68 (m, 8 H), 1.49 (s, 9 H); MS (ESI+) for C23 H25 F2 N3 04 $S_2$ *m/z* 510 $(M+H)^+$. HPLC 64 %, $R_T$ 2.02 min (YMC ODS AQ, 33x3mm, 10-90 % acetonitrile in 3 min).

EXAMPLE 108

**2-[(2,3-Difluorobenzyl)sulfonyl]-4-piperazin-1-ylthieno[3,2-c]pyridine hydrochloride**

The BOC group was removed from *tert*-butyl 4-{2-[(3,4-difluorobenzyl)sulfonyl]thieno[3,2-c]pyridin-4-yl}piperazine-1-carboxylate using Method R. Yield 0.068 g (100 %). White solid. $^1$H NMR (300 MHz, DMSO-$d_6$) δ 9.34 (s, 1 H), 8.22 (s, 1 H), 8.18 (d, *J*=*5.5* Hz, 1 H), 7.70 (d, *J*=5.5 Hz, 1 H), 7.26-7.35 (m, 1 H), 7.15-7.22 (m, 1 H), 7.05-7.14 (m, 1

H), 5.08 (s, 2 H), 3.34-3.42 (m, 4 H), 3.25-3.34 (m, 4 H); MS (ESI+) for C18 H17 F2 N3 02 S2. Cl H *m/z* 410 $(M+H)^+$. HPLC 90 %, $R_T$ 1.07min (YMC ODS AQ, 33x3mm, 20-50 % acetonitrile in 1.5 min).

EXAMPLE 109

**2-[(4-Bromobenzyl)sulfonyl]-4-piperazin-1-ylthieno[3,2-c]pyridinehydrochloride**

Lithium 4-chlorothieno[3,2-c]pyridine-2-sulfinate (0.42 mmol) was treated with 4-bromobenzylbromide (0.59 mmol) as described in Method P above and then reacted further with *tert*-butyl-piperazine-1-carboxylate as described in Method Q. The BOC protecting group was removed using Method R. Yield 0.024 g (12 % over three steps). White solid. $^1$H NMR (300 MHz, DMSO-d6) δ 8.99 (s, 1 H), 8.18 (d, *J*= 5.5 Hz, 1 H), 8.02 (s, 1 H), 7.69 (d, *J*= 5.5 Hz, 1 H), 7.53 (d, *J*= 8.5 Hz, 2 H), 7.17 (d, *J*= 8.5 Hz, 2H), 4.95 (s, 2 H), 3.62-3.68 (m, 4 H), 3.27-3.32 (m, 4 H); MS (ESI+) for C18 H18 Br N3 02 S2. Cl H m/z 454 $(M+H)^+$. HPLC 90 %, $R_T$ 1.24min (YMC ODS AQ, 33x3mm, 20-50 % acetonitrile in 1.5 min).

INTERMEDIATE 84

***tert*-Butyl-4-(2-{[2,5-bis(tritluoromethyl)benzyl]sulfonyl}thieno[3,2-c]pyridin-4-yl)piperazine-1-carboxylate**

Lithium 4-chlorothieno[3,2-c]pyridine-2-sulfinate (0.44 mmol) was treated with 2,5-bis(trifluoromethyl)benzylbromide (0.59 mmol) as described in Method P above and then reacted further with *tert*-butyl-piperazine-1-carboxylate as described in Method Q. Yield 0.01 g (4 % over two steps). Beige solid. $^1$H NMR (300 MHz, $CDCl_3$) δ 8.16 (d, *J*= 5.8 Hz1 H), 8.00 (s, 1 H), 7.74-7.85 (m, 3 H), 4.76 (s, 2 H), 3.56-3.64 (m, 4 H), 3.47-3.56 (m, 4 H), 1.49 (s, 9 H); MS (ESI+) for C25 H25 F6 N3 04 $S_2$ *m/z* 610 $(M+H)^+$. HPLC 73 %, $R_T$ 2.36min (YMC ODS AQ, 33x3mm, 10-90 % acetonitrile in 3 min).

EXAMPLE 110

**2-{[2,5-Bis(trifluoromethyl)benzyl]sulfonyl}-4-piperazin-1-ylthieno[3,2-c]pyridine hydrochloride**

The BOC group was removed from *tert*-butyl 4-(2-{[2,5-(trifluoromethyl)-benzyl]sulfonyl}thieno[3,2-c]pyridin-4-yl)piperazine-1-carboxylate using Method R. Yield 0.024 g (100 %). White solid. $^1$H NMR (300 MHz, DMSO-$d_6$) δ 9.30 (s, 1 H), 8.25 (s, 1 H), 8.19 (d, *J*=5.5 Hz, 1 H), 8.00-8.07 (m, 2 H), 7.85 (s, 1 H), 7.69 (d, *J*=5.5 Hz, 1 H), 5.22 (s, 2 H), 3.24-3.33 (m, 4 H); MS (ESI+) for C20 H17 F6 N3 02 S2. Cl H *m/z* 510 $(M+H)^+$. HPLC 90 %, $R_T$ 1.08min (YMC ODS AQ, 33x3mm, 30-60 % acetonitrile in 1.5 min).

INTERMEDIATE 85

***tert*-Butyl 4-{2-[(4-methylbenzyl)sulfonyl]thieno[3,2-c]pyridin-4-yl}piperazine-1-carboxylate**

Lithium 4-chlorothieno[3,2-c]pyridine-2-sulfinate (0.44 mmol) was treated with 4-methylbenzylbromide (0.59 mmol) as described in Method P above and then reacted further with *tert*-butyl-piperazine-1-carboxylate as described in Method Q. Yield 0.005 g (3 % over two steps). Beige solid. $^1$H NMR (300 MHz, $CDCl_3$) δ 8.15 (d, *J*= 6 Hz 1 H), 7.40 (s, 1 H), 7.00-7.16 (m, 4 H), 4.42 (s, 2 H), 3.46-3.60 (m, 4 H), 3.37-3.46 (m, 4 H), 2.34 (s, 3 H), 1.49 (s, 9 H); MS (ESI+) for C24 H29 N3 04 $S_2$ *m/z* 488 $(M+H)^+$. HPLC 69 %, $R_T$ 2.06min (YMC ODS AQ, 33x3mm, 10-90 % acetonitrile in 3 min).

EXAMPLE 111

**2-[(4-Methylbenzyl)sulfonyl]-4-piperazin-1-ylthieno[3,2-c]pyridine hydrochloride**

The BOC group was removed from *tert*-butyl 4-{2-[(4-methylbenzyl)sulfonyl]thieno[3,2-c]pyridin-4-yl}piperazine-1-carboxylate using Method R. Yield 0.05 g (75 %). White solid. $^1$H NMR (300 MHz, DMSO-d6) δ 9.18 (s, 1 H), 8.17 (d, *J*=5.5 Hz, 1 H), 8.01 (s, 1 H), 7.67 (d, *J*=5.5 Hz, 1 H), 7.38 (s, 1 H), 7.19 (s, 1 H), 7.11 (s, 1 H), 7.00 (s, 1 H), 4.86 (s, 2 H); MS (ESI+) for C19 H21 N3 02 S2. Cl H m/z 388 $(M+H)^+$.HPLC 90 %, $R_T$ 1.65 min (ACE3 C8 50x3.0mm, 10-97 % acetonitrile in 3 min).

INTERMEDIATE 86

***tert*-Butyl 4-(2-{[5-chloro-2-(trifluoromethyl)benzyl]sulfonyl}thieno[3,2-c]pyridin-4-yl)piperazine-1-carboxylate**

Lithium 4-chlorothieno[3,2-c]pyridine-2-sulfinate (0.44 mmol) was treated with 5-chloro-2-(trifluoromethyl)benzylbromide (0.59 mmol) as described in Method P above and then reacted further with *tert*-butyl-piperazine-1-carboxylate as described in Method Q. Yield 0.019 g (7.5 % over two steps). Beige solid. $^1$H NMR (300 MHz, $CDCl_3$) δ 8.12-8.14 (m, 1 H), 7.80-7.88 (m, 2 H), 7.47-7.66 (m, 2 H), 4.71 (s, 2 H), 3.74-3.83 (m, 4 H), 3.63-3.72 (m, 4 H), 1.49 (s, 9 H); MS (ESI+) for C24 H25 Cl F3 N3 04 $S_2$ *m/z* 576 $(M+H)^+$. HPLC 74 %, $R_T$ 2.30min (YMC ODS AQ, 33x3mm, 10-90 % acetonitrile in 3 min).

EXAMPLE 112

**2-{[5-Chloro-2-(trinuoromethyl)benzyl]sulfonyl}-4-piperazin-1-ylthieno[3,2-c]pyridine hydrochloride**

The BOC group was removed from *tert*-butyl 4-(2-{[5-chloro-2-(trifluoromethyl)benzyl]sulfonyl}thieno[3,2-c]pyridin-4-yl)piperazine-1-carboxylate using Method r. Yield 0.012 g (92 %). White solid. $^1$H NMR (300 MHz, DMSO-$d_6$) δ 9.05 (s, 1 H), 8.18-8.23 (m, 2 H), 7.78-7.83 (m, 1 H), 7.72-7.76 (m, 1 H), 7.69 (d, *J*=5.5 Hz, 1 H), 7.62-7.65 (m, 1 H), 5.07 (s, 2 H), 3.65-3.72 (m, 4 H), 7.25-7.34 (m, 4 H); MS (ESI+) for C19 H17 Cl F3 N3 O2 S2. Cl H *m/z* 476 $(M+H)^+$. HPLC 90 %, $R_T$ 1.65 min (ACE3 C8 50x3.0mm, 10-97 % acetonitrile in 3 min).

INTERMEDIATE 87

***tert*-Butyl 4-{2-[(3,5-dimethoxybenzyl)sulfonyl]thieno[3,2-c]pyridin-4-yl}piperazine-1-carboxylate**

Lithium 4-chlorothieno[3,2-c]pyridine-2-sulfinate (0.44 mmol) was treated with 3,5-dimethoxybenzylbromide (0.59 mmol) as described in Method P above and then reacted further with *tert*-butyl-piperazine-1-carboxylate as described in Method Q. Yield 0.02 g (10 % over two steps). Beige solid. $^1$H NMR (300 MHz, $CDCl_3$) δ 8.14-8.18 (m, 1 H), 7.55 (s, 1 H), 6.40-6.45 (m, 1 H), 6.26-6.34 (m, 2 H), 4.39 (s, 2 H), 3.54-3.72 (m, 14 H), 1.50 (s, 9 H); MS (ESI+) for C25 H31 N3 06 $S_2$ *m/z* 534 $(M+H)^+$. HPLC 69 %, $R_T$ 1.99min (YMC ODS AQ, 33x3mm, 10-90 % acetonitrile in 3 min).

EXAMPLE 113

**2-[(3,5-Dimethoxybenzyl)sulfonyl]-4-piperazin-1-ylthieno[3,2-c]pyridine hydrochloride**

The BOC group was removed from *tert*-butyl 4-{2-[(3,5-dimethoxybenzyl)sulfonyl]thieno[3,2-c]pyridin-4-yl}piperazine-1-carboxylate using Method R. Yield 0.01g (62%). White solid. $^1$H NMR (300 MHz, DMSO-$d_6$) δ 9.09 (s, 1 H), 8.18 (d, *J*= 6.7 Hz, 1 H), 8.02 (s, 1 H), 7.69 (d, *J*= 6.7 Hz, 1H), 6.45-6.48 (m, 1 H), 6.35-6.38 (m, 2 H), 4.84 (s, 2H), 3.61-3.67 (m, 4H), 3.58 (s, 6H), 3.24-3.33 (m, 4H).MS (ESI+) for $C_{20}H_{22}N_3O_4S_2$ *m/z* 434 $(M+H)^+$. HPLC 90 %, $R_T$ 1.60 min (ACE3 C8 50x3.0mm, 10-97 % acetonitrile in 3 min).

EXAMPLE 114

**2-[(2-Naphthylmethyl)sulfonyl]-4-piperazin-1-ylthieno[3,2-c]pyridine hydrochloride**

2-(Bromomethyl)naphthalene was used according to Method P-R to give 12.4 mg of the desired product. $^1$H NMR (270 MHz, $CH_3OH$-$d_4$) δ ppm (obscured by $CH_3OH$, 4H) 3.70-3.79 (m, 4 H) 4.95 (s, 2 H) 7.36-7.58 (m, 3 H) 7.70-7.91 (m, 6 H) 8.02 (d, J=6.60 Hz, 1 H). MS (M+1) 424.

EXAMPLE 115

**4-Piperazin-1-yl-2-{[4-(1,2,3-thiadiazol-4-yl)benzyl]sulfonyl}thieno[3,2-c]pyridine hydrochloride**

4-[4-(Bromomethyl)phenyl]-1,2,3-thiadiazole was used according to Method P-R to give 4.8 mg of the desired product. $^1$H NMR (270 MHz, $CH_3OH$-$d_4$) δ ppm 3.41-3.50 (m, 4 H) 3.54 (s, 2 H) 3.89-3.98 (m, 4 H) 7.45 (d, *J*=8.44 Hz, 2 H) 7.75 (d, *J*=6.33 Hz, 1 H) 7.96-8.13 (m, 4 H) 9.31 (s, 1 H). MS (M+1) 458.

EXAMPLE 116

**1-(4-Pyrrolidin-1-ylphenyl)-2-[(4-piperazine-1-ylthieno[3,2-c]pyridin-2-yl) sulfonyl] ethanone hydrochloride**

2-Bromo-1-(4-pyrrolidin-1-ylphenyl)ethanone was used according to Method P-R to give 19.6 mg of the desired product. $^1$H NMR (270 MHz, $CH_3OH-d_4$) δ ppm 2.02-2.12 (m, 4 H) 2.69 (s, 1 H) 3.37 (t, *J*=6.73 Hz, 4 H) 3.54 (s, 1 H) 3.56.3.64 (m, 4 H) 4.12-4.22 (m, 4 H) 6.55 (d, *J*=8.97 Hz, 2 H) 7.78-7.90 (m, 3 H) 8.03 (d, *J*=6.86 Hz, 1 H) 8.41 (s, 1 H). MS (M+1) 471.

EXAMPLE 117

**1-[4-(Diethylamino)phenyl]-2-[(4-piperazine-1-ylthieno[3,2-c]pyridin-2-yl)** sulfonyl] **ethanone hydrochloride**

2-Bromo-1-[4-(diethylamino)phenyl]ethanone ethanone was used according to Method P-R to give 9.0 mg of the desired product. $^1$H NMR (500 MHz, $CH_3OH-d_4$) δ ppm 1.16 (t, *J*=7.06 Hz, 6 H) 3.49-3.66 (m, 10 H) 4.14-4.27 (m, 4 H) 7.13 (br.s, 2 H) 7.85 (d, *J*=6.59 Hz, 1 H) 7.98 (d, *J*=8.48 Hz, 2 H) 8.03 (d, *J*=6.59 Hz, 1 H) 8.47 (s, 1 H). MS (M+1) 473.

EXAMPLE 118

**1-(4-Bromophenyl)-2-[(4-piperazin-1-ylthieno[3,2-c]pyridin-2-yl) sulfonyl] ethanone**

2-Bromo-1-(4-bromophenyl)ethanone was used according to method A to give 3.4 mg of the desired product. $^1$H NMR (270 MHz, $CH_3OH-d_4$) δ ppm 3.55 (s, 2 H) 3.56-3.67 (m, 4 H) 4.08-4.26 (m, 4 H) 7.68 (d, *J*=8.44 Hz, 2 H) 7.79-7.98 (m, 3 H) 8.06 (d, *J*=6.60 Hz, 1 H) 8.45 (s, 1 H). MS (M+1) 481.

EXAMPLE 119

**1-(3-Methoxyphenyl)-2-[(4-piperazin-1-ylthieno[3,2-*c*]pyridin-2-yl) sulfonyl] ethanone**

2-bromo-1-(3-methoxyphenyl)ethanone was used according to method A to give 1.0 mg of the desired product. $^1$H NMR (270 MHz, $CH_3OH-d_4$) δ ppm 3.54 (s, 2 H) 3.55-6.62 (m, *J*=10.03 Hz, 4 H) 3.82 (s, 3 H) 4.06-4.18 (m, 4 H) 7.20 (dd, *J*=8.05, 2.24 Hz, 1 H) 7.34-7.49 (m, 2 H) 7.57 (d, *J*=7.39 Hz, 1 H) 7.84 (d, *J*=6.60 Hz, 1 H) 8.06 (d, *J*=6.60 Hz, 1 H) 8.41 (s, 1 H). MS (M+1) 432.

EXAMPLE 120

**1-Phenyl-2-[(4-piperazin-1-ylthieno[*3,2-c*]pyridin-2-yl)sulfonyl]ethanone**

2-Bromo-1-phenylethanone was used according to method A to give 1.2 mg of the desired product. $^1$H NMR (270 MHz, $CH_3OH-d_4$) δ ppm 3.55 (s, 2 H) 3.57-3.66 (m, 4 H) 4.10-4.24 (m, 4 H) 7.46-4.57 (m, 2 H) 7.66 (t, *J*=7.39 Hz, 1 H) 7.86 (d, *J*=6.60 Hz, 1 H) 8.02 (dd, *J*=14.12, 6.99 Hz, 3 H) 8.46 (s, 1 H). MS (M+1) 402.

**Table 6**

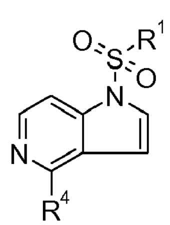

(continued)

| EXAMPLE | | $R^1$ | $R^4$ |
|---|---|---|---|
| 121 | 4-Piperazin-1-yl-1-(toluene-4-sulfo nyl)-1H-pyrrolo[3,2-c] pyridine hydrochloride | 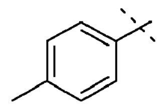 | 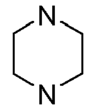 |
| 122 | 1-(3-Chloro-2-methyl-benzenesulfony 1)-4-piperazin-1-yl-1H-pyrrolo[3,2-c]pyridine hydrochloride | 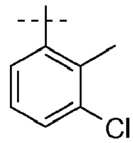 | 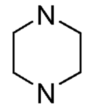 |
| 123 | 1-(3,4-Dimethoxy-benzenesulfonyl)-4 -piperazin-1-yl-1H-pyrrolo[3,2-c]py ridine hydrochloride | 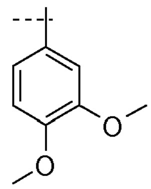 | 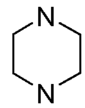 |
| 124 | 4-(4-Piperazin-1-yl-pyrrolo[3,2-c]p yridine-1-sulfonyl)-benzonitrile hydrochloride | 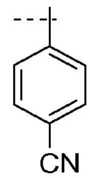 | 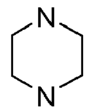 |
| 125 | 1-(4,5-Dichloro-thiophene-2-sulfonyl)-4-piperazin-1-yl-1H-pyrrolo[3,2-c]pyridine hydrochloride | 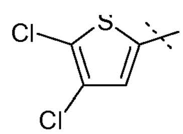 | 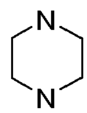 |
| 126 | 1-(2-Chloro-4-fluoro-benzenesulfony 1)-4-piperazin-1-yl-1H-pyrrolo[3,2-c]pyridine hydrochloride | 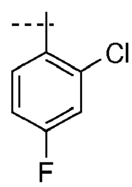 | 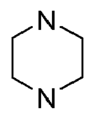 |
| 127 | 1-Phenylmethanesulfonyl-4-piperazin-1-yl-1H-pyrrolo [3,2-c] pyridine hydrochloride | 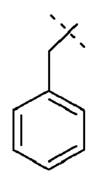 | 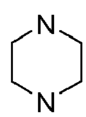 |
| 128 | 1-(5-Chloro-thiophene-2-sulfonyl)-4 -piperazin-1-yl-1H-pyrrolo [3,2-c]py ridine hydrochloride | 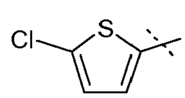 | 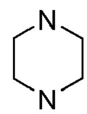 |

(continued)

| EXAMPLE | | $R^1$ | $R^4$ |
|---|---|---|---|
| 129 | 1-(4-Butyl-benzenesulfonyl)-4-piperazin-1-yl-1H-pyrrolo(3,2-c) pyridine hydrochloride | 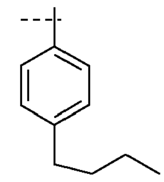 | 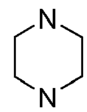 |
| 130 | 1-(4-Phenoxy-benzenesulfonyl)-4-piperazin-1-yl-1H-pyrrolo (3,2-c)pyridine hydrochloride | 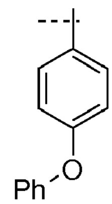 | 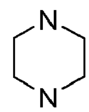 |
| 131 | 1-(Phenylsulfonyl)-4-piperazin-1-yl-1H-pyrrolo[3,2-c]pyridine hydrochloride | 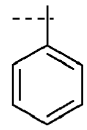 | 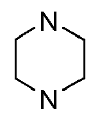 |
| 132 | 1-[(4-Chlorophenyl)sulfonyl]-4-piperazin-1-yl-1H-pyrrolo[3,2-c] pyridine hydrochloride | 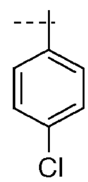 | 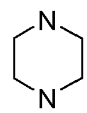 |
| 133 | 1-[(4-Methoxyphenyl)sulfonyl]-4-piperazin-1-yl-H-pyrrolo[3,2-c]pyridine hydrochloride | 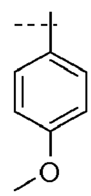 | 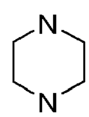 |
| 134 | 1-[(2-Methoxy-5-methylphenyl)sulfonyl]-4-piperazin-1-yl-1H-pyrrolo[3,2-c]pyridine hydrochloride | 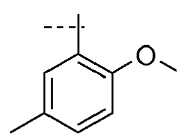 | 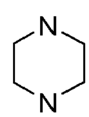 |
| 135 | 4-Piperazin-1-yl-1-{[2-(trifluoromethyl)phenyl]sulfonyl}-1H-pyrrolo[3,2-c]pyridine hydrochloride | 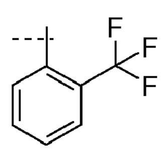 | 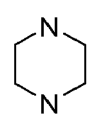 |

**Scheme 8**

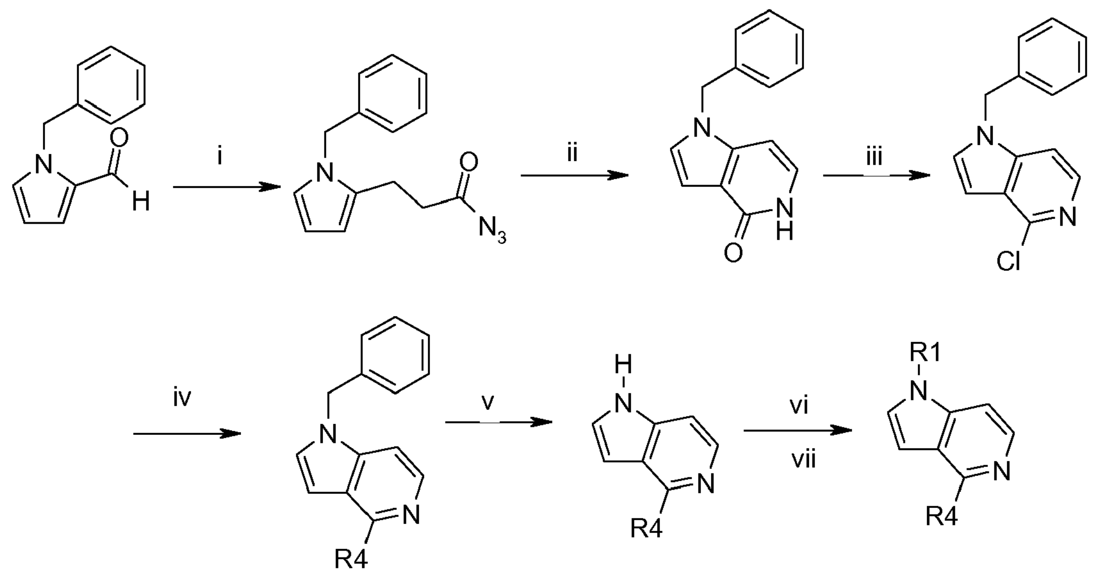


**Legend to Scheme 8:** i) Ethylchloroformate, TEA, acetone, $NaN_3$; ii) $Bu_3N$, DCM and diphenylether; iii) $POCl_3$, NaOH; iv) BOC protected amines ($R^4$), $K_2CO_3$, DMSO; v) $NH_3$ gas Na, $NH_4Cl$, THF vi) Sulphonyl chlorides ($R^1$), NaH, THF; vii) HCl/diethyl ether, methanol.

INTERMEDIATE 88

**(2E)-3-(1-Benzyl-1H-pyrrol-2-yl)acryloyl azide**

To a mixture of 1-benzyl-1H-pyrrole-2-carbaldehyde (28.4g, 0.125mol) and TEA (13.5 mL, 0.187 mol) in acetone (300 mL) was added ethylchloroformate (17.9 mL, 0.87 mol) dropwise. The reaction was stirred for 1.5 h after which $NaN_3$ (13 g, 0.200 mol) in $H_2O$ (100 mL) was added. After 2h, the reaction was diluted with water and left overnight. The acetone was removed and the product was filtered off to afford 21.4 g of a light brown solid. This compound was taken to the next step.

INTERMEDIATE 89

**1-Benzyl-1,5-dihydro-pyrrolo[3,2-c]pyridin-4-one** was prepared by the literature **procedure according to C. Ducrocq; E. Bisangi; J-M, Lhoste; J. Mispelter; Tetrahedron, Vol 32, pp 773-780, (1976).**

To a stirred solution of n-tributylamine (30 mL) in diphenyl ether (150 mL) heated to 195 ˚C was slowly added during 30 minutes a solution of the acyl azide dissolved in DCM (150 mL). The reaction mixture was stirred at 195 ˚C for 1 hour and then cooled to room temperature. Pentane (1.0 L) and ether (1.0 L) was added to the reaction mixture and the precipitate was collected by filtration. The crude solid was triturated with ether to give 6.89 g (81%) of the pure product. Purity HPLC >95%; MS (ESI) m/z 225 (m+H); $^1H$ NMR (DMSO-d6, 25 ˚C, 270.16) δ 10.84 (br s, 1 H), 7.43-7.14 (m, 6 H), 7.00 (d, J = 7.12 Hz, 1 H), 6.57-6.49 (m, 2 H), 5.83 (s, 2 H).

INTERMEDIATE 90

**1-Benzyl-4-chloro-1H-indole**

$POCl_3$ (3.11 mL, 33.4 mmol) was added to 1-benzyl-1,5-dihydro-4H-pyrrolo[3,2-c]pyridin-4-one (3.75 g, 16.7 mmol) and the reaction was stirred at 120˚C for 2h. NaOH (1M) was added and the mixture was extracted with DCM

three times. The organic layers were dried ($MgSO_4$), filtered and the solvent was removed. Flash chromatography (DCM/Heptane/MeOH 4:15:1) gave 1.17 g (29 %) of product. The product was taken to the next step.

INTERMEDIATE 91

**tert-Butyl 4-(1-benzyl-1H-pyrrolo[3,2-c]pyridin-4-yl)piperazine-1-carboxylate**

A mixture of 1-benzyl-4-chloro-1H-indole (1.17 g, 4.82 mmol), $K_2CO_3$ (2.0 g, mmol) and Boc-piperazine (1.79 g, 9.64 mmol) in DMSO (75 mL) was stirred at 120 ˚C for 48 h. Additional of Boc-piperazine (4 equiv.) was added and the reaction was run for another 48 h. The reaction was diluted with ethyl acetate (200 mL) and the mixture was washed with several portions of water. Flash chromatography (DCM/MeOH/Heptane 4:1:15) gave 0.51 g of starting material and 0.38 g of product. $^1$HNMR ($CD_3OD$) δ 7.87-7.85 (m, 1H), 7.25-7.24 (m, 3H), 7.04-6.98 (m, 3H), 6.73-6.71 (m, 1H), 6.53-6.52 (m, 1H), 5.19 (s, 2H), 3.63-3.59 (m, 8H), 1.47 (s, 9H); MS (ESI) 393 $(M + H)^+$; Purity (HPLC, column ACE) 95%

INTERMEDIATE 92

***tert-Butyl*** **4-(1H-pyrrolo[3,2-c]pyridin-4-yl)piperazine-1-carboxylate**

*tert*-Butyl 4-(1-benzyl-1H-pyrrolo[3,2-c]pyridin-4-yl)piperazine-1-carboxylate (383 mg, 0.488 mmol) was dissolved in THF (6 mL) and liquid ammonia (10 mL) in a 30 mL vial. Na (67 mg, 2.93 mmol) was added in portions and the reaction turned violet. After 30 min $NH_4Cl$ (sat) was added and the reaction was let to room temperature The THF was removed and the residue was extracted with DCM. Recrystallization (DCM/Heptane) gave 112 mg of a white solid. $^1$HNMR ($CD_3OD$) δ 8.66 (s, 1H), 7.89 (d, 1H, J = 5.80 Hz), 7.13-7.11 (m, 1H), 6.89-6.86 (m, 1H), 6.57-6.56 (m, 1H), 3.67-3.60 (m, 8H), 1.48 (s, 9H); MS (ESI) 303 $(M + H)^+$; Purity (HPLC, column ACE) 95%.

**Method S for sulphonylation:** *tert*-butyl 4-(1*H*-pyrrolo[3,2-*c*]pyridin-4-yl)piperazine-1-carboxylate (total 1.391 mmol, 1 equiv.) dissolved in THF (14 mL) and dispense to 10 mL vials with screwcap. A suspension of NaH (0.1488 mmol, 1.5 equiv.) in THF (15 mL) was dispense evenly to the vials containing the solution of *tert*-butyl 4-(1*H*-pyrrolo [3,2-*c*]pyridin-4-yl)piperazine-1-carboxylate and stired for approximately for 15 min. Different sulfonylchlorides were dissolved in THF (2 mL) each and added drop-wise to the reaction mixtures. The reactions were quenched with MeOH (100 μL) and PS-Trisamine (3 equiv.) was added to each vial and shake for 2 hours. The mixtures were filtered and the filtrates were concentrate under vacuum. The products that were not pure enough (Purity < 90%) were purified by preparative chromatography using acetonitrile-water gradients containing 0.1% triflouroacetic acid. After HPLC analysis fractions that were ≥ 90% pure were collected and concentrated.

**Method T BOC deprotection;** The Boc-protected compound was dissolved in MeOH (2 mL) and HCL/ether (2 mL) was added. After 45 min the solvent was removed.

INTERMEDIATE 93

***tert*****-Butyl-4-[1-(phenylsulfonyl)-1H-pyrrolo[3,2-c]pyridin-4-yl]piperazine-1-carboxylate**

Purification by recrystallization gave 16 mg (56 %) after Boc-deprotection.
$^1$HNMR ($CDCl_3$) δ 8.03-8.01 (m, 1H), 7.89-7.86 (m, 2H), 7.57-7.39 (m, 5H), 6.67-6.64 (m, 1H), 3.55-3.52 (m, 8H), 1.47 (s, 9H); MS (ESI) 443 $(M + H)^+$; Purity (HPLC, column ACE) 95%.

INTERMEDIATE 94

***tert*****-Butyl-4-{1-[(4-chlorophenyl)sulfonyl]-1H-pyrrolo[3,2-c]pyridin-4-yl}piperazine-1-carboxylate**

Purification by preparative HPLC gave 4 mg (11 %) after Boc-deprotection.
$^1$HNMR ($CDCl_3$) δ 8.03-7.51 (m, 7H), 6.89-6.87 (m, 1H), 3.91-3.66 (m, 8H), 1.47 (s, 9H); MS (ESI) 377 $(M + H)^+$; Purity (HPLC, column ACE) 95%.

INTERMEDIATE 95

***tert*****-Butyl-4-{1-[(4-methoxyphenyl)sulfonyl]-1H-pyrrolo[3,2-c]pyridin-4-yl}piperazine-1-carboxylate**

Purification by recrystallization (MeOH/Ether) gave 21 mg (67 %) after boc-deprotection.
$^1$HNMR ($CDCl_3$) δ 8.02-8.00 (m, 1H), 7.84-7.80 (m, 2H), 7.48-7.46 (m, 1H), 7.41-7.38 (m, 1H), 6.92-6.86 (m, 2H),

6.64-6.62 (m, 1H), 3.79 (s, 3H), 3.57-3.52 (m, 8H), 1.48 (s, 9H); MS (ESI) 473 $(M + H)^+$; Purity (HPLC, column ACE) 95%.

INTERMEDIATE 96

**tert-Butyl 4-(1-{[2-(trifluoromethyl)phenyl]sulfonyl}-1H-pyrrolo[3,2-c]pyridin-4-yl)piperazine-1-carboxylate**

Purification by preparative HPLC gave 8.6 mg (25 %) after boc-deprotection. $^1$HNMR ($CDCl_3$) δ 8.14-8.11 (m, 1H), 8.01-7.94 (m, 2H), 7.89-7.72 (m, 3H), 7.37-7.34 (m, 1H), 6.89-6.88 (m, 1H), 3.93-3.89 (m, 4H), 3.71-3.67 (m, 4H), 1.47 (s, 9H); MS (ESI) 511 $(M + H)^+$; Purity (HPLC, column ACE) 95%.

INTERMEDIATE 97

**tert-Butyl 4-{1-[(2-methoxy-5-methylphenyl)sulfonyl]-1H-pyrrolo[3,2-c]pyridin-4-yl}piperazine-1-carboxylate**

Purification by preparative HPLC gave 10.3 mg (32 %) after boc-deprotection. $^1$HNMR (CDCl3) δ 7.95-7.92 (m, 2H), 7.74-7.72 (m, 1H), 7.44-7.40 (m, 2H), 6.85-6.77 (m, 2H), 3.92-3.88 (m, 4H), 3.70 (s, 3H), 3.69-3.66 (m, 4H), 2.39 (s, 3H), 1.47 (s, 9H); MS (ESI) 487 $(M + H)^+$; Purity (HPLC, column ACE) 95%.

EXAMPLE 121

**4-Piperazin-1-yl-1-(toluene-4-sulfonyl)-1H-pyrrolo[3,2-c]pyridine hydrochloride**

p-Toulenesulfonyl chloride (24.6 mg) was added to *tert*-butyl 4-(1*H*-pyrrolo[3,2-*c*]pyridin-4-yl)piperazine-1-carboxylate the title compound (4.3 mg). LC/MS $R_T$: 1.374 (System 10 till 40% MeCN over 1.5 min, ACE C8), Purity. 91%. MS: 357 (M+1) $^1$HNMR ($CD_3OD$) δ ppm 2.39 (s, 3 H) 3.48 (m, 4 H) 4.06 (m, 4 H) 7.22 (d, *J*=3.71 Hz, 1 H) 7.43 (d, *J*=8.16 Hz, 2 H) 7.95 (m, 5H).

EXAMPLE 122

**1-(3-Chloro-2-methyl-benzenesulfonyl)-4-piperazin-1-yl-1H-pyrrolo[3,2-c]pyridine hydrochloride**

3-Chloro-2-methylbenzenesulfonyl chloride (29.0 mg) was added to *tert*-butyl 4-(1*H*-pyrrolo[3,2-c]pyridin-4-yl) piperazine-1-carboxylate the title compound (6.3 mg). LC/MS $R_T$: 1.563 (System 10 till 40% MeCN over 1.5 min, ACE C8), Purity. 96%. MS: 392 (M+1).

EXAMPLE 123

**1-(3,4-Dimethoxy-benzenesulfonyl)-4-piperazin-1-yl-1H-pyrrolo[3,2-c]pyridine hydrochloride**

3,4-Dimethoxybenzenulfonyl chloride (30.5 mg) was added to *tert*-butyl 4-(1*H*-pyrrolo[3,2-*c*]pyridin-4-yl)piperazine-1-carboxylate the title compound (8.5 mg). LC/MS $R_T$: 1.284 (System 10 till 40% MeCN over 1.5 min, ACE C8), Purity. 92%. MS: 404 (M+1) $^1$HNMR ($CD_3OD$) δ ppm 3.50 (m, *J*=4.21 Hz, 2 H) 3.85 (d, *J*=3.22 Hz, 4 H) 4.10 (m, *J*=3.96 Hz, 2 H) 7.11 (d, *J*=8.66 Hz, 1 H) 7.23 (d, *J*=3.46 Hz, 1 H) 7.48 (d, *J*=1.73 Hz, 1 H) 7.74 (dd, *J*=8.54, 1.86 Hz, 1 H) 7.92 (s, 2 H) 8.07 (d, *J*=3.46 Hz, 1 H).

EXAMPLE 124

**4-(4-Piperazin-1-yl-pyrrolo[3,2-c]pyridine-1-sulfonyl)-benzonitrile hydrochloride**

4-Cyanobenzenesulfonyl chloride (26.0mg) was added to *tert*-butyl 4-(1*H*-pyrrolo[3,2-*c*]pyridin-4-yl)piperazine-1-carboxylate the title compound (9.1 mg). LC/MS $R_T$: 1.150 (System 10 till 40% MeCN over 1.5 min, ACE C8), Purity. 93%. MS: 369 (M+1) $^1$HNMR ($CD_3OD$) δ ppm 3.50 (m, 4 H) 4.08 (m, 4 H) 7.29 (d, *J*=3.71 Hz, 2 H) 7.98 (m, 4 H) 8.29 (d, *J*=8.66 Hz, 2 H).

EXAMPLE 125

**1-(4,5-Dichloro-thiophene-2-sulfonyl)-4-piperazin-1-yl-1H-pyrrolo[3,2-c]pyridine hydrochloride**

4,5-Dichloro-thiophene-2-sulfonyl chloride (32.4 mg) was added to *tert*-butyl 4-(1*H*-pyrrolo[3,2-c]pyridin-4-yl) piperazine-1-carboxylate the title compound (0.3 mg). LC/MS $R_T$: 1.119 (System 10 till 40% MeCN over 1.5 min, ACE C8), Purity. 92%. MS: 418 (M+1).

EXAMPLE 126

**1-(2-Chloro-4-fluoro-benzenesulfonyl)-4-piperazin-1-yl-1H-pyrrolo[3,2-c]pyridine hydrochloride**

2-Chloro-4-flourobenzenesulfonyl chloride (29.5 mg) was added to *tert*-butyl 4-(1*H*-pyrrolo[3,2-c]pyridin-4-yl) piperazine-1-carboxylate the title compound (2.4 mg). LC/MS $R_T$: 1.361 (System 10 till 40% MeCN over 1.5 min, ACE C8), Purity. 90%. MS: 396 (M+1) $^1$HNMR ($CD_3OD$) δ ppm 3.51 (m, 4 H) 4.08 (m, 4 H) 7.23 (dd, *J*=3.96, 0.49 Hz, 1 H) 7.47 (m, 1 H) 7.55 (dd, *J*=8.41, 2.47 Hz, 2 H) 7.62 (d, *J*=6.93 Hz, 1 H) 7.91 (d, *J*=7.18 Hz, 1 H) 8.06 (d, *J*=3.96 Hz, 1 H).

EXAMPLE 127

**1-Phenylmethanesulfonyl-4-piperazin-1-yl-1H-pyrrolo [3,2-c]pyridine hydrochloride**

Phenyl-methanesulfonyl chloride (24.6 mg) was added to *tert*-butyl 4-(1*H*-pyrrolo[3,2-c]pyridin-4-yl)piperazine-1-carboxylate the title compound (0.2 mg). LC/MS $R_T$: 1.007 (System 10 till 40% MeCN over 1.5 min, ACE C8), Purity. 90%. MS: 357 (M+1).

EXAMPLE 128

**1-(5-Chloro-thiophene-2-sulfonyl)-4-piperazin-1-yl-1H-pyrrolo[3,2-c]pyridine hydrochloride**

5-Chlorothiophene-2-sulfonyl chloride (28.0 mg) was added to *tert*-butyl 4-(1*H*-pyrrolo[3,2-*c*]pyridin-4-yl)piperazine-1-carboxylate the title compound (7.2 mg). LC/MS $R_T$: 1.381 (System 10 till 40% MeCN over 1.5 min, ACE C8), Purity. 97%. MS: 483 (M+1).

EXAMPLE 129

**1-(4-Butyl-benzenesulfonyl)-4-piperazin-1-yl-1H-pyrrolo(3,2-c)pyridine hydrochloride**

4-N-Butylbenzenesulfonylchloride (30.0 mg) was added to *tert*-butyl 4-(1*H*-pyrrolo[3,2-*c*]pyridin-4-yl)piperazine-1-carboxylate the title compound (11.9 mg). LC/MS $R_T$: 1.904 (System 10 till 40% MeCN over 1.5 min, ACE C8), Purity. 95%. MS: 400 (M+1) $^1$HNMR ($CD_3OD$) δ ppm 0.90 (t, *J*=7.18 Hz, 3 H) 1.31 (m, 2 H) 1.55 (m, 2 H) 2.67 (m, 2 H) 3.50 (m, 4 H) 4.09 (m, *J*=3.96 Hz, 4 H) 7.25 (d, *J*=3.71 Hz, 2 H) 7.44 (d, *J*=8.16 Hz, 2 H) 7.91 (m, 2 H) 8.02 (m, 2 H).

EXAMPLE 130

**1-(4-Phenoxy-benzenesulfonyl)-4-piperazin-1-yl-1H-pyrrolo(3,2-c)pyridine hydrochloride**

(4-Phenoxy)benzene)sulfonyl chloride (34.7 mg) was added to *tert*-butyl 4-(1*H*-pyrrolo[3,2-c]pyridin-4-yl)piperazine-1-carboxylate the title compound (12.8 mg). LC/MS $R_T$: 1.839 (System 10 till 40% MeCN over 1.5 min, ACE C8), Purity. 95%. MS: 436 (M+1) $^1$HNMR ($CD_3OD$) δ ppm 3.50 (m, *J*=3.96 Hz, 4 H) 4.09 (m, *J*=4.45 Hz, 4 H) 7.05 (dd, *J*=8.16, 6.43 Hz, 2 H) 7.26 (m, 2 H) 7.44 (t, *J*=7.79 Hz, 2 H) 7.90 (m, 3 H) 8.01 (d, *J*=3.71 Hz, 2 H) 8.07 (d, *J*=8.91 Hz, 2 H).

EXAMPLE 131

**1-(Phenylsulfonyl)-4-piperazin-1-yl-1H-pyrrolo[3,2-c]pyridine hydrochloride**

Purification by recrystallization gave 16 mg (56 %) after Boc-deprotection. MS (ESI) 343.1 $(M + H)^+$; Purity (HPLC, column ACE) 94%.

EXAMPLE 132

**1-[(4-Chlorophenyl)sulfonyl]-4-piperazin-1-yl-1H-pyrrolo[3,2-c]pyridine hydrochloride**

Purification by preparative HPLC gave 4 mg (11 %) after Boc-deprotection. MS (ESI) 377 $(M + H)^+$; Purity (HPLC, column ACE) 96%.

EXAMPLE 133

**1-[(4-Methoxyphenyl)sulfonyl]-4-piperazin-1-yl-1H-pyrrolo [3,2-c] pyridine hydrochloride**

Purification by recrystallization (MeOH/Ether) gave 21 mg (67 %) after Boc-deprotection. MS (ESI) 373 $(M + H)^+$; Purity (HPLC, column ACE) 92%

EXAMPLE 134

**1-[(2-Methoxy-5-methylphenyl)sulfonyl]-4-piperazin-1-yl-1H-pyrrolo[3,2-c]pyridine hydrochloride**

Purification by preparative HPLC gave 10.3 mg (32 %) after Boc-deprotection. MS (ESI) 387 $(M + H)^+$; Purity (HPLC, column ACE) 95%.

EXAMPLE 135

**4-Piperazin-1-yl-1-{[2-(trinuoromethyl)phenyl]sulfonyl}-1H-pyrrolo[3,2-c]pyridine hydrochloride**

Purification by preparative HPLC gave 8.6 mg (25 %) after Boc-deprotection. MS (ESI) 411 $(M + H)^+$; Purity (HPLC, column ACE) 94%.

BIOLOGICAL TESTS

The ability of a compound according to the invention to bind a 5-$HT_6$ receptor, and to be pharmaceutically useful, can be determined using *in vivo and in vitro* assays known in the art.

*(a) 5-$HT_6$ binding Assay*

Binding affinity experiment for the 5-$HT_6$ receptor are performed in HEK293 cells transfected with 5-$HT_6$ receptor using (3H)-LSD as labeled ligand according to the general method as described by Boess F.G et al. Neuropharmacology vol. 36(4/5) 713-720, 1997.

**Materials**

Cell culture

The HEK-293 cell line transfected with the 5-$HT_6$ receptor was cultured in Dulbeccos Modified Eagles Medium containing 5 % dialyzed foetal bovine serum, (Gibco BRL 10106-169), 0.5 mM sodium pyruvate and 400 μg/ml Geneticin (G-418) (Gibco BRL10131-019). The cells were passaged 1:10, twice a week.

Chemicals

The radio ligand [$^3$H] LSD 60-240 Ci/mmol, obtained from Amersham Pharmacia Biotech, (Buckinghamshire, England) was in ethanol and stored at -20°C. The unlabelled ligands, representing different selectivity profiles, are presented in Table 1. The compounds were dissolved in 100% DMSO and diluted with binding buffer.

Disposable

Compounds were diluted in Costar 96 well V-bottom polypropylene plates (Coming Inc. Costar, NY, USA). Samples were incubated in Packard Optiplate (Packard Instruments B.V., Groningen, The Netherlands). The total amount of added radio ligand was measured in Packard 24-well Barex plates (Packard Instruments B.V., Groningen, The Netherlands) in the presence of Microscint™ 20 scintillation fluid (Packard Bioscience, Meriden, CT, USA).

Buffer

The binding buffer consisted of 20 mM HEPES, 150 mM NaCl, 10 mM $MgCl_2$, and 1 mM, EDTA, pH 7.4.

**Methods**

Membrane preparation

Cells were grown to approximately 90% confluence on 24.5 x 24.5 NUNC culture dishes. The medium was aspirated, and after rinsing with ice-cold PBS, the cells were scraped off using 25 ml Tris buffer (50 mM Tris-HCl, 1 mM EDTA, 1 mM EGTA, pH 7.4) and a window scraper. The cells were then broken with a Polytron homogeniser, and remaining particulate matter was removed by low-speed centrifugation, 1000x g for 5 min. Finally, the membranes were collected by high-speed centrifugation (20 000x g), suspended in binding buffer, and frozen in aliquots at -70°C.

Radio ligand binding

Frozen cell membranes were thawed, immediately rehomogenized with a Polytron homogenizer, and coupled to SPA wheat germ agglutinin beads (Amersham Life Sciences, Cardiff, England) for 30 min under continuous shaking of the tubes. After coupling, the beads were centrifuged for 10 minutes at 1000 g, and subsequently suspended in 20 ml of binding buffer per 96-well plate. The binding reaction was then initiated by adding radio ligand and test compounds to the bead-membrane suspension. Following incubation at room temperature, the assay plates were subjected to scintillation counting. The original SPA method was followed except for that membranes were prepared from HEK293 cells expressing the human 5-$HT_6$ receptor instead of from HeLa cells (Dinh DM, Zaworski PG, Gill GS, Schlachter SK, Lawson CF, Smith MW. Validation of human 5-$HT_6$ receptors expressed in HeLa cell membranes: saturation binding studies, pharmacological profiles of standard CNS agents and SPA development. The Upjohn Company Technical Report 7295-95-064 1995;27 December). The specific binding of [$^3$H]LSD was saturable, while the non-specific binding increased linearly with the concentration of added radio ligand. [$^3$H] LSD bound with high affinity to 5-$HT_6$ receptors. The $K_d$ value was estimated to 2.6± 0.2 nM based on four separate experiments.

The total binding at 3 nM of [$^3$H] LSD, the radio ligand concentration used in the competition experiments, was typically 6000 dpm, and the specific binding more than 70%. 5-HT caused a concentration dependent inhibition of [$^3$H] LSD binding with an over all average Ki value of 236 nM when tested against two different membrane preparations. The inter assay variability over three experiments showed a CV of 10% with an average $K_i$ values of 173 nM (SD 30) and a Hill coefficient of 0.94 (SD 0.09). The intra assay variation was 3% (n=4). Ki values for a limited set of reference compounds with reported binding affinities at 5-$HT_6$ receptor are presented in Table 7. All unlabelled ligands displaced the specific binding of [$^3$H] LSD in a concentration-dependent manner, albeit at different potencies. The rank order of potency for the compounds was methiothepin (2 nM) >mianserin (190 nM) ≈ 5-HT (236 nM) >methysergide (482 nM) >mesulergide (1970 nM).

**Protein determination**

Protein concentrations were determined with BioRad Protein Assay (Bradford MM. A rapid and sensitive method for the quantitation of microgram quantities of protein utilizing the principle of protein-dye binding. Anal Biochem 1976; 72:248-54). Bovine serum albumin was used as standard.

**Scintillation counting**

The radioactivity was determined in a Packard TopCount™ scintillation counter (Packard Instruments, Meriden, CT, USA) at a counting efficiency of approximately 20 %. The counting efficiency was determined in separate sets of experiments.

**Saturation experiments**

At least 6 concentrations in duplicates of radioligand (0.1-20 nM of [$^3$H] LSD) were used in saturation experiments. The specific binding was calculated as the difference between total binding and non-specific binding, which was determined as the binding of radioligand in the presence of 5 μM lisuride. $B_{max}$ and the dissociation constant, $K_d$, were determined from the non-linear regression analysis using equation 1. $L_u$ is the unbound concentration of radio ligand,

and is *y* is the amount bound.

$$y = \frac{B_{\max} \cdot Lu}{Lu + Kd} \qquad \text{(equation 1)}$$

**Competition experiments**

Total- and non-specific binding of radioligand was defined in eight replicates of each. Samples containing test compound were run in duplicate at 11 concentrations. Incubations were carried out at room temperature for 3 hours. The $IC_{50}$ value, i.e. the concentration of test compound that inhibited 50% of the specific binding of radioligand, was determined with non linear regression analysis and the $K_i$ value was calculated using the method of [Cheng Y.C. Biochem. Pharmacol. 22, 3099-3108, 1973S] equation 2.

$$Ki = \frac{IC_{50}}{1 + \frac{L}{K_d}} \qquad \text{(equation 2)}$$

L = concentration of radioligand
$K_d$ = Affinity of radio ligand

*(b) 5-HT$_6$ Intrinsic Activity Assay*

Antagonists to the 5-$HT_6$ receptor were characterized by measuring inhibition of 5-HT induced increase in cAMP in HEK 293 cells expressing the human 5-$HT_6$ receptor (see Boess et al. (1997) Neuropharmacology 36: 713-720). Briefly, HEK293/5-$HT_6$ cells were seeded in polylysine coated 96-well plates at a density of 25,000 / well and grown in DMEM (Dulbecco's Modified Eagle Medium) (without phenol-red) containing 5% dialyzed Foetal Bovine Serum for 48 h at 37˚C in a 5% $CO_2$ incubator. The medium was then aspirated and replaced by 0.1 ml assay medium (Hanks Balance Salt Solution containing 20 mM HEPES, 1.5 mM isobutylmethylxanthine and 1 mg/ml bovine serum albumin). After addition of test substances, 50 μl dissolved in assay medium, the cells were incubated for 10 min at 37˚C in a 5% $CO_2$ incubator. The medium was again aspirated and the cAMP content was determined using a radioactive cAMP kit (Amersham Pharmacia Biotech, BIOTRAK RPA559). The potency of antagonists was quantified by determining the concentration that caused 50% inhibition of 5-HT (at [5-HT]= 8 times $EC_{50}$) evoked increase in cAMP, using the formula $IC_{50,corr}=IC_{50}/(1+[5HT]/EC_{50})$.

The compounds in accordance with the invention have a selective affinity to 5-$HT_6$ receptors with Ki and $IC_{50,corr}$ values between 0.5 nM and 5 μM or display a % inhibition of [$^3$H] LSD ≥ 20 % at 50 nM and are antagonists, agonist or partial agonist at 5-$HT_6$ . The compounds show good selectivity over 5-$HT_{1a}$, 5-$HT_{2a}$, 5-$HT_{2a}$, 5-$HT_{2b}$, 5-$HT_{2c}$.

*(c) In vivo assay of reduction of food intake*

For a review on serotonin and food intake, see Blundell, J.E. and Halford, J.C.G. (1998) Serotonin and Appetite Regulation. Implications for the Pharmacological Treatment of Obesity. CNS Drugs 9:473-495.

Obese (ob/ob) mouse is selected as the primary animal model for screening as this mutant mouse consumes high amounts of food resulting in a high signal to noise ratio. To further substantiate and compare efficacy data, the effect of the compounds on food consumption is also studied in wild type (C57BL/6J) mice. The amount of food consumed during 15 hours of infusion of compounds is recorded.

Male mice (obese C57BL/6JBom-Lep$^{ob}$ and lean wild-type C57B1/6JBom; Bomholtsgaard, Denmark) 8-9 weeks with an average body weight of 50 g (obese) and 25 g (lean) are used in all the studies. The animals are housed singly in cages at 23±1˚C, 40-60 % humidity and have free access to water and standard laboratory chow. The 12/12-h light/dark cycle is set to lights off at 5 p.m. The animals are conditioned for at least one week before start of study.

The test compounds are dissolved in solvents suitable for each specific compound such as cyclodextrin, cyclodextrin/methane sulfonic acid, polyethylene glycol/methane sulfonic acid, saline. Fresh solutions are made for each study. Doses of 30, 50 and 100 mg $kg^{-1}day^{-1}$ are used. The purity of the test compounds is of analytical grade.

The animals are weighed at the start of the study and randomized based on body weight. Alzet osmotic

minipumps (Model 2001D; infusion rate 8 μl/h) are used and loaded essentially as recommended by the Alzet technical information manual (Alza Scientific Products, 1997; Theeuwes, F. and Yam, S.I. Ann. Biomed. Eng. 4(4). 343-353, 1976). Continuous subcutaneous infusion with 24 hours duration is used. The minipumps are either filled with different concentrations of test compounds dissolved in vehicle or with only vehicle solution and maintained in vehicle pre-warmed to 37˚C (approx. 1h). The minipumps are implanted subcutaneously in the neck/back region under short acting anesthesia (metofane/enflurane). This surgical procedure lasts approximately 5 min. It takes about 3 h to reach steady state delivery of the compound.

The weight of the food pellets are measured at 5 p.m. and at 8 p. m. for two days before (baseline) and one day after the implantation of the osmotic minipumps. The weigh-in is performed with a computer assisted Mettler Toledo PR 5002 balance. Occasional spillage is corrected for. At the end of the study the animals are killed by neck dislocation and trunk blood sampled for later analysis of plasma drug concentrations.

The plasma sample proteins are precipitated with methanol, centrifuged and the supernatant is transferred to HPLC vials and injected into the liquid chromatography /mass spectrometric system. The mass spectrometer is set for electrospray positive ion mode and Multiple Reaction Monitoring. A linear regression analysis of the standards forced through the origin is used to calculate the concentrations of the unknown samples.

Food consumption for 15 hours is measured for the three consecutive days and the percentage of basal level values is derived for each animal from the day before and after treatment. The values are expressed as mean ± SD and ± SEM from eight animals per dose group. Statistical evaluation is performed by Kruskal-Wallis one-way ANOVA using the percent basal values. If statistical significance is reached at the level of $p<0.05$, Mann-Whitney U-test for statistical comparison between control and treatment groups is performed.

The compounds according to the invention show an effect in the range of 5-200 mg/kg.

**Table 7. Biological data**

| In vitro binding at the human 5-$HT_6$ receptor | |
|---|---|
| **EXAMPLE** | **$K_i$ (nM) human 5-$HT_6$** |
| **43** | 4.5 |
| **68** | 13 |
| **85** | 32 |
| **131** | 5 |

## Claims

**1.** A compound of the formula (I):

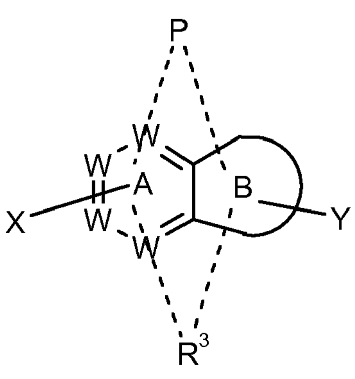

(I)

or a pharmaceutically acceptable salt thereof, wherein:
ring B is

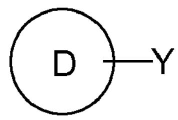

;

in which D is a five-membered heterocyclic or heteroaryl ring, said ring comprising one or two atoms selected from the group consisting of nitrogen, sulfur and oxygen, with the proviso that when D contains an oxygen atom, D is heteroaryl;
each W is independently -N-, -(CH)-, or -C- provided that not more than three groups W are -N- ;
P is any one of formula (a), (b) or (c)

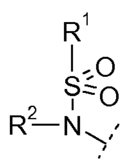 , 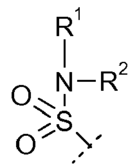 , or 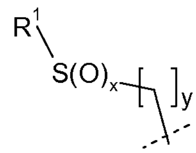 ;

(a) (b) (c)

wherein x = 0, 1, or 2 and y = 0, 1, or 2;
and P and $R^3$ can be attached to any carbon atom that allows the substitution in one of either the A- or B-ring, or when ring A contains at least one nitrogen atom and P is (c), then P can also be attached to any nitrogen in ring B that allows the substitution;
the dashed bonds denote that P and $R^3$, respectively, may be attached to either the A or B ring; but each P or $R^3$ may not be simultaneously bound to both rings A and B;

$R^1$ is

(a) $C_{1-6}$ alkyl,
(b) $C_{1-6}$ alkoxyalkyl,
(c) straight-chained or branched $C_{1-6}$ hydroxyalkyl,
(d) straight-chained or branched $C_{1-6}$ alkylhalides,
(e) aryl carbonylmethyl,
(f) $C_{3-7}$ cycloalkyl, which is optionally partially unsaturated,
(g) $C_{3-7}$ cycloalkyl-$C_{1-6}$ alkyl, wherein the cyclic ring is optionally partially unsaturated, or
(h) a group Ar;

wherein Ar is

(a) phenyl,
(b) 1-naphthyl,
(c) 2-naphthyl,
(d) aryl-$C_{1-6}$ alkyl,
(e) cinnamyl,
(f) a 5 to 7-membered, optionally aromatic, partially saturated or completely saturated, mono- or bi-cyclic heterocyclic ring, each containing 1 to 4 heteroatoms, selected from oxygen, sulfur, and nitrogen,
(g) a bicyclic ring system comprising at least one heterocyclic ring according to (f) and a group Ar, wherein the group Ar is substituted in one or more positions with

(a) H, X or Y, or
(b) a 5 to 7-membered, optionally aromatic, partially saturated or completely saturated, heterocyclic ring each containing 1 to 4 heteroatoms selected from oxygen, nitrogen or sulfur;

$R^2$ is

(a) H,
(b) $C_{1-6}$ alkyl,
(c) $C_{2-6}$ alkoxyalkyl,
(d) straight or branched $C_{1-6}$ hydroxyalkyl, or
(e) straight or branched $C_{1-6}$ alkylhalides;
(f) a group Ar,
or $R^1$ and $R^2$ are linked to form a group $-CH_2CH_2OCH_2CH_2-$ or

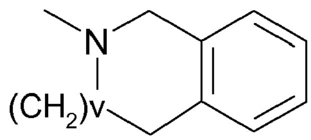

wherein v is 0-2,
X and Y are independently

(a) H,
(b) halogen,
(c) $C_{1-6}$ alkyl,
(d) $CF_3$,
(e) hydroxy,
(f) $C_{1-6}$ alkoxy,
(g) $C_{2-6}$ alkenyl,
(h) phenyl,
(i) phenoxy,
(j) benzyloxy,
(k) benzoyl,
(1)-$OCF_3$,
(m) -CN,
(n) straight or branched $C_{1-6}$ hydroxyalkyl,
(o) straight or branched $C_{1-6}$ alkylhalides,
(p) $-NH_2$,
(q) $-NHR^4$,
(r) $-NR^4R^5$,
(s) $-NO_2$,
(t) $-CONR^4R^5$,
(u) $-NHSO_2R^4$,
(v) $-NR^4COR^5$,
(x) $-SO_2NR^4R^5$,
(z) $-C(=O)R^4$,
(aa) $-CO_2R^4$, or
(ab) $-S(O)_nR^4$, wherein n is 0, 1, 2 or 3,
(ac) $-S-(C_{1-6})$ alkyl, or
(ad) $-SCF_3$; and

$R^4$ and $R^5$ are independently

(a) H,
(b)$C_{1-6}$ alkyl,
(c) $C_{3-7}$ cycloalkyl, or
(d) Ar, as defined above for $R^1$;

alternatively, $R^4$ and $R^5$ are linked to form a group $-CH_2OCH_2-$, $-CH_2CH_2OCH_2CH_2-$ or $(CH_2)_{3-5}$;
$R^3$ is a group selected from any one of

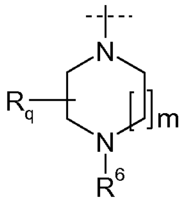
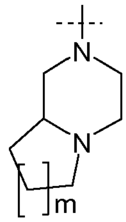
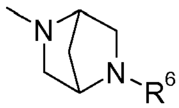
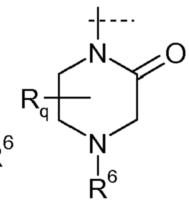
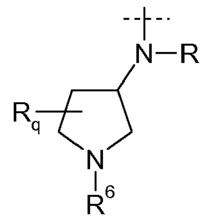

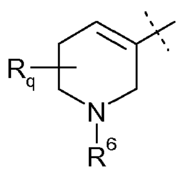
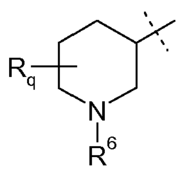
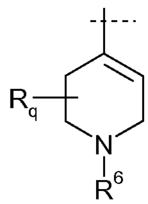
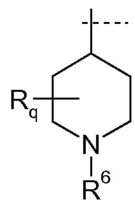
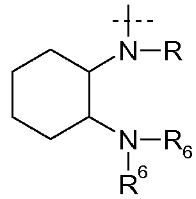

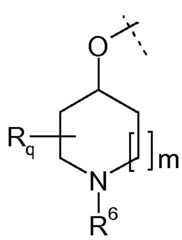
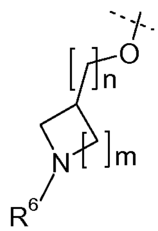
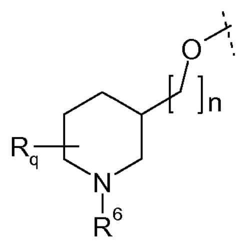
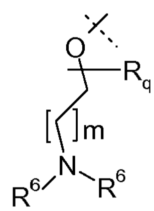

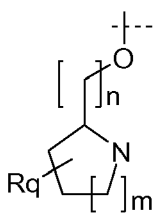
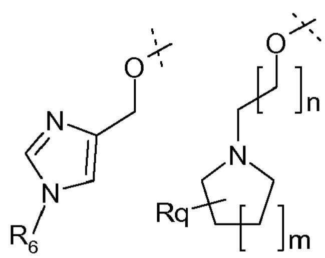
and
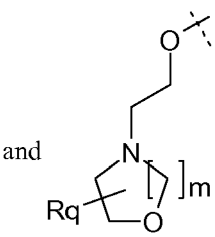

wherein $R^3$ is optionally substituted on each carbon atom that allows the substitution with Rq groups, wherein Rq is independently H, or $(C_{1-6})$ alkyl, and
wherein two Rq groups can be present on the same carbon atom simultaneously,
wherein

q=1,2,3,4,5 or 6,
m = 1 or 2, and
n = 0, 1 or 2;

$R^6$ is independently

(a) H,
(b) linear or branched $C_{1-6}$ alkyl,
(c) benzyl,
(d) $-CH_2-CH_2-OH$, or
(e) $-CH_2-CH_2-O-C_{1-6}$ alkyl;

P and $R^3$ can be attached to the same ring or to different rings of rings A and B;
provided that
when P is

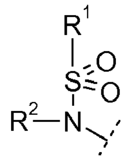 (a) or 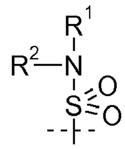 (b),

and P and $R^3$ both are attached to ring A in the meta- or para-position relative to one another then $R^3$ is selected from any one of

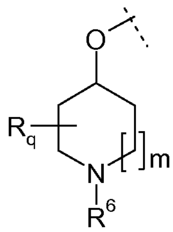 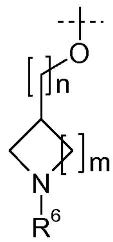 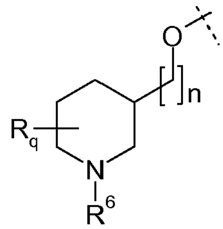 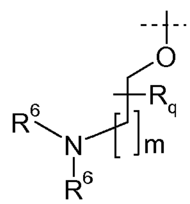

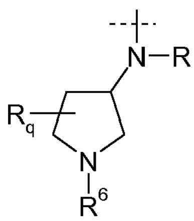 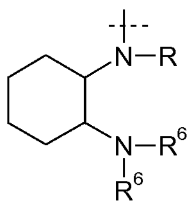 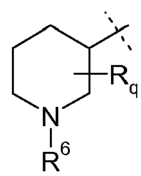 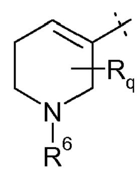

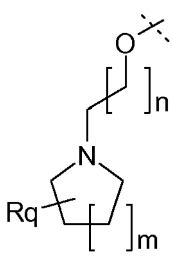 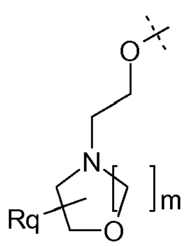 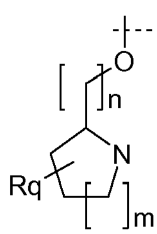 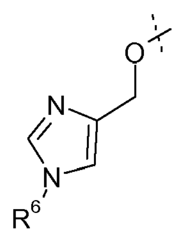 ;

when the ring system A + B is benzofurane or benzothiophene, and P is

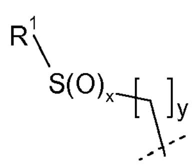 (c)

and attached to position 3 in the A+B ring system, and $R^3$ is a group selected from any one of

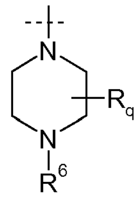 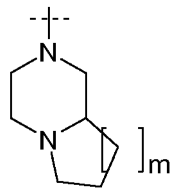 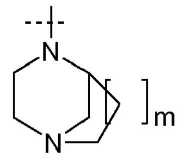

and attached to position 7 in the A+B ring system, then y = 1 or 2;
when the ring system A + B is indole, and P is

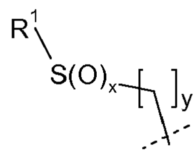 (c)

and P is attached to position 3 in the A+B ring system, and $R^3$ is a group selected from any one of

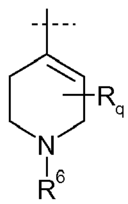 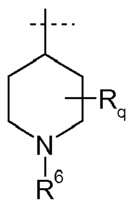 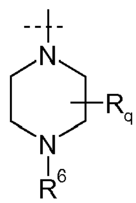 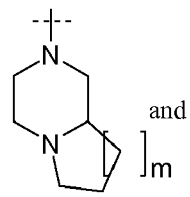 and 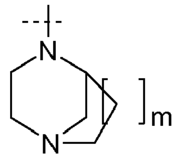

and $R^3$ is attached to any one of positions 5, 6 or 7 in the A+B ring system, then y = 1 or 2; or
when ring D is a pyrrole ring, P is of the formula (c), then $R^3$ is not of the formula

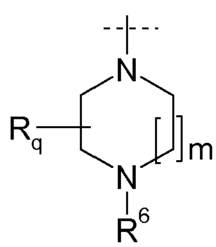 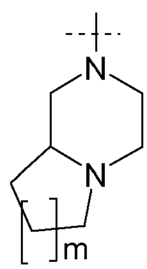 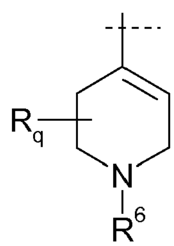 or 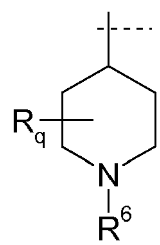

substituted in position 3 on the pyrrole ring.

**2.** The compound according to claim 1, wherein

$R^1$ is

(a) $C_{1-6}$ alkyl, or
(e) a group Ar;

Ar is

(a) phenyl,

(b) 1-naphthyl,
(c) 2-naphthyl, or
(f) a 5 to 7-membered, optionally aromatic, partially saturated or completely saturated, heterocyclic ring containing 1 to 4 heteroatoms, selected from oxygen, nitrogen and sulfur,

wherein the group Ar is substituted in one or more positions with

(a) H,
(b) halogen,
(c) $C_{1-6}$ alkyl,
(d) $-CF_3$,
(f) $C_{1-6}$ alkoxy,
(g) $C_{2-6}$ alkenyl,
(1) $-OCF_3$,
(m) straight or branched $C_{1-6}$ hydroxyalkyl,
(n) phenyloxy,
(o) benzyloxy,
(v) $-NR^4COR^5$,
(x) $-SO_2NR^4R^5$,
(z) $-C(=O)R^4$,
(ab) $-S(O)_nR^4$, wherein n is 0, 1, 2 or 3;
(ac) $-S-(C_{1-6})$ alkyl, or
(ad) $-SCF_3$;

$R^2$ is

(a) H, or
(b) $C_{1-6}$ alkyl;
or $R^1$ and $R^2$ are linked to form a group $-CH_2CH_2OCH_2CH_2-$;

X and Y are H;
$R^4$ and $R^5$ are each independently H or $C_{1-3}$ alkyl; and
$R^3$ is selected from any one of

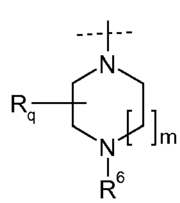
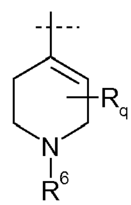
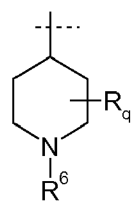

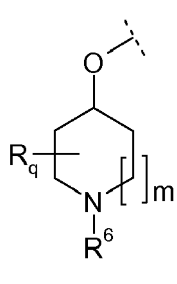
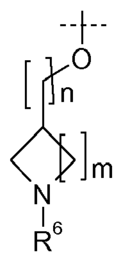
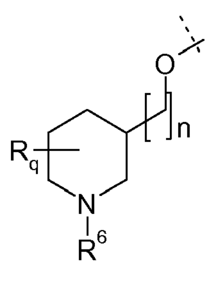
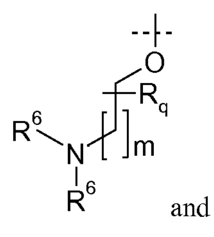
and
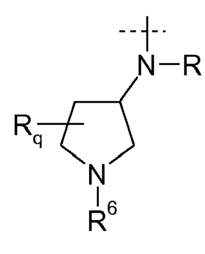

wherein $R^3$ can be substituted on each carbon atom that allows the substitution with Rq groups, wherein Rq is independently H, or $C_{1-6}$ alkyl, and wherein two Rq groups can be present on the same carbon atom simultaneously, wherein

q = 1 or 2,
m = 1 or 2,
n = 0, and

$R^6$ is independently

(a) H,
(b) $C_{1-6}$ alkyl, in particular methyl,
(d) $-CH_2-CH_2-OH$, or
(e) $-CH_2-CH_2-OCH_3$.

**3.** The compound according to claims 1 or 2 wherein $R^3$ is selected from any one of

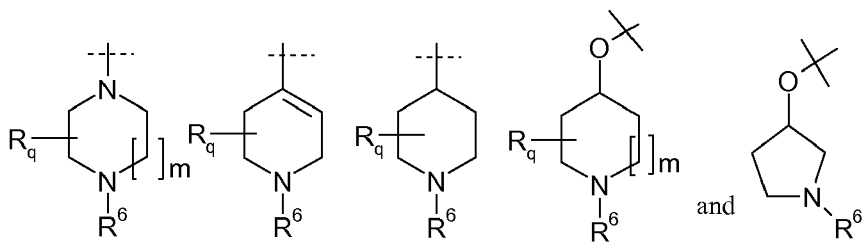

and

wherein $R^3$ can be substituted on each carbon atom that allows the substitution with Rq groups, wherein Rq is independently H, or $C_{1-2}$ alkyl, and wherein two Rq groups can be present on the same carbon atom simultaneously, wherein

q = 1 or 2,
m = 1 or 2; and
$R^6$ is independently

(a) H,
(b) $C_{1-3}$ alkyl,
(d) $-CH_2-CH_2-OH$, or
(e) $-CH_2-CH_2-OCH_3$.

**4.** The compound according to claim 1 or 2 wherein $R^3$ is selected from any one of

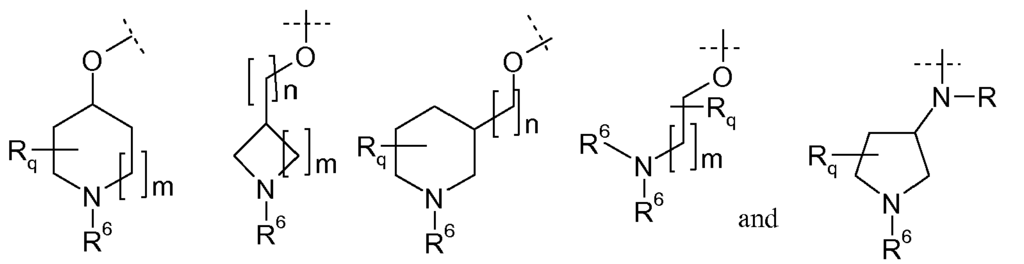

and

wherein $R^3$ can be substituted on each carbon atom that allows the substitution with Rq groups, wherein Rq is independently H, or $C_{1-6}$ alkyl, and wherein two Rq groups can be present on the same carbon atom simultaneously, wherein

q = 1 or 2,
m = 1 or 2,
n = 0, and
$R^6$ is independently

(a) H,
(b)$C_{1-3}$ alkyl,
(d) $-CH_2-CH_2-OH$, or
(e) $-CH_2-CH_2-OCH_3$.

**5.** The compound according to claim 1 or 2 wherein $R^3$ is selected from any one of

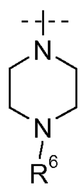 ; 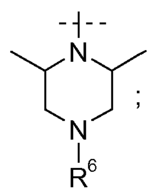 ; 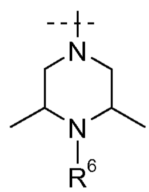 ; 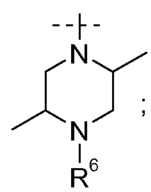 ; 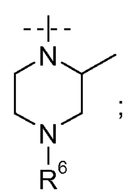 ;

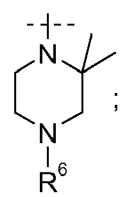 ; 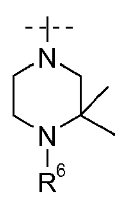 ; 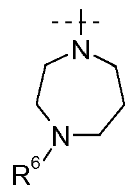 ; 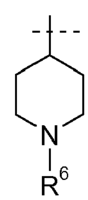 ; 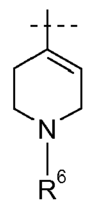 ;

$R^6$ is independently

(a) H,
(b) $C_{1-3}$ alkyl,
(d) $-CH_2-CH_2-OH$, or
(e) $-CH_2-CH_2-OCH_3$.

**6.** A compound according to any one of claims 1 to 5 wherein $R^6$ is H or methyl.

**7.** A compound according to claim 1 or 2 wherein $R^3$ is piperazine; homopiperazine; 2,6-dimethylpiperazine; 3,5-dimethylpiperazine; 2,5-dimethylpiperazine; 2-methylpiperazine; 3-methylpiperazine; 2,2-dimethylpiperazine; 3,3-dimethylpiperazine; piperidine; 1,2,3,6-tetrahydro-pyrazine; or 4-pyrrolidin-3-yloxy.

**8.** The compound according to any one of claims 1 to 7 wherein the groups Y and X are attached to any unsubstituted carbon atom.

**9.** The compound according to any one of claims 1 to 8, wherein D is pyrrolyl, thienyl or furanyl.

**10.** The compound according to any one of claims 1 to 9, wherein P is

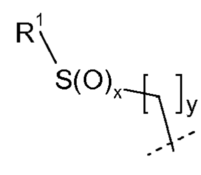 (c)

wherein $R^1$, x, and y are as defined in claim 1.

**11.** The compound according to any one of claims 1 to 9, wherein P is

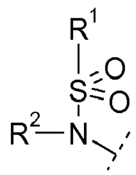 (a) or 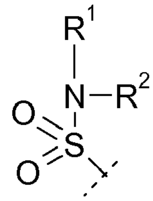 (b)

wherein $R^1$ and $R^2$ are as defined in claim 1.

**12.** The compound according to claim 11, wherein $R^2$ is H.

**13.** The compound according to claim 10 of the general formula (IV)

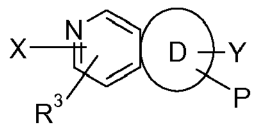

(IV)

wherein P is of the formula (c), $R^1$, x, y, X, and Y are as defined in claim 1, and $R^3$ is as defined in claim 2, and wherein D is a five-membered heteroaryl ring, said ring comprising one or two atoms selected from the group consisting of nitrogen, sulfur and oxygen; and when the heteroaryl ring comprises one or two nitrogen atoms, a group $R^6$ is attached at any nitrogen atom which allows the substitution

**14.** The compound according to claim 13 wherein D is a thiophene and P is attached to the D ring.

**15.** The compound according to claim 13 wherein D is pyrrole and P is attached to the nitrogen atom in the D ring.

**16.** The compound according to claim 13 wherein D is furan and P is attached to the D ring.

**17.** The compound according to claim 10 of the general formula (V)

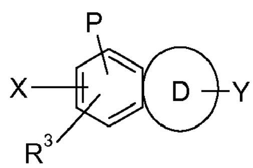

(V)

wherein P is of the formula (c) as defined in claim 1, $R^1$, x, y, X, Y, and $R^3$ are as defined in claim 1, and wherein D is a five-membered heteroaryl ring, said heteroaryl ring comprising one or two atoms selected from the group consisting of nitrogen, sulfur and oxygen; and when the heteroaryl ring comprises one or two nitrogen atoms, a group $R^6$ is attached at any nitrogen atom which allows the substitution.

**18.** The compound according to claim 11 or 12 of the general formula (V)

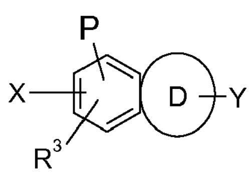

(V)

wherein P is of the formula (a) or (b) as defined in claim 1, preferably wherein $R^2$ is H, X, Y, and $R^3$ are as defined in claim 1, and
wherein D is a five-membered heteroaryl ring, said heteroaryl ring comprising one or two atoms selected from the group consisting of nitrogen, sulfur and oxygen; and when the heteroaryl ring comprises one or two nitrogen atoms, a group $R^6$ is attached at any nitrogen atom which allows the substitution.

**19.** The compound according to any one of claims 11 and 12 of the general formula (VI)

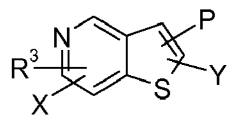

(VI)

wherein P is of the formula (a) or (b) as defined in claim 1, preferably wherein $R^2$ is H, X and Y are as defined in claim 1, and $R^3$ is as defined in claim 2.

**20.** The compound according to any one of claims 11 and 12 of the general formula (VII)

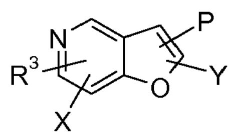

(VII)

wherein P is of the formula (a) or (b) as defined in claim 1, preferably wherein $R^2$ is H, X and Y are as defined in claim 1, and $R^3$ is as defined in claim 4.

**21.** The compound according to any one of claims 11 and 12 of the general formula (VIII)

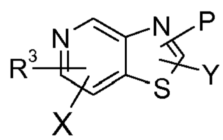

(VIII)

wherein P is of the formula (a) or (b) as defined in claim 1, preferably wherein $R^2$ is H, X, Y, and $R^3$ are as defined in claim 1.

**22.** The compound according to any one of claims 11 and 12 of the general formula (IX)

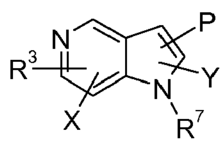

(IX)

wherein $R^7$ in formula (IX) is:

(a) H,
(b) $C_{1-6}$ alkyl,
(c) benzyl,
(d) $-CH_2-CH_2-OH$, or
(e) $CH_2-CH_2-O-CH_3$, and

wherein P is of the formula (a) or (b) as defined in claim 1, preferably wherein $R^2$ is H, X, Y, and $R^3$ are as defined in claim 1.

**23.** The compound according to claim 10 of the general formula (X)

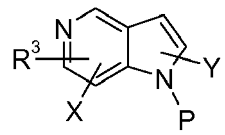

(X)

wherein P is of the formula (a) or (b) as defined in claim 1, preferably wherein $R^2$ is H, X, Y, and $R^3$ are as defined in claim 1.

**24.** A compound according to any one of claims 1 to 12 of the general formula (XII):

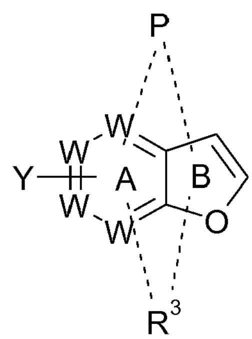

(XII)

or a pharmaceutically acceptable salt thereof, wherein P and $R^3$ are attached to the same ring or to different rings of rings A and B, wherein A, B, Y, P, and $R_3$ are as defined in claim 1.

**25.** The compound according to claim 13, which is the compound

4-(1,4-Diazepan-1-yl)-2-(phenylsulfonyl)thieno[3,2-c]pyridine hydrochloride;
4-(1,4-Diazepan-1-yl)-2-[(3,4-dichlorophenyl)sulfonyl]thieno[3,2-c]pyridine hydrochloride;
4-(1,4-Diazepan-1-yl)-2-[4-tert-butylphenylsulfonyl)thieno[3,2-c]pyridine hydrochloride;
4-(1,4-Diazepan-1-yl)-2-[4-tert-butylphenylsulfonyl)thieno[3,2-c]pyridine hydrochloride;
4-(1,4-Diazepan-1-yl)-2-[3,4-dimethylphenylsulfonyl)thieno[3,2-c]pyridine hydrochloride;
2-[(4-Bromophenyl)sulfonyl]-4-(1,4-diazepan-1-yl)thieno[3,2-c]pyridine hydrochloride;
2-(Phenylsulfonyl)-4-piperazin-1-ylthieno[3,2-c]pyridine hydrochloride;
2-(3-Methoxy-benzenesulfonyl)-4-piperazin-1-yl-thieno[3,2-c]pyridine hydrochloride;
2-(4-Methoxy-benzenesulfonyl)-4-piperazin-1-yl-thieno[3,2-c]pyridine hydrochloride;
4-Piperazin-1-yl-2- {[4-trifluoromethyl)phenyl]sulfonyl}thieno[3,2-c]pyridine hydrochloride;
2-[[2-tert-Butylphenyl)sulfonyl]-4-piperazin-1-ylthieno[3,2-c]pyridine hydrochloride;
2-[(3,4-Dichlorophenyl)sulfonyl]-4-piperazin-1-ylthieno [3,2-c]pyridine-2-sulfonamide hydrochloride;
2-[(4-tert-Butylphenyl)sulfonyl]-4-piperazin-1-ylthieno[3,2-c]pyridine hydrochloride;
2-(1-Naphthyl sulfonyl)-4-piperazin-1-ylthieno[3,2-c]pyridine hydrochloride;
2-[(3-Fluorophenyl)sulfonyl]-4-piperazin-1-ylthieno[3,2-c]pyridine-2-sulfonamide hydrochloride;
2-(Mesitylsulfonyl)-4-piperazin-1-ylthieno[3,2-c]pyridine hydrochloride;
2-[(2-Methoxyphenyl)sulfonyl]-4-piperazin-1-ylthieno[3,2-c]pyridine hydrochloride;
2-[(2,4-Dimethoxyphenyl)sulfonyl]-4-piperazin-1-ylthieno[3,2-c]pyridine hydrochloride;
2-[(2,4-Dimethylphenyl)sulfonyl]-4-piperazin-1-ylthieno[3,2-c]pyridine hydrochloride;
2-[(2,5-Dimethylphenyl)sulfonyl]-4-piperazin-1-ylthieno[3,2-c]pyridine hydrochloride;
2-[(2-Ethylphenyl)sulfonyl]-4-piperazin-1-ylthieno[3,2-c]pyridine hydrochloride;

4-(Piperazinyl)-2-(3-methoxybenzyl-sulfonyl)-thienopyridine hydrochloride;
2-(Benzylsulfonyl)-4-piperazin-1-ylthieno[3,2-c]pyridine hydrochloride;
4-Piperazin-1-yl-2-{[4-(trifluoromethyl)benzyl]sulfonyl}thieno[3,2-c]pyridine hydrochloride;
2-[(3-Bromobenzyl)sulfonyl]-4-piperazin-1-ylthieno[3,2-c]pyridine hydrochloride;
2-[(2,3-Difluorobenzyl)sulfonyl]-4-piperazin-1-ylthieno[3,2-c]pyridine hydrochloride;
2-[(4-Bromobenzyl)sulfonyl]-4-piperazin-1-ylthieno[3,2-c]pyridine hydrochloride;
2- {[2,5-bis(Trifluoromethyl)benzyl]sulfonyl}-4-piperazin-1-ylthieno [3,2-c]pyridine hydrochloride;
2-[(4-Methylbenzyl)sulfonyl]-4-piperazin-1-ylthieno[3,2-c]pyridine hydrochloride;
2-{[5-Chloro-2-(trifluoromethyl)benzyl]sulfonyl}-4-piperazin-1-ylthieno[3,2-c]pyridine hydrochloride;
2-[(3,5-Dimethoxybenzyl)sulfonyl]-4-piperazin-1-ylthieno[3,2-c]pyridine hydrochloride;
2-[(2-Naphthylmethyl)sulfonyl]-4-piperazin-1-ylthieno[3,2-*c*]pyridine hydrochloride;
4-Piperazin-1-yl-2-{[4-(1,2,3-thiadiazol-4-yl)benzyl]sulfonyl}thieno[3,2-*c*]pyridine hydrochloride;
1-(4-Pyrrolidin-1-ylphenyl)-2-[(4-piperazine-1-ylthieno[3,2-c]pyridin-2-yl) sulfonyl] ethanone hydrochloride; or
1-[4-(Diethylamino)phenyl]-2-[(4-piperazine-1-ylthieno[3,2-c]pyridin-2-yl) sulfonyl] ethanone hydrochloride.

**26.** The compound according to claim 13, which is the compound:

1-(4-Methylphenylsulphonyl)-4-piperazin-1-yl-1*H*-pyrrolo[3,2-*c*]pyridine hydrochloride;
1-(3-Chloro-2-methylphenylsulphonyl)-4-piperazin-1-yl-1*H*-pyrrolo[3,2-*c*]pyridine hydrochloride;
1-(3,4-Dimethoxyphenylsulphonyl)-4-piperazin-1-yl-1*H*-pyrrolo[3,2-*c*]pyridine hydrochloride;
4-(4-Piperazin-1-yl-pyrrolo[3,2-c]pyridine-1-sulfonyl)-benzonitrile hydrochloride;
1-(4,5-Dichloro-thiophene-2-sulfonyl)-4-piperazin-1-yl-1H-pyrrolo[3,2-c]pyridine hydrochloride;
1-(2-Chloro-4-fluorophenylsulphonyl)-4-piperazin-1-yl-1*H*-pyrrolo[3,2-*c*]pyridine hydrochloride;
1-Phenylmethanesulfonyl-4-piperazin-1-yl-1H-pyrrolo [3,2-c]pyridine hydrochloride;
1-(5-Chloro-thiophene-2-sulfonyl)-4-piperazin-1-yl-1H-pyrrolo[3,2-c]pyridine hydrochloride;
1-(4-Butyl-benzenesulfonyl)-4-piperazin-1-yl-1H-pyrrolo(3,2-c)pyridine hydrochloride;
1-(4-Phenoxy-benzenesulfonyl)-4-piperazin-1-yl-1H-pyrrolo(3,2-c)pyridine hydrochloride;
1-(Phenylsulfonyl)-4-piperazin-1-yl-1H-pyrrolo[3,2-c]pyridine hydrochloride;
1-[(4-Chlorophenyl)sulfonyl]-4-piperazin-1-yl-1H-pyrro10[3,2-c]pyridine hydrochloride;
1-[(4-Methoxyphenyl)sulfonyl]-4-piperazin-1-yl-1H-pyrrolo[3,2-c]pyridine hydrochloride;
1-[(2-Methoxy-5-methylphenyl)sulfonyl]-4-piperazin-1-yl-1H-pyrrolo[3,2-c]pyridine hydrochloride; or
4-Piperazin-1-yl-1- ([2-(trifluoromethyl)phenyl] sulfonyl)-1H-pyrrolo[3,2-c]pyridine hydrochloride.

**27.** The compound according to claim 19, which is the compound

N-(4-Methylphenyl)-4-(4-methylpiperazin-1-yl)thieno[3,2-c]pyridine-2-sulfonamide hydrochloride;
2-Bromo-4-(4-methyl-piperazin-1-yl)-thieno[3,2-c]pyridine-3-sulfonic acid p-tolylamide hydrochloride; 1
4-(4-Methylpiperazin-1-yl)-n-phenylthieno[3,2-c]pyridine-2-sulfonamide hydrochloride;
4-Piperazin-1-yl-thieno[3,2-c]pyridine-2-sulfonic acid (3-fluoro-5-trifluoromethyl-phenyl)-amide hydrochloride;
4-Piperazin-1-yl-thieno[3,2-c]pyridine-2-sulfonic acid (4-chloro-phenyl)-amide hydrochloride;
4-Piperazin-1-yl-thieno[3,2-c]pyridine-2-sulfonic acid (4-isopropyl-phenyl)-amide hydrochloride;
4-Piperazin-1-yl-thieno[3,2-c]pyridine-2-sulfonic acid *p*-tolylamide hydrochloride;
4-(4-Methylpiperazin-1-yl)-*N*- (2-cyclohex-1-en-1-ylethyl) thieno [3,2-*c*] pyridine-2-sulfonamide hydrochloride;
2-(4-(4-Methylpiperazin-1-yl) thieno [3,2-*c*] pyridin-2-ylsulfonyl)-1,2,3,4-tetrahydroisoquinoline hydrochloride;
4-(4-Methylpiperazin-1-yl)-*N*- (2-thien-2-ylethyl) thieno [3,2-*c*] pyridine-2-sulfonamide hydrochloride;
4-(4-Methylpiperazin-1-yl)-*N*-[1-(1-naphthyl) ethyl] thieno [3,2-c] pyridine-2-sulfonamide hydrochloride;
4-(4-Methylpiperazin-1-yl)-*N*-(4-hexylphenyl) thieno [3,2-c] pyridine-2-sulfonamide hydrochloride;
*N*- (3-Chlorobenzyl)-4-(4-methylpiperazin-1-yl) thieno [3,2-*c*] pyridine-2-sulfonamide;
4-(4-Methylpiperazin-1-yl)-*N*- [1-(4-fluorophenyl) ethyl] thieno [3,2-*c*] pyridine-2-sulfonamide hydrochloride;
*N*- (2,3-Difluorobenzyl)-4-(4-methylpiperazin-1-yl) thieno [3,2-*c*] pyridine-2-sulfonamide hydrochloride;
4-(4-Methylpiperazin-1-yl)-*N*-(4-chloro-2, 5-dimethoxyphenyl) thieno [3,2-c] pyridine-2-sulfonamide hydrochloride;
2-Bromo-4- (4-methylpiperazin-1-yl)-*N* (2-cyclohex-1-en-1-ylethyl) thieno [3,2-c] pyridine-3-sulfonamide hydrochloride;
2-Bromo-4- (4-methylpiperazin-1-yl)-*N*- [(1S)-1-(2-naphthyl) ethyl] thieno [3,2-c] pyridine-3-sulfonamide hydrochloride;
2-Bromo-4- (4-methylpiperazin-1-yl)-*N*-[1-(4-fluorophenyl) ethyl] thieno [3,2-c] pyridine-3-sulfonamide hydrochloride;

2-Bromo-4- (4-methylpiperazin-1-yl)-*N*-(2,4,5-trimethoxyphenyl) thieno [3,2-c] pyridine-3-sulfonamide;
N-(3,4-Dichlorophenyl)-4-piperazin-1-ylthieno [3,2-c]pyridine-2-sulfonamide hydrochloride;
N-(2,4-Difluorophenyl)-4-piperazin-1-ylthieno [3,2-c]pyridine-2-sulfonamide hydrochloride;
4-Piperazin-1-yl-N-[-3-(trifluoromethyl)phenyl]thieno[3,2-c]pyridine-2-sulfonamide hydrochloride;
N-(3-Ethylphenyl)-4-piperazin-1-ylthieno[3,2-c]pyridine-2-sulfonamide hydrochloride;
N-(3,4-Dimethoxyphenyl)-4-piperazin-1-ylthieno[3,2-c]pyridine-2-sulfonamide hydrochloride;
N-(4-Bromo-2-methylphenyl)-4-piperazin-1-ylthieno[3,2-c]pyridine-2-sulfonamide hydrochloride;
2-(4-Piperazin-1-yl-thieno[3,2-c]pyridine-2-sulfonyl)-1,2,3,4-tetrahydro-isoquinoline hydrochloride;
4-Piperazin-1-yl-thieno[3,2-c]pyridine-2-sulfonic acid (2-thiophen-2-yl-ethyl)-amide hydrochloride;
4-Piperazin-1-yl-thieno[3,2-c]pyridine-2-sulfonic acid (4-chloro-2,5-dimethoxy-phenyl)-amide hydrochloride;
4-Piperazin-1-yl-thieno[3,2-c]pyridine-2-sulfonic acid phenethyl-amide hydrochloride;
4-Piperazin-1-yl-thieno[3,2-c]pyridine-2-sulfonic acid (2,6-diethyl-phenyl)-amide hydrochloride;
4-Piperazin-1-yl-thieno[3,2-c]pyridine-2-sulfonic acid (3-phenyl-propyl)-amide hydrochloride;
4-Piperazin-1-yl-thieno[3,2-c]pyridine-2-sulfonic acid (3,3-diphenyl-propyl)-amide hydrochloride;
4-Piperazin-1-yl-thieno[3,2-c]pyridine-2-sulfonic acid [2-(5-methoxy-1H-indol-3-yl)-ethyl]-amide hydrochloride;
4-Piperazin-1-yl-thieno[3,2-c]pyridine-2-sulfonic acid 4-trifluoromethyl-benzylamide hydrochloride;
4-Piperazin-1-yl-thieno[3,2-c]pyridine-2-sulfonic acid benzyl-ethyl-amide hydrochloride;
N-(3-Ethylphenyl)-4-piperazin-1-ylthieno[3,2-c]pyridine-3-sulfonamide hydrochloride;
N-(4-Isopropylphenyl)-4-piperazin-1-ylthieno[3,2-c]pyridine-3-sulfonamide hydrochloride;
N-(4-Methylphenyl)-4-(pyrrolidin-3-yloxy)thieno[3,2-c]pyridine-2-sulfonamide hydrochloride;
N-(4-Methylphenyl)-4-(piperidin-4-yloxy)thieno[3,2-c]pyridine-2-sulfonamide hydrochloride;
N-(2,3-Difluorobenzyl)-4-piperazin-1-ylthieno [3,2-c]pyridine-2-sulfonamide hydrochloride;
N-(3-Chlorobenzyl)-4-piperazin-1-ylthieno[3,2-c]pyridine-2-sulfonamide hydrochloride;
4-Piperazin-1-yl-thieno[3,2-c]pyridine-2-sulfonic acid phenylamide hydrochloride;
4-Piperazin-1-yl-thieno[3,2-c]pyridine-2-sulfonic acid (4-tert-butyl-phenyl)-amide hydrochloride;
4-(4-Methyl-piperazin-1-yl)-thieno[3,2-c]pyridine-2-sulfonic acid phenylamide hydrochloride;
4-(4-Methyl-piperazin-1-yl)-thieno[3,2-c]pyridine-2-sulfonic acid (3-chloro-phenyl)-amide hydrochloride;
2-Bromo-4-(4-methyl-piperazin-1-yl)-thieno[3,2-c]pyridine-3-sulfonic acid phenylamide hydrochloride;
4-(4-Methyl-piperazin-1-yl)-thieno[3,2-c]pyridine-3-sulfonic acid (4-methylphenyl)-amide hydrochloride; or
N-Phenyl-7-piperazin-1-ylthieno[2,3-c]pyridine-2-sulfonamide hydrochloride.

**28.** A process for the preparation of a compound according to claim 10, wherein P is

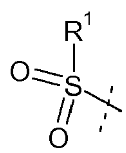

,

said method comprising the steps of:
preparation of the heteroaromatic 5-member ring fused halogen-substituted pyridine, reduction of an aromatic nitro group; aromatic nucleophilic substitution with a thiol via a diazointermediate; oxidation of the thiol derivative to a sulphone; introduction of a halogen atom by electrophilic aromatic substitution; aromatic nucleophilic substitution of the halogen with a diamine.

**29.** A process for the preparation of a compound according to claim 11, wherein P is

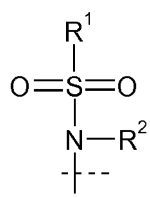

,

said method comprising the steps of: preparation of the heteroaromatic 5-member ring fused pyridine; introduction

of a carboxylic moiety; conversion of the carboxylic moiety to amine by Curtius rearrangement; reaction of the amine group with a sulphonylchloride.

**30.** A process for the preparation of a compound according to claim 11, wherein P is

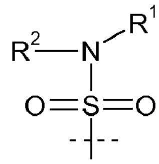

,

said method comprising the steps of: preparation of the heteroaromatic 5-member ring fused pyridine; introduction of sulfonylchloride moiety by nucleophilic addition; reaction of sulphonylchloride moiety with an aniline to obtained a sulfonamide; aromatic nucleophilic substitution of the chloro with a diamine.

**31.** A compound according to any one of claims 1 to 27 for use in therapy.

**32.** A pharmaceutical formulation comprising a compound according to any one of claims 1 to 27 as an active ingredient, in combination with a pharmaceutically acceptable diluent or carrier.

**33.** A pharmaceutical formulation comprising a compound according to any one of claims 1 to 27 for use in the treatment or prophylaxis of a 5-$HT_6$ receptor related disorder, such as obesity, type II diabetes, and/or disorders of the central nervous system, to achieve reduction of body weight and of body weight gain.

**34.** A pharmaceutical formulation comprising a compound according to any one of claims 1 to 27 as an active ingredient, for use in the treatment or prophylaxis of disorders of the central nervous system.

**35.** A pharmaceutical formulation comprising a compound according to any one of claims 1 to 38, and for the case when ring D is a pyrrole ring, P is of the formula (c) and $R^3$ is of the formula

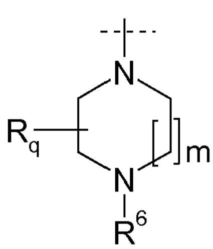 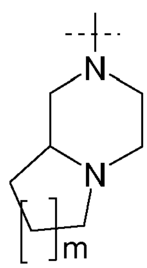 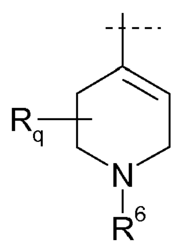 or 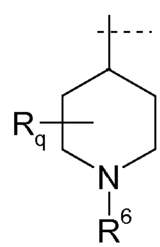

substituted in position 3 on the pyrrole ring, as an active ingredient, for use in the treatment or prophylaxis of type II diabetes.

**36.** A pharmaceutical formulation comprising a compound according to any one of claims 1 to 27, and for the case when ring D is a pyrrole ring, P is of the formula (c) and $R^3$ is of the formula

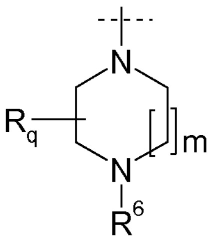 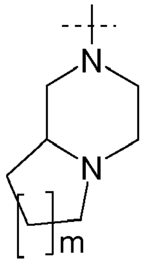 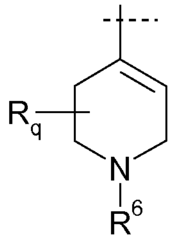 or 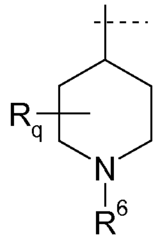

substituted in position 3 on the pyrrole ring, as an active ingredient, for use in the treatment or prophylaxis of obesity, to achieve reduction of body weight and of body weight gain.

**37.** Use of a compound according to any one of claims 1 to 27, for the manufacture of a medicament for use in the treatment or prophylaxis of a 5-$HT_6$ receptor related disorder, such as obesity, type II diabetes, and/or disorders of the central nervous system, to achieve reduction of body weight and of body weight gain.

**38.** Use of a compound according to any one of claims 1 to 27 for the manufacture of a medicament for use in the treatment or prophylaxis of disorders of the central nervous system.

**39.** Use of a compound according to any one of claims 1 to 27, and for the case when ring D is a pyrrole ring, P is of the formula (c) and $R^3$ is of the formula

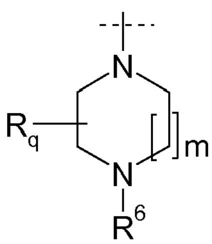 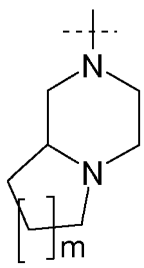 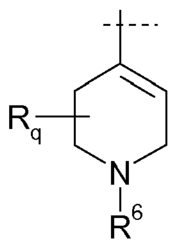 or 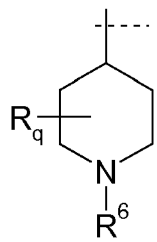

substituted in position 3 on the pyrrole ring, for the manufacture of a medicament for use in the treatment or prophylaxis of type II diabetes.

**40.** Use of a compound according to any one of claims 1 to 27, and for the case when ring D is a pyrrole ring, P is of the formula (c) and $R^3$ is of the formula

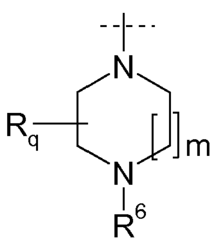 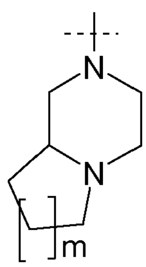 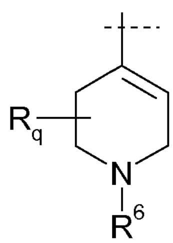 or 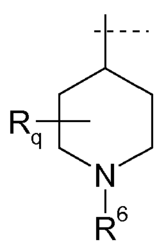

substituted in position 3 on the pyrrole ring, for the manufacture of a medicament for use in the treatment or prophylaxis of obesity, to achieve reduction of body weight and of body weight gain.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0034242 A
- WO 9942465 A
- WO 0132646 A1
- WO 9937623 A2
- WO 9942465 A3
- EP 0815861 A1
- WO 9902502 A2
- WO 9827081 A1
- EP 0701819 A
- US 6191141 B
- WO 0112629 A


### Non-patent literature cited in the description


- **RUAT, M. et al.** *Biochem. Biophys. Res. Commun.,* 1993, vol. 193, 268-276
- **SEBBEN, M. et al.** *NeuroReport,* 1994, vol. 5, 2553-2557
- **BENTLEY, J.C. et al.** *Br J Pharmac. Suppl.,* 1999, vol. 126, 66
- **BENTLEY, J.C. et al.** *J. Psychopharmacol. Suppl.,* 1997, vol. A64, 255
- **WOOLLEY M.L. et al.** *Neuropharmacology,* 2001
- **ISAAC, M. et al.** 6-Bicyclopiperazinyl-1-arylsulfonylindoles and 6-Bicyclopiperidinyl-1-arylsulfonylindoles derivatives as novel, potent and selective 5-HT6 receptor antagonists. *Bioorganic & Medicinal Chemistry Letters,* 2000, vol. 10, 1719-1721
- *J. Med. Chem.,* 1970, vol. 13 (4), 592-598
- **R. LAROCK.** Comprehensive Organic Transformations. VCH Publishers, 1989
- **T.W. GREENE ; P.G.M. WUTS.** Protective Groups in Organic Synthesis. John Wiley and Sons, 1991
- **L. FIESER ; M. FIESER.** Fieser and Fieser's Reagents for Organic Synthesis. John Wiley and Sons, 1994
- Encyclopedia of Reagents for Organic Synthesis. John Wiley and Sons, 1995
- **BOESS F.G et al.** *Neuropharmacology,* 1997, vol. 36 (4/5), 713-720
- **BRADFORD MM.** A rapid and sensitive method for the quantitation of microgram quantities of protein utilizing the principle of protein-dye binding. *Anal Biochem,* 1976, vol. 72, 248-54
- **CHENG Y.C.** *Biochem. Pharmacol.,* 1973, vol. 22, 3099-3108
- **BOESS et al.** *Neuropharmacology,* 1997, vol. 36, 713-720
- **BLUNDELL, J.E. ; HALFORD, J.C.G.** Serotonin and Appetite Regulation. Implications for the Pharmacological Treatment of Obesity. *CNS Drugs,* 1998, vol. 9, 473-495
- **THEEUWES, F. ; YAM, S.I.** *Ann. Biomed. Eng.,* 1976, vol. 4 (4), 343-353